# EUROPEAN PATENT APPLICATION

(11) **EP 2 335 721 A1**
(43) Date of publication of application: **22.06.2011**
(21) Application number: 09290987.8
(22) Date of filing: 21.12.2009
(51) Int. Cl.: A61K 39/04, G01N 33/50

(54) **Streptavidin and Biotin-based antigen delivery system**

(71) Applicant: Institut Pasteur, 75724 Paris Cedex 15 (FR)
(72) Inventor: Leclerc, Claude, 75015 Paris (FR); Majlessi, Laleh, 78180 Montigny Le Bretonneux (FR); Dong, Hui, Yangzhou, Jiangsu 225003 (CN); Sebo, Peter, 142 00 Prague 4 (CZ); Stanek, Ondrej, 199 00 Prague 9 (CZ)
(74) Representative: Desaix, Anne

(57) **Abstract**

The invention is directed to a composition, which comprises:
(i) a fusion polypeptide comprising a streptavidin (SA) or avidin polypeptide and one or several effector molecule(s), wherein said fusion polypeptide retains the property of SA and avidin polypeptides to bind biotin;
(ii) biotinylated targeting molecule(s), which are capable of targeting subset(s) of cells and/or cell surface molecule(s), and in particular dendritic cells (DC), subsets of DC and/or surface molecule(s) (including surface receptor(s)) of DC.
The combination of the invention is suitable for diagnosing or immunomonitoring a disease in a mammal or in prophylactic treatment and especially in vaccination and in therapy including in immunotherapy.

SEQUENCE LISTING

<110>  INSTITUT PASTEUR

<120>  A VERSATILE DELIVERY SYSTEM FOR ANTIGENS OR BIOLOGICALLY ACTIVE
       MOLECULES

<130>  B08659 _AD/SL/CAL

<140>  EPXXXX
<141>  2009-12-21

<160>  41

<170>  PatentIn version 3.3

<210>  1
<211>  159
<212>  PRT
<213>  Steptomyces avidinii


<220>
<221>  MISC_FEATURE
<222>  (1)..(159)
<223>  Mature, full-length SA protein

<400>  1

Asp Pro Ser Lys Asp Ser Lys Ala Gln Val Ser Ala Ala Glu Ala Gly
1               5                   10                  15


Ile Thr Gly Thr Trp Tyr Asn Gln Leu Gly Ser Thr Phe Ile Val Thr
            20                  25                  30


Ala Gly Ala Asp Gly Ala Leu Thr Gly Thr Tyr Glu Ser Ala Val Gly
        35                  40                  45


Asn Ala Glu Ser Arg Tyr Val Leu Thr Gly Arg Tyr Asp Ser Ala Pro
    50                  55                  60


Ala Thr Asp Gly Ser Gly Thr Ala Leu Gly Trp Thr Val Ala Trp Lys
65                  70                  75                  80


Asn Asn Tyr Arg Asn Ala His Ser Ala Thr Thr Trp Ser Gly Gln Tyr
                85                  90                  95


Val Gly Gly Ala Glu Ala Arg Ile Asn Thr Gln Trp Leu Leu Thr Ser
            100                 105                 110


Gly Thr Thr Glu Ala Asn Ala Trp Lys Ser Thr Leu Val Gly His Asp
        115                 120                 125


Thr Phe Thr Lys Val Lys Pro Ser Ala Ala Ser Ile Asp Ala Ala Lys
    130                 135                 140


Lys Ala Gly Val Asn Asn Gly Asn Pro Leu Asp Ala Val Gln Gln
145                 150                 155

<210>  2
<211>  127
<212>  PRT
<213>  Artificial

<220>
<223>  polypeptide


<220>
<221>  MISC_FEATURE
<222>  (1)..(127)
<223>  Natural core SA (residues 13-139 of the mature, full-length SA
       protein)

<400>  2

Ala Glu Ala Gly Ile Thr Gly Thr Trp Tyr Asn Gln Leu Gly Ser Thr
1               5                   10                  15


Phe Ile Val Thr Ala Gly Ala Asp Gly Ala Leu Thr Gly Thr Tyr Glu
            20                  25                  30


Ser Ala Val Gly Asn Ala Glu Ser Arg Tyr Val Leu Thr Gly Arg Tyr
        35                  40                  45


Asp Ser Ala Pro Ala Thr Asp Gly Ser Gly Thr Ala Leu Gly Trp Thr
    50                  55                  60


Val Ala Trp Lys Asn Asn Tyr Arg Asn Ala His Ser Ala Thr Thr Trp
65                  70                  75                  80


Ser Gly Gln Tyr Val Gly Gly Ala Glu Ala Arg Ile Asn Thr Gln Trp
                85                  90                  95


Leu Leu Thr Ser Gly Thr Thr Glu Ala Asn Ala Trp Lys Ser Thr Leu
            100                 105                 110


Val Gly His Asp Thr Phe Thr Lys Val Lys Pro Ser Ala Ala Ser
        115                 120                 125


<210>  3
<211>  126
<212>  PRT
<213>  Artificial

<220>
<223>  polypeptide - Stv-25

<400>  3

Met Glu Ala Gly Ile Thr Gly Thr Trp Tyr Asn Gln Leu Gly Ser Thr
1               5                   10                  15


Phe Ile Val Thr Ala Gly Ala Asp Gly Ala Leu Thr Gly Thr Tyr Glu
            20                  25                  30

Ser Ala Val Gly Asn Ala Glu Ser Arg Tyr Val Leu Thr Gly Arg Tyr
        35                  40                  45

Asp Ser Ala Pro Ala Thr Asp Gly Ser Gly Thr Ala Leu Gly Trp Thr
    50                  55                  60

Val Ala Trp Lys Asn Asn Tyr Arg Asn Ala His Ser Ala Thr Thr Trp
65                  70                  75                  80

Ser Gly Gln Tyr Val Gly Gly Ala Glu Ala Arg Ile Asn Thr Gln Trp
                85                  90                  95

Leu Leu Thr Ser Gly Thr Thr Glu Ala Asn Ala Trp Lys Ser Thr Leu
            100                 105                 110

Val Gly His Asp Thr Phe Thr Lys Val Lys Pro Ser Ala Ala
        115                 120                 125

<210>  4
<211>  119
<212>  PRT
<213>  Artificial

<220>
<223>  polypeptide - Stv-13

<400>  4

Met Gly Ile Thr Gly Thr Trp Tyr Asn Gln Leu Gly Ser Thr Phe Ile
1               5                   10                  15

Val Thr Ala Gly Ala Asp Gly Ala Leu Thr Gly Thr Tyr Glu Ser Ala
            20                  25                  30

Val Gly Asn Ala Glu Ser Arg Tyr Val Leu Thr Gly Arg Tyr Asp Ser
        35                  40                  45

Ala Pro Ala Thr Asp Gly Ser Gly Thr Ala Leu Gly Trp Thr Val Ala
    50                  55                  60

Trp Lys Asn Asn Tyr Arg Asn Ala His Ser Ala Thr Thr Trp Ser Gly
65                  70                  75                  80

Gln Tyr Val Gly Gly Ala Glu Ala Arg Ile Asn Thr Gln Trp Leu Leu
                85                  90                  95

Thr Ser Gly Thr Thr Glu Ala Asn Ala Trp Lys Ser Thr Leu Val Gly
            100                 105                 110

His Asp Thr Phe Thr Lys Val
        115

<210>  5
<211>  128

<212>  PRT
<213>  Artificial

<220>
<223>  polypeptide - SA polypeptide

<400>  5

Glu Ala Gly Ile Thr Gly Thr Trp Tyr Asn Gln Leu Gly Ser Thr Phe
1               5                   10                  15

Ile Val Thr Ala Gly Ala Asp Gly Ala Leu Thr Gly Thr Tyr Glu Ser
            20                  25                  30

Ala Val Gly Asn Ala Glu Ser Arg Tyr Val Leu Thr Gly Arg Tyr Asp
        35                  40                  45

Ser Ala Pro Ala Thr Asp Gly Ser Gly Thr Ala Leu Gly Trp Thr Val
    50                  55                  60

Ala Trp Lys Asn Asn Tyr Arg Asn Ala His Ser Ala Thr Thr Trp Ser
65                  70                  75                  80

Gly Gln Tyr Val Gly Gly Ala Glu Ala Arg Ile Asn Thr Gln Trp Leu
                85                  90                  95

Leu Thr Ser Gly Thr Thr Glu Ala Asn Ala Trp Lys Ser Thr Leu Val
            100                 105                 110

Gly His Asp Thr Phe Thr Lys Val Lys Pro Ser Ala Ala Ser Leu Glu
        115                 120                 125

<210>  6
<211>  24
<212>  DNA
<213>  Artificial

<220>
<223>  primer ESAT-6-I

<400>  6
attaccatga cagagcagca gtgg                                              24

<210>  7
<211>  30
<212>  DNA
<213>  Artificial

<220>
<223>  primer ESAT-6-II

<400>  7
attttccatg gatgcgaaca tcccagtgac                                        30

<210>  8
<211>  28
<212>  DNA
<213>  Artificial

<220>
<223>  TB10.4-I

<400>  8
atgctagcat gtcgcaaatc atgtacaa                                          28


<210>  9
<211>  24
<212>  DNA
<213>  Artificial

<220>
<223>  TB10.4-II oligonucleotide

<400>  9
atgaattcgc cgccccattt ggcg                                              24


<210>  10
<211>  23
<212>  DNA
<213>  Artificial

<220>
<223>  MCS-N-ter.I

<400>  10
catggctagc ggatccctgc agg                                               23


<210>  11
<211>  23
<212>  DNA
<213>  Artificial

<220>
<223>  MCS-N-ter.II

<400>  11
aattcctgca gggatccgct agc                                               23


<210>  12
<211>  33
<212>  DNA
<213>  Artificial

<220>
<223>  MCS-C-ter.I

<400>  12
tcgaaactag tgagctcaag ctttaactcg aga                                    33


<210>  13
<211>  33
<212>  DNA
<213>  Artificial

<220>
<223>  MCS-C-ter.II

<400>  13
agcttctcga gttaaagctt gagctcacta gtt                                    33

<210>  14
<211>  30
<212>  DNA
<213>  Artificial

<220>
<223>  primer OVA-I

<400>  14
ctaccatggc taagatcctg gagcttccat                                        30


<210>  15
<211>  29
<212>  DNA
<213>  Artificial

<220>
<223>  primer OVA-II

<400>  15
tacgaattcg acagatgtga ggttgtatt                                         29


<210>  16
<211>  30
<212>  DNA
<213>  Artificial

<220>
<223>  primer E7-I

<400>  16
taccatggat atgcatggag atacacctac                                        30


<210>  17
<211>  28
<212>  DNA
<213>  Artificial

<220>
<223>  primer E7-II

<400>  17
tcgaattcag gtttctgaga acagatgg                                          28


<210>  18
<211>  32
<212>  DNA
<213>  Artificial

<220>
<223>  primer CMV-G2-I

<400>  18
tacccatgga tatcctggct cgtaacctgg tt                                     32


<210>  19
<211>  26
<212>  DNA
<213>  Artificial

<220>
<223>  primer CMV-G2-II

<400>  19
cgctgcagta cggtgaattc agcacc                                          26


<210>  20
<211>  32
<212>  DNA
<213>  Artificial

<220>
<223>  primer CMV-G3-I

<400>  20
atccatgggt gatatctggc cgccgtggca gg                                   32


<210>  21
<211>  26
<212>  DNA
<213>  Artificial

<220>
<223>  primer CMV-G3-II

<400>  21
ctctgcagca gttcagcgaa gatacg                                          26


<210>  22
<211>  34
<212>  DNA
<213>  Artificial

<220>
<223>  CMV-G4-I

<400>  22
taccatggta gatatcatga cccgtaaccc gcag                                 34


<210>  23
<211>  27
<212>  DNA
<213>  Artificial

<220>
<223>  primer CMV-G4-II

<400>  23
taggatccca gttcagcgaa gatacgg                                         27


<210>  24
<211>  96
<212>  DNA
<213>  Artificial

<220>
<223>  primer OTII-I

<400>  24
aattggatat cgctgaatct ctgaaaatct ctcaggctgt tcacgctgct cacgctgaaa     60

tcaacgaagc tggtcgtgaa gttgaattta ccgtac                               96


<210>  25
<211>  96

<212>  DNA
<213>  Artificial

<220>
<223>  primer OTII-II

<400>  25
aattgtacgg taaattcaac ttcacgacca gcttcgttga tttcagcgtg agcagcgtga     60

acagcctgag agattttcag agattcagcg atatcc                               96


<210>  26
<211>  23
<212>  DNA
<213>  Artificial

<220>
<223>  primer TB7.7-I

<400>  26
atactagtgg cagcggccac gcg                                             23


<210>  27
<211>  28
<212>  DNA
<213>  Artificial

<220>
<223>  primer TB7.7-II

<400>  27
agtaagcttc tacggcggat caccccgg                                        28


<210>  28
<211>  230
<212>  PRT
<213>  Artificial

<220>
<223>  Fusion polypeptide: CFP-10-SA (pET28b-CFP-10-SA)

<400>  28

Met Ala Glu Met Lys Thr Asp Ala Ala Thr Leu Ala Gln Glu Ala Gly
1               5                   10                  15


Asn Phe Glu Arg Ile Ser Gly Asp Leu Lys Thr Gln Ile Asp Gln Val
            20                  25                  30


Glu Ser Thr Ala Gly Ser Leu Gln Gly Gln Trp Arg Gly Ala Ala Gly
        35                  40                  45


Thr Ala Ala Gln Ala Ala Val Val Arg Phe Gln Glu Ala Ala Asn Lys
    50                  55                  60


Gln Lys Gln Glu Leu Asp Glu Ile Ser Thr Asn Ile Arg Gln Ala Gly
65                  70                  75                  80


Val Gln Tyr Ser Arg Ala Asp Glu Glu Gln Gln Gln Ala Leu Ser Ser
                85                  90                  95

Gln Met Gly Phe Ser Met Glu Ala Gly Ile Thr Gly Thr Trp Tyr Asn
            100                 105                 110

Gln Leu Gly Ser Thr Phe Ile Val Thr Ala Gly Ala Asp Gly Ala Leu
        115                 120                 125

Thr Gly Thr Tyr Glu Ser Ala Val Gly Asn Ala Glu Ser Arg Tyr Val
    130                 135                 140

Leu Thr Gly Arg Tyr Asp Ser Ala Pro Ala Thr Asp Gly Ser Gly Thr
145                 150                 155                 160

Ala Leu Gly Trp Thr Val Ala Trp Lys Asn Asn Tyr Arg Asn Ala His
                165                 170                 175

Ser Ala Thr Thr Trp Ser Gly Gln Tyr Val Gly Gly Ala Glu Ala Arg
            180                 185                 190

Ile Asn Thr Gln Trp Leu Leu Thr Ser Gly Thr Thr Glu Ala Asn Ala
        195                 200                 205

Trp Lys Ser Thr Leu Val Gly His Asp Thr Phe Thr Lys Val Lys Pro
    210                 215                 220

Ser Ala Ala Ser Leu Glu
225                 230

<210>  29
<211>  225
<212>  PRT
<213>  Artificial

<220>
<223>  Fusion polypeptide: Esat-6-SA (pET28b-Esat-6-SA)

<400>  29

Met Thr Glu Gln Gln Trp Asn Phe Ala Gly Ile Glu Ala Ala Ala Ser
1               5                   10                  15

Ala Ile Gln Gly Asn Val Thr Ser Ile His Ser Leu Leu Asp Glu Gly
            20                  25                  30

Lys Gln Ser Leu Thr Lys Leu Ala Ala Ala Trp Gly Gly Ser Gly Ser
        35                  40                  45

Glu Ala Tyr Gln Gly Val Gln Gln Lys Trp Asp Ala Thr Ala Thr Glu
    50                  55                  60

Leu Asn Asn Ala Leu Gln Asn Leu Ala Arg Thr Ile Ser Glu Ala Gly
65                  70                  75                  80

Gln Ala Met Ala Ser Thr Glu Gly Asn Val Thr Gly Met Phe Ala Ser
                85                  90                  95

Met Glu Ala Gly Ile Thr Gly Thr Trp Tyr Asn Gln Leu Gly Ser Thr
            100                 105                 110

Phe Ile Val Thr Ala Gly Ala Asp Gly Ala Leu Thr Gly Thr Tyr Glu
        115                 120                 125

Ser Ala Val Gly Asn Ala Glu Ser Arg Tyr Val Leu Thr Gly Arg Tyr
    130                 135                 140

Asp Ser Ala Pro Ala Thr Asp Gly Ser Gly Thr Ala Leu Gly Trp Thr
145                 150                 155                 160

Val Ala Trp Lys Asn Asn Tyr Arg Asn Ala His Ser Ala Thr Thr Trp
                165                 170                 175

Ser Gly Gln Tyr Val Gly Gly Ala Glu Ala Arg Ile Asn Thr Gln Trp
            180                 185                 190

Leu Leu Thr Ser Gly Thr Thr Glu Ala Asn Ala Trp Lys Ser Thr Leu
        195                 200                 205

Val Gly His Asp Thr Phe Thr Lys Val Lys Pro Ser Ala Ala Ser Leu
    210                 215                 220

Glu
225

<210>  30
<211>  100
<212>  PRT
<213>  Artificial

<220>
<223>  CFP-10 polypeptide expressed from pET-28b-CFP-10: Esat-6-SA

<400>  30

Met Ala Glu Met Lys Thr Asp Ala Ala Thr Leu Ala Gln Glu Ala Gly
1               5                   10                  15

Asn Phe Glu Arg Ile Ser Gly Asp Leu Lys Thr Gln Ile Asp Gln Val
            20                  25                  30

Glu Ser Thr Ala Gly Ser Leu Gln Gly Gln Trp Arg Gly Ala Ala Gly
        35                  40                  45

Thr Ala Ala Gln Ala Ala Val Val Arg Phe Gln Glu Ala Ala Asn Lys
    50                  55                  60

Gln Lys Gln Glu Leu Asp Glu Ile Ser Thr Asn Ile Arg Gln Ala Gly
65                  70                  75                  80

Val Gln Tyr Ser Arg Ala Asp Glu Glu Gln Gln Gln Ala Leu Ser Ser
                85                  90                  95

Gln Met Gly Phe
            100

<210>  31
<211>  226
<212>  PRT
<213>  Artificial

<220>
<223>  Fusion polypeptide Esat-6-SA expressed from pET28b-CFP-10:
       Esat-6-SA

<400>  31

Met Thr Glu Gln Gln Trp Asn Phe Ala Gly Ile Glu Ala Ala Ala Ser
1               5                   10                  15

Ala Ile Gln Gly Asn Val Thr Ser Ile His Ser Leu Leu Asp Glu Gly
            20                  25                  30

Lys Gln Ser Leu Thr Lys Leu Ala Ala Ala Trp Gly Gly Ser Gly Ser
        35                  40                  45

Glu Ala Tyr Gln Gly Val Gln Gln Lys Trp Asp Ala Thr Ala Thr Glu
    50                  55                  60

Leu Asn Asn Ala Leu Gln Asn Leu Ala Arg Thr Ile Ser Glu Ala Gly
65                  70                  75                  80

Gln Ala Met Ala Ser Thr Glu Gly Asn Val Thr Gly Met Phe Ala Glu
                85                  90                  95

Phe Met Glu Ala Gly Ile Thr Gly Thr Trp Tyr Asn Gln Leu Gly Ser
            100                 105                 110

Thr Phe Ile Val Thr Ala Gly Ala Asp Gly Ala Leu Thr Gly Thr Tyr
        115                 120                 125

Glu Ser Ala Val Gly Asn Ala Glu Ser Arg Tyr Val Leu Thr Gly Arg
    130                 135                 140

Tyr Asp Ser Ala Pro Ala Thr Asp Gly Ser Gly Thr Ala Leu Gly Trp
145                 150                 155                 160

Thr Val Ala Trp Lys Asn Asn Tyr Arg Asn Ala His Ser Ala Thr Thr
                165                 170                 175

Trp Ser Gly Gln Tyr Val Gly Gly Ala Glu Ala Arg Ile Asn Thr Gln
            180                 185                 190

Trp Leu Leu Thr Ser Gly Thr Thr Glu Ala Asn Ala Trp Lys Ser Thr
        195                 200                 205

Leu Val Gly His Asp Thr Phe Thr Lys Val Lys Pro Ser Ala Ala Ser
    210                 215                 220

Leu Glu
225

<210>  32
<211>  100
<212>  PRT
<213>  Artificial

<220>
<223>  CFP-10 polypeptide expressed from pET28b-CFP-10:Esat-6-SA-Tb7.7

<400>  32

Met Ala Glu Met Lys Thr Asp Ala Ala Thr Leu Ala Gln Glu Ala Gly
1               5                   10                  15

Asn Phe Glu Arg Ile Ser Gly Asp Leu Lys Thr Gln Ile Asp Gln Val
            20                  25                  30

Glu Ser Thr Ala Gly Ser Leu Gln Gly Gln Trp Arg Gly Ala Ala Gly
        35                  40                  45

Thr Ala Ala Gln Ala Ala Val Val Arg Phe Gln Glu Ala Ala Asn Lys
    50                  55                  60

Gln Lys Gln Glu Leu Asp Glu Ile Ser Thr Asn Ile Arg Gln Ala Gly
65                  70                  75                  80

Val Gln Tyr Ser Arg Ala Asp Glu Glu Gln Gln Gln Ala Leu Ser Ser
                85                  90                  95

Gln Met Gly Phe
            100

<210>  33
<211>  309
<212>  PRT
<213>  Artificial

<220>
<223>  Fusion polypeptide Esat-6-SA-Tb7.7 expressed from pET28b-CFP-10:
       Esat-6-SA-Tb 7.7

<400>  33

Met Thr Glu Gln Gln Trp Asn Phe Ala Gly Ile Glu Ala Ala Ala Ser
1               5                   10                  15

Ala Ile Gln Gly Asn Val Thr Ser Ile His Ser Leu Leu Asp Glu Gly
            20                  25                  30

Lys Gln Ser Leu Thr Lys Leu Ala Ala Ala Trp Gly Gly Ser Gly Ser
        35                  40                  45

Glu Ala Tyr Gln Gly Val Gln Gln Lys Trp Asp Ala Thr Ala Thr Glu
    50                  55                  60

Leu Asn Asn Ala Leu Gln Asn Leu Ala Arg Thr Ile Ser Glu Ala Gly
65                  70                  75                  80

Gln Ala Met Ala Ser Thr Glu Gly Asn Val Thr Gly Met Phe Ala Glu
                85                  90                  95

Phe Met Glu Ala Gly Ile Thr Gly Thr Trp Tyr Asn Gln Leu Gly Ser
            100                 105                 110

Thr Phe Ile Val Thr Ala Gly Ala Asp Gly Ala Leu Thr Gly Thr Tyr
        115                 120                 125

Glu Ser Ala Val Gly Asn Ala Glu Ser Arg Tyr Val Leu Thr Gly Arg
    130                 135                 140

Tyr Asp Ser Ala Pro Ala Thr Asp Gly Ser Gly Thr Ala Leu Gly Trp
145                 150                 155                 160

Thr Val Ala Trp Lys Asn Asn Tyr Arg Asn Ala His Ser Ala Thr Thr
                165                 170                 175

Trp Ser Gly Gln Tyr Val Gly Gly Ala Glu Ala Arg Ile Asn Thr Gln
            180                 185                 190

Trp Leu Leu Thr Ser Gly Thr Thr Glu Ala Asn Ala Trp Lys Ser Thr
        195                 200                 205

Leu Val Gly His Asp Thr Phe Thr Lys Val Lys Pro Ser Ala Ala Ser
    210                 215                 220

Leu Glu Thr Ser Gly Ser Gly His Ala Leu Ala Ala Arg Thr Leu Leu
225                 230                 235                 240

Ala Ala Ala Asp Glu Leu Val Gly Gly Pro Pro Val Glu Ala Ser Ala
                245                 250                 255

Ala Ala Leu Ala Gly Asp Ala Ala Gly Ala Trp Arg Thr Ala Ala Val
            260                 265                 270

Glu Leu Ala Arg Ala Leu Val Arg Ala Val Ala Glu Ser His Gly Val
        275                 280                 285

Ala Ala Val Leu Phe Ala Ala Thr Ala Ala Ala Ala Ala Ala Val Asp
    290                 295                 300

Arg Gly Asp Pro Pro
305

<210> 34
<211> 230
<212> PRT
<213> Artificial

<220>
<223> Fusion polypeptide: Tb10.4-SA (pET28b-Tb10.4-SA)

<400> 34

Met Ala Ser Met Ser Gln Ile Met Tyr Asn Tyr Pro Ala Met Leu Gly
1               5                   10                  15

His Ala Gly Asp Met Ala Gly Tyr Ala Gly Thr Leu Gln Ser Leu Gly
            20                  25                  30

Ala Glu Ile Ala Val Glu Gln Ala Ala Leu Gln Ser Ala Trp Gln Gly
        35                  40                  45

Asp Thr Gly Ile Thr Tyr Gln Ala Trp Gln Ala Gln Trp Asn Gln Ala
    50                  55                  60

Met Glu Asp Leu Val Arg Ala Tyr His Ala Met Ser Ser Thr His Glu
65                  70                  75                  80

Ala Asn Thr Met Ala Met Met Ala Arg Asp Thr Ala Glu Ala Ala Lys
                85                  90                  95

Trp Gly Gly Glu Phe Met Glu Ala Gly Ile Thr Gly Thr Trp Tyr Asn
            100                 105                 110

Gln Leu Gly Ser Thr Phe Ile Val Thr Ala Gly Ala Asp Gly Ala Leu
        115                 120                 125

Thr Gly Thr Tyr Glu Ser Ala Val Gly Asn Ala Glu Ser Arg Tyr Val
    130                 135                 140

Leu Thr Gly Arg Tyr Asp Ser Ala Pro Ala Thr Asp Gly Ser Gly Thr
145                 150                 155                 160

Ala Leu Gly Trp Thr Val Ala Trp Lys Asn Asn Tyr Arg Asn Ala His
                165                 170                 175

Ser Ala Thr Thr Trp Ser Gly Gln Tyr Val Gly Gly Ala Glu Ala Arg
            180                 185                 190

Ile Asn Thr Gln Trp Leu Leu Thr Ser Gly Thr Thr Glu Ala Asn Ala
        195                 200                 205

Trp Lys Ser Thr Leu Val Gly His Asp Thr Phe Thr Lys Val Lys Pro
    210                 215                 220

Ser Ala Ala Ser Leu Glu
225                 230

<210>  35
<211>  234
<212>  PRT
<213>  Artificial

<220>
<223>  Fusion polypeptide: OVAepitope-SA (pET28b-OVAepitope-SA)

<400>  35

Met Ala Lys Ile Leu Glu Leu Pro Phe Ala Ser Gly Thr Met Ser Met
1               5                   10                  15

Leu Val Leu Leu Pro Asp Glu Val Ser Gly Leu Glu Gln Leu Glu Ser
            20                  25                  30

Ile Ile Asn Phe Glu Lys Leu Thr Glu Trp Thr Ser Ser Asn Val Met
        35                  40                  45

Glu Glu Arg Lys Ile Lys Val Tyr Leu Pro Arg Met Lys Met Glu Glu
    50                  55                  60

Lys Tyr Asn Leu Thr Ser Val Glu Leu Asp Ile Ala Glu Ser Leu Lys
65                  70                  75                  80

Ile Ser Gln Ala Val His Ala Ala His Ala Glu Ile Asn Glu Ala Gly
                85                  90                  95

Arg Glu Val Glu Phe Thr Val Gln Phe Met Glu Ala Gly Ile Thr Gly
            100                 105                 110

Thr Trp Tyr Asn Gln Leu Gly Ser Thr Phe Ile Val Thr Ala Gly Ala
        115                 120                 125

Asp Gly Ala Leu Thr Gly Thr Tyr Glu Ser Ala Val Gly Asn Ala Glu
    130                 135                 140

Ser Arg Tyr Val Leu Thr Gly Arg Tyr Asp Ser Ala Pro Ala Thr Asp
145                 150                 155                 160

Gly Ser Gly Thr Ala Leu Gly Trp Thr Val Ala Trp Lys Asn Asn Tyr
                165                 170                 175

Arg Asn Ala His Ser Ala Thr Thr Trp Ser Gly Gln Tyr Val Gly Gly
            180                 185                 190

Ala Glu Ala Arg Ile Asn Thr Gln Trp Leu Leu Thr Ser Gly Thr Thr
        195                 200                 205

Glu Ala Asn Ala Trp Lys Ser Thr Leu Val Gly His Asp Thr Phe Thr
    210                 215                 220

Lys Val Lys Pro Ser Ala Ala Ser Leu Glu
225                 230

<210>  36
<211>  171
<212>  PRT
<213>  Artificial

<220>
<223>  Fusion polypeptide: CMVg2-SA(pET28b-CMVg2-SA)

<400>  36

Met Asp Ile Leu Ala Arg Asn Leu Val Pro Met Val Ala Thr Val Gln
1               5                   10                  15

Gly Gln Glu Arg Lys Thr Pro Arg Val Thr Gly Gly Gly Ala Met Ala
            20                  25                  30

Gly Ala Glu Phe Thr Val Leu Gln Glu Phe Met Glu Ala Gly Ile Thr
        35                  40                  45

Gly Thr Trp Tyr Asn Gln Leu Gly Ser Thr Phe Ile Val Thr Ala Gly
    50                  55                  60

Ala Asp Gly Ala Leu Thr Gly Thr Tyr Glu Ser Ala Val Gly Asn Ala
65                  70                  75                  80

Glu Ser Arg Tyr Val Leu Thr Gly Arg Tyr Asp Ser Ala Pro Ala Thr
                85                  90                  95

Asp Gly Ser Gly Thr Ala Leu Gly Trp Thr Val Ala Trp Lys Asn Asn
            100                 105                 110

Tyr Arg Asn Ala His Ser Ala Thr Thr Trp Ser Gly Gln Tyr Val Gly
        115                 120                 125

Gly Ala Glu Ala Arg Ile Asn Thr Gln Trp Leu Leu Thr Ser Gly Thr
    130                 135                 140

Thr Glu Ala Asn Ala Trp Lys Ser Thr Leu Val Gly His Asp Thr Phe
145                 150                 155                 160

Thr Lys Val Lys Pro Ser Ala Ala Ser Leu Glu
                165                 170

<210>  37
<211>  181
<212>  PRT
<213>  Artificial

<220>
<223>  Fusion polypeptide: CMVg3-SA (pET28b-CMVg3-SA)

<400>  37

Met Gly Asp Ile Trp Pro Pro Trp Gln Ala Gly Ile Leu Ala Arg Asn
1               5                   10                  15

Leu Val Pro Met Val Ala Thr Val Gln Gly Gln Asn Leu Lys Tyr Gln
            20                  25                  30

Glu Phe Phe Trp Asp Ala Asn Asp Ile Tyr Arg Ile Phe Ala Glu Leu
        35                  40                  45

Leu Gln Glu Phe Met Glu Ala Gly Ile Thr Gly Thr Trp Tyr Asn Gln
    50                  55                  60

Leu Gly Ser Thr Phe Ile Val Thr Ala Gly Ala Asp Gly Ala Leu Thr
65                  70                  75                  80

Gly Thr Tyr Glu Ser Ala Val Gly Asn Ala Glu Ser Arg Tyr Val Leu
                85                  90                  95

Thr Gly Arg Tyr Asp Ser Ala Pro Ala Thr Asp Gly Ser Gly Thr Ala
            100                 105                 110

Leu Gly Trp Thr Val Ala Trp Lys Asn Asn Tyr Arg Asn Ala His Ser
        115                 120                 125

Ala Thr Thr Trp Ser Gly Gln Tyr Val Gly Gly Ala Glu Ala Arg Ile
    130                 135                 140

Asn Thr Gln Trp Leu Leu Thr Ser Gly Thr Thr Glu Ala Asn Ala Trp
145                 150                 155                 160

Lys Ser Thr Leu Val Gly His Asp Thr Phe Thr Lys Val Lys Pro Ser
                165                 170                 175

Ala Ala Ser Leu Glu
            180

<210>  38
<211>  411
<212>  PRT
<213>  Artificial

<220>
<223>  Fusion polypeptide: CMVg4-SA (pET28b-CMVg4-SA)

<400>  38

Met Val Asp Ile Met Thr Arg Asn Pro Gln Pro Phe Met Arg Pro His
1               5                   10                  15

Glu Arg Asn Gly Phe Thr Val Leu Cys Pro Lys Asn Met Ile Ile Lys
            20                  25                  30

Pro Gly Lys Ile Ser His Ile Met Leu Asp Val Ala Phe Thr Ser His
        35                  40                  45

Glu His Phe Gly Leu Leu Cys Pro Lys Ser Ile Pro Gly Leu Ser Ile
    50                  55                  60

Ser Gly Asn Leu Leu Met Asn Gly Gln Gln Ile Phe Leu Glu Val Gln
65                  70                  75                  80

Ala Ile Arg Glu Thr Val Glu Leu Arg Gln Tyr Asp Pro Val Ala Ala
                85                  90                  95

Leu Phe Phe Phe Asp Ile Asp Leu Leu Leu Gln Arg Gly Pro Gln Tyr
            100                 105                 110

Ser Glu His Pro Thr Phe Thr Ser Gln Tyr Arg Ile Gln Gly Lys Leu
        115                 120                 125

Glu Tyr Arg His Thr Trp Asp Arg His Asp Glu Gly Ala Ala Gln Gly
    130                 135                 140

Asp Asp Asp Val Trp Thr Ser Gly Ser Asp Ser Asp Glu Glu Leu Val
145                 150                 155                 160

Thr Thr Glu Arg Lys Thr Pro Arg Val Thr Gly Gly Gly Ala Met Ala
                165                 170                 175

Gly Ala Ser Thr Ser Ala Gly Arg Lys Arg Lys Ser Ala Ser Ser Ala
            180                 185                 190

Thr Ala Cys Thr Ser Gly Val Met Thr Arg Gly Arg Leu Lys Ala Glu
        195                 200                 205

Ser Thr Val Ala Pro Glu Glu Asp Thr Asp Glu Asp Ser Asp Asn Glu
    210                 215                 220

Ile His Asn Pro Ala Val Phe Thr Trp Pro Pro Trp Gln Ala Gly Ile
225                 230                 235                 240

Leu Ala Arg Asn Leu Val Pro Met Val Ala Thr Val Gln Gly Gln Asn
                245                 250                 255

Leu Lys Tyr Gln Glu Phe Phe Trp Asp Ala Asn Asp Ile Tyr Arg Ile
            260                 265                 270

Phe Ala Glu Leu Gly Ser Leu Gln Glu Phe Met Glu Ala Gly Ile Thr
        275                 280                 285

Gly Thr Trp Tyr Asn Gln Leu Gly Ser Thr Phe Ile Val Thr Ala Gly
    290                 295                 300

Ala Asp Gly Ala Leu Thr Gly Thr Tyr Glu Ser Ala Val Gly Asn Ala
305                 310                 315                 320

Glu Ser Arg Tyr Val Leu Thr Gly Arg Tyr Asp Ser Ala Pro Ala Thr
                325                 330                 335

Asp Gly Ser Gly Thr Ala Leu Gly Trp Thr Val Ala Trp Lys Asn Asn
            340                 345                 350

Tyr Arg Asn Ala His Ser Ala Thr Thr Trp Ser Gly Gln Tyr Val Gly
        355                 360                 365

Gly Ala Glu Ala Arg Ile Asn Thr Gln Trp Leu Leu Thr Ser Gly Thr
    370                 375                 380

Thr Glu Ala Asn Ala Trp Lys Ser Thr Leu Val Gly His Asp Thr Phe
385                 390                 395                 400

Thr Lys Val Lys Pro Ser Ala Ala Ser Leu Glu
                405                 410

<210>  39
<211>  231
<212>  PRT
<213>  Artificial

<220>
<223>  Fusion polypeptide: E7-SA (pET28b-E7-SA)

<400>  39

Met Asp Met His Gly Asp Thr Pro Thr Leu His Glu Tyr Met Leu Asp
1               5                   10                  15

Leu Gln Pro Glu Thr Thr Asp Leu Tyr Cys Tyr Glu Gln Leu Asn Asp
            20                  25                  30

Ser Ser Glu Glu Glu Asp Glu Ile Asp Gly Pro Ala Gly Gln Ala Glu
        35                  40                  45

Pro Asp Arg Ala His Tyr Asn Ile Val Thr Phe Cys Cys Lys Cys Asp
    50                  55                  60

Ser Thr Leu Arg Leu Cys Val Gln Ser Thr His Val Asp Ile Arg Thr
65                  70                  75                  80

Leu Glu Asp Leu Leu Met Gly Thr Leu Gly Ile Val Cys Pro Ile Cys
                85                  90                  95

Ser Gln Lys Pro Glu Phe Met Glu Ala Gly Ile Thr Gly Thr Trp Tyr
            100                 105                 110

Asn Gln Leu Gly Ser Thr Phe Ile Val Thr Ala Gly Ala Asp Gly Ala
        115                 120                 125

Leu Thr Gly Thr Tyr Glu Ser Ala Val Gly Asn Ala Glu Ser Arg Tyr
    130                 135                 140

Val Leu Thr Gly Arg Tyr Asp Ser Ala Pro Ala Thr Asp Gly Ser Gly
145                 150                 155                 160

Thr Ala Leu Gly Trp Thr Val Ala Trp Lys Asn Asn Tyr Arg Asn Ala
                165                 170                 175

His Ser Ala Thr Thr Trp Ser Gly Gln Tyr Val Gly Gly Ala Glu Ala
            180                 185                 190

Arg Ile Asn Thr Gln Trp Leu Leu Thr Ser Gly Thr Thr Glu Ala Asn
        195                 200                 205

Ala Trp Lys Ser Thr Leu Val Gly His Asp Thr Phe Thr Lys Val Lys
    210                 215                 220

Pro Ser Ala Ala Ser Leu Glu
225                 230

<210>  40
<211>  6267
<212>  DNA
<213>  Artificial

<220>
<223>  pET28b-CFP-10:Esat-6-SA (vector for co-expression of CFP-10 with
       EsatT6-SA)

<400>  40
gtgggccatc gccctgatag acggtttttc gccctttgac gttggagtcc acgttcttta      60

atagtggact cttgttccaa actggaacaa cactcaaccc tatctcggtc tattcttttg     120

atttataagg gattttgccg atttcggcct attggttaaa aaatgagctg atttaacaaa     180

aatttaacgc gaattttaac aaaatattaa cgtttacaat ttcaggtggc acttttcggg     240

gaaatgtgcg cggaacccct atttgtttat ttttctaaat acattcaaat atgtatccgc     300

tcatgaatta attcttagaa aaactcatcg agcatcaaat gaaactgcaa tttattcata     360

tcaggattat caataccata tttttgaaaa agccgtttct gtaatgaagg agaaaactca     420

ccgaggcagt tccataggat ggcaagatcc tggtatcggt ctgcgattcc gactcgtcca     480

acatcaatac aacctattaa tttcccctcg tcaaaaataa ggttatcaag tgagaaatca     540

ccatgagtga cgactgaatc cggtgagaat ggcaaaagtt tatgcatttc tttccagact     600

tgttcaacag gccagccatt acgctcgtca tcaaaatcac tcgcatcaac caaaccgtta     660

ttcattcgtg attgcgcctg agcgagacga aatacgcgat cgctgttaaa aggacaatta     720

caaacaggaa tcgaatgcaa ccggcgcagg aacactgcca gcgcatcaac aatattttca 780
cctgaatcag gatattcttc taatacctgg aatgctgttt tcccggggat cgcagtggtg 840
agtaaccatg catcatcagg agtacggata aaatgcttga tggtcggaag aggcataaat 900
tccgtcagcc agtttagtct gaccatctca tctgtaacat cattggcaac gctacctttg 960
ccatgtttca gaaacaactc tggcgcatcg ggcttcccat acaatcgata gattgtcgca 1020
cctgattgcc cgacattatc gcgagcccat ttatacccat ataaatcagc atccatgttg 1080
gaatttaatc gcggcctaga gcaagacgtt tcccgttgaa tatggctcat aacacccctt 1140
gtattactgt ttatgtaagc agacagtttt attgttcatg accaaaatcc cttaacgtga 1200
gttttcgttc cactgagcgt cagaccccgt agaaaagatc aaaggatctt cttgagatcc 1260
tttttttctg cgcgtaatct gctgcttgca aacaaaaaaa ccaccgctac cagcggtggt 1320
ttgtttgccg gatcaagagc taccaactct ttttccgaag gtaactggct tcagcagagc 1380
gcagatacca aatactgtcc ttctagtgta gccgtagtta ggccaccact tcaagaactc 1440
tgtagcaccg cctacatacc tcgctctgct aatcctgtta ccagtggctg ctgccagtgg 1500
cgataagtcg tgtcttaccg ggttggactc aagacgatag ttaccggata aggcgcagcg 1560
gtcgggctga acggggggtt cgtgcacaca gcccagcttg gagcgaacga cctacaccga 1620
actgagatac ctacagcgtg agctatgaga aagcgccacg cttcccgaag ggagaaaggc 1680
ggacaggtat ccggtaagcg gcagggtcgg aacaggagag cgcacgaggg agcttccagg 1740
gggaaacgcc tggtatcttt atagtcctgt cgggtttcgc cacctctgac ttgagcgtcg 1800
atttttgtga tgctcgtcag gggggcggag cctatggaaa aacgccagca acgcggcctt 1860
tttacggttc ctggcctttt gctggccttt tgctcacatg ttctttcctg cgttatcccc 1920
tgattctgtg gataaccgta ttaccgcctt tgagtgagct gataccgctc gccgcagccg 1980
aacgaccgag cgcagcgagt cagtgagcga ggaagcggaa gagcgcctga tgcggtattt 2040
tctccttacg catctgtgcg gtatttcaca ccgcatatat ggtgcactct cagtacaatc 2100
tgctctgatg ccgcatagtt aagccagtat acactccgct atcgctacgt gactgggtca 2160
tggctgcgcc ccgacacccg ccaacacccg ctgacgcgcc ctgacgggct tgtctgctcc 2220
cggcatccgc ttacagacaa gctgtgaccg tctccgggag ctgcatgtgt cagaggtttt 2280
caccgtcatc accgaaacgc gcgaggcagc tgcggtaaag ctcatcagcg tggtcgtgaa 2340
gcgattcaca gatgtctgcc tgttcatccg cgtccagctc gttgagtttc tccagaagcg 2400
ttaatgtctg gcttctgata aagcgggcca tgttaagggc ggttttttcc tgtttggtca 2460
ctgatgcctc cgtgtaaggg ggatttctgt tcatgggggt aatgataccg atgaaacgag 2520
agaggatgct cacgatacgg gttactgatg atgaacatgc ccggttactg gaacgttgtg 2580
agggtaaaca actggcggta tggatgcggc gggaccagag aaaaatcact cagggtcaat 2640
gccagcgctt cgttaataca gatgtaggtg ttccacaggg tagccagcag catcctgcga 2700
tgcagatccg gaacataatg gtgcagggcg ctgacttccg cgtttccaga ctttacgaaa 2760

cacggaaacc gaagaccatt catgttgttg ctcaggtcgc agacgttttg cagcagcagt 2820
cgcttcacgt tcgctcgcgt atcggtgatt cattctgcta accagtaagg caaccccgcc 2880
agcctagccg ggtcctcaac gacaggagca cgatcatgcg cacccgtggg gccgccatgc 2940
cggcgataat ggcctgcttc tcgccgaaac gtttggtggc gggaccagtg acgaaggctt 3000
gagcgagggc gtgcaagatt ccgaataccg caagcgacag gccgatcatc gtcgcgctcc 3060
agcgaaagcg gtcctcgccg aaaatgaccc agagcgctgc cggcacctgt cctacgagtt 3120
gcatgataaa gaagacagtc ataagtgcgg cgacgatagt catgccccgc gcccaccgga 3180
aggagctgac tgggttgaag gctctcaagg gcatcggtcg agatcccggt gcctaatgag 3240
tgagctaact tacattaatt gcgttgcgct cactgcccgc tttccagtcg ggaaacctgt 3300
cgtgccagct gcattaatga atcggccaac gcgcggggag aggcggtttg cgtattgggc 3360
gccagggtgg tttttctttt caccagtgag acgggcaaca gctgattgcc cttcaccgcc 3420
tggccctgag agagttgcag caagcggtcc acgctggttt gccccagcag gcgaaaatcc 3480
tgtttgatgg tggttaacgg cgggatataa catgagctgt cttcggtatc gtcgtatccc 3540
actaccgaga tatccgcacc aacgcgcagc ccggactcgg taatggcgcg cattgcgccc 3600
agcgccatct gatcgttggc aaccagcatc gcagtgggaa cgatgccctc attcagcatt 3660
tgcatggttt gttgaaaacc ggacatggca ctccagtcgc cttcccgttc cgctatcggc 3720
tgaatttgat tgcgagtgag atatttatgc cagccagcca gacgcagacg cgccgagaca 3780
gaacttaatg ggcccgctaa cagcgcgatt tgctggtgac ccaatgcgac cagatgctcc 3840
acgcccagtc gcgtaccgtc ttcatgggag aaaataatac tgttgatggg tgtctggtca 3900
gagacatcaa gaaataacgc cggaacatta gtgcaggcag cttccacagc aatggcatcc 3960
tggtcatcca gcggatagtt aatgatcagc ccactgacgc gttgcgcgag aagattgtgc 4020
accgccgctt tacaggcttc gacgccgctt cgttctacca tcgacaccac cacgctggca 4080
cccagttgat cggcgcgaga tttaatcgcc gcgacaattt gcgacggcgc gtgcagggcc 4140
agactggagg tggcaacgcc aatcagcaac gactgtttgc ccgccagttg ttgtgccacg 4200
cggttgggaa tgtaattcag ctccgccatc gccgcttcca ctttttcccg cgttttcgca 4260
gaaacgtggc tggcctggtt caccacgcgg gaaacggtct gataagagac accggcatac 4320
tctgcgacat cgtataacgt tactggtttc acattcacca ccctgaattg actctcttcc 4380
gggcgctatc atgccatacc gcgaaaggtt ttgcgccatt cgatggtgtc cgggatctcg 4440
acgctctccc ttatgcgact cctgcattag gaagcagccc agtagtaggt tgaggccgtt 4500
gagcaccgcc gccgcaagga atggtgcatg caaggagatg gcgcccaaca gtcccccggc 4560
cacggggcct gccaccatac ccacgccgaa acaagcgctc atgagcccga agtggcgagc 4620
ccgatcttcc ccatcggtga tgtcggcgat ataggcgcca gcaaccgcac ctgtggcgcc 4680
ggtgatgccg gccacgatgc gtccggcgta gaggatcgag atctcgatcc cgcgaaatta 4740
atacgactca ctatagggga attgtgagcg gataacaatt cccctctaga aataattttg 4800

tttaacttta agaaggagat ataccatggc agagatgaag accgatgccg ctaccctcgc 4860
gcaggaggca ggtaatttcg agcggatctc cggcgacctg aaaacccaga tcgaccaggt 4920
ggagtcgacg gcaggttcgt tgcagggcca gtggcgcggc gcggcgggga cggccgccca 4980
ggccgcggtg gtgcgcttcc aagaagcagc caataagcag aagcaggaac tcgacgagat 5040
ctcgacgaat attcgtcagg ccggcgtcca atactcgagg gccgacgagg agcagcagca 5100
ggcgctgtcc tcgcaaatgg gcttctgacc cgctaatacg aaaagaggat ctaggagata 5160
taccatgaca gagcagcagt ggaatttcgc gggtatcgag gccgcggcaa gcgcaatcca 5220
gggaaatgtc acgtccattc attccctcct tgacgagggg aagcagtccc tgaccaagct 5280
cgcagcggcc tggggcggta gcggttcgga ggcgtaccag ggtgtccagc aaaaatggga 5340
cgccacggct accgagctga acaacgcgct gcagaacctg gcgcggacga tcagcgaagc 5400
cggtcaggca atggcttcga ccgaaggcaa cgtcactggg atgttcgcag aattcatgga 5460
agctggtatt accggcacct ggtataatca gctgggcagc acctttattg tgaccgcggg 5520
cgcggatggt gcgctgaccg gtacctatga aagcgcggtg ggtaacgcgg aaagccgtta 5580
tgtgctgacc ggccgttatg atagcgcgcc ggcgaccgat ggcagcggca ccgcgctggg 5640
ctggaccgtg gcgtggaaaa acaactatcg caacgcgcat agcgcgacca cctggagcgg 5700
tcagtatgtg ggcggcgcgg aagcgcgtat taatacccag tggctgctga ccagcggcac 5760
caccgaagcg aacgcgtgga aaagcaccct ggtgggccat gataccttta ccaaagtgaa 5820
accgagcgcg gcgagcctcg agtaaaagct tgcggccgca ctcgagcacc accaccacca 5880
ccactgagat ccggctgcta acaaagcccg aaaggaagct gagttggctg ctgccaccgc 5940
tgagcaataa ctagcataac cccttggggc ctctaaacgg gtcttgaggg gttttttgct 6000
gaaaggagga actatatccg gattggcgaa tgggacgcgc cctgtagcgg cgcattaagc 6060
gcggcgggtg tggtggttac gcgcagcgtg accgctacac ttgccagcgc cctagcgccc 6120
gctcctttcg ctttcttccc ttcctttctc gccacgttcg ccggctttcc ccgtcaagct 6180
ctaaatcggg ggctcccttt agggttccga tttagtgctt tacggcacct cgaccccaaa 6240
aaacttgatt agggtgaggt tcacgta 6267

<210> 41
<211> 126
<212> PRT
<213> Artificial

<220>
<223> Residues 14-139 of the mature, full-length SA protein

<400> 41

Glu Ala Gly Ile Thr Gly Thr Trp Tyr Asn Gln Leu Gly Ser Thr Phe
1               5                   10                  15

Ile Val Thr Ala Gly Ala Asp Gly Ala Leu Thr Gly Thr Tyr Glu Ser
            20                  25                  30

Ala Val Gly Asn Ala Glu Ser Arg Tyr Val Leu Thr Gly Arg Tyr Asp
        35                  40                  45

Ser Ala Pro Ala Thr Asp Gly Ser Gly Thr Ala Leu Gly Trp Thr Val
    50                  55                  60

Ala Trp Lys Asn Asn Tyr Arg Asn Ala His Ser Ala Thr Thr Trp Ser
65                  70                  75                  80

Gly Gln Tyr Val Gly Gly Ala Glu Ala Arg Ile Asn Thr Gln Trp Leu
                85                  90                  95

Leu Thr Ser Gly Thr Thr Glu Ala Asn Ala Trp Lys Ser Thr Leu Val
            100                 105                 110

Gly His Asp Thr Phe Thr Lys Val Lys Pro Ser Ala Ala Ser
        115                 120                 125

## Description

The present invention provides an innovative versatile system, which allows delivery of one or several antigens or biologically active molecules onto and/or into subset of cells and in particular onto and/or into dendritic cells (DC) or subsets of DC.

This two-component system combines (i) a fusion polypeptide comprising a streptavidin or avidin polypeptide and effector molecule(s), which is capable of binding biotin molecules, and (ii) biotinylated targeting molecules, which are capable of targeting subset(s) of cells and/or cell surface molecules(s).

Using this system, the invention allows for efficient and specific delivery of effector molecules, in particular antigens, onto and/or into subset(s) of cells which have been targeted via biotinylated targeting molecules.

The invention is more particularly directed to a combination of compounds and in particular to a composition comprising the aforementioned components (i) and (ii).

The combination and the composition of the invention are suitable for use for diagnosing or immunomonitoring a disease in a mammal or for use in prophylactic or curative treatment and especially in vaccination and in therapy including in immunotherapy.

The invention also relates to the use of a fusion polypeptide as defined above, in combination with biotinylated targeting molecule as defined above, for targeting, *in vivo, in vitro* or *ex vivo,* of one or several effector molecule(s) to subset(s) of cells and/or cell surface molecules(s).

The invention also relates to a method for the production of a fusion polypeptide of the invention and to a kit for a diagnostic test of a disease in a mammal, for immunomonitoring a disease in a mammal or for the prevention or treatment of a disease in a mammal.

Hence, a first object of the invention is directed to a combination of compounds (or kit-of-parts) which comprises or consists of at least two components:
(i) a fusion polypeptide comprising or consisting of:
   - a streptavidin (SA) or avidin polypeptide; and
   - one or several effector molecule(s),
      wherein said fusion polypeptide retains the property of SA and avidin proteins to bind biotin; and
(ii) one or several biotinylated targeting molecule(s), which is(are) capable of specifically interacting with subset(s) of cells and/or cell surface molecule(s).

According to the invention, components (i) and (ii) are present either in distinct compositions or in the same (*i.e.,* in a single) composition.

Hence, in a particular embodiment, the invention also relates to a composition comprising or consisting of components (i) and (ii).

By "composition", it is meant herein in particular a pharmaceutical or an immunological composition.

By "fusion polypeptide" it is meant herein that the SA or avidin polypeptide is genetically fused to the polypeptidic structure of one or several effector molecule(s). One or several (in particular 2, 3, 4, 5 or more) effector molecule(s) can be fused at the N-terminal end, at the C-terminal end or at both ends of the SA or avidin polypeptide.

In a particular embodiment of the invention, this fusion polypeptide is the expression product of a recombinant polynucleotide, which can be expressed in a cell, for example an *Escherichia coli* (*E. coli*) cell, which is transformed (as a result of recombination) to comprise said recombinant polynucleotide or a plasmid or a recombinant vector comprising said polynucleotide.

In a particular embodiment of the invention, the fusion polypeptide also comprises one or several linker(s) (or spacer(s)), in particular one or several flexible linker(s), which is(are) located, for example, between the SA or avidin polypeptide (more specifically, between a SA or avidin monomer) and an effector molecule. A "linker" as used herein consists in a polypeptide product having an amino acid sequence of at least 2 amino acid residues, preferably at least 4 residues, for example 4, 5, 6, 7, 8, 9, 10, 11, 12, 13, 14, 15, 16, 17, 18, 19 or 20 residues.

By "SA or avidin polypeptide", it is meant herein a full length native SA or avidin protein, a variant thereof or a derivative of this native protein or variant thereof, which variant and derivative retain the property of the native protein to bind biotin, and in particular to selectively bind biotin.

In a particular embodiment of the invention, a SA polypeptide is used to build the fusion polypeptide. This SA polypeptide is for example derived from the SA protein obtainable from *Streptomyces avidinii.*

In a particular embodiment of the invention, a "variant" of a SA or avidin protein consists in an amino acid sequence having at least 50%, preferably at least 70% and more preferably at least 90% identity with the sequence of a native full length SA or avidin protein, for example SA from *Steptomyces avidinii* (SEQ ID NO.: 1).

In a particular embodiment of the invention, a "variant" of a SA or avidin protein consists in a polypeptide variant having an amino acid sequence which differs from that of the full length SA or avidin protein (for example from that of SA from *Steptomyces avidinii*) by insertion, deletion and/or substitution, preferably by insertion and/or substitution of one or several amino acid residues, for example 1, 2, 3, 4, 5, or 6 amino acid residues. Hence, a "variant" includes a portion of a native full-length SA or avidin protein.

In a particular embodiment of the invention, said variant is a fragment of the full-length SA or avidin protein, which retains the capacity of binding biotin. The invention especially relates in this respect, to such a variant which is a fragment devoid of the N-terminal and C-terminal regions of the native full-length SA or avidin protein.

In a particular embodiment of the invention, the SA polypeptide present in the polypeptide fusion is the portion called natural core, which ranges from amino acid residues 13 to 139 or 14 to 139 in the SA protein from *Steptomyces avidinii* (SEQ ID NO.: 2 and 41 respectively).

Alternatively, a variant consisting in a sequence having at least 70%, preferably at least 80% and more preferably at least 90% or 95% identity with the amino acid sequence of this natural core and retaining the property to bind biotin can be used. For example, a polypeptide comprising or consisting of polypeptides stv-25 or stv-13 (Sano et al., 1995), of sequence SEQ ID NO.: 3 and SEQ ID NO.: 4 respectively, can be used as SA polypeptide in the fusion polypeptide of the invention.

A "derivative" of a SA or avidin polypeptide as used herein designates a polypeptide modified chemically, for example by deglycosylation, or by PEGylation of a SA or avidin full-length protein or a variant thereof. An example of a deglycosylated version of a avidin polypeptide is the protein called neutravidin.

Preferably, the "variant" and "derivative" of a SA or avidin native protein also retain the property of these proteins to form a tetramer.

Indeed, in a particular embodiment of the invention, the fusion polypeptide is in the form of a tetramer. This tetrameric fusion polypeptide can be a homotetramer or a heterotetramer, *i.e.,* comprises or consists of either four identical monomers or two or more (two, three or four) different monomers respectively. Every monomer of this tetramer (homotetramer or heterotetramer) comprises at least a monomer of the SA or avidin polypeptide.

Hence, when the fusion polypeptide of the invention is in the form of a heterotetramer, at least one monomer of this tetramer comprises or consists of (i) a monomer of the SA or avidin polypeptide and (ii) one or several effector molecule(s). The other monomers of the tetramer then comprise or consist of a monomer of the SA or avidin polypeptide, and optionally one or several effector molecule(s).

In a particular embodiment of the invention, each monomer of the tetrameric fusion polypeptide of the invention (homotetramer or heterotetramer) comprises or consists of both a monomer of the SA or avidin polypeptide and one or several effector molecule(s) (preferably several effector molecules).

In a particular embodiment of the invention, each monomers of a tetramer comprise the same effector molecule(s).

In another particular embodiment of the invention, monomers of the tetramer (at least two monomers of the tetramer) have a different content in effector molecule(s).

When the fusion polypeptide of the invention is in the form of a heterotetramer, at least one monomer of this tetramer (*i.e.,* one, two, three or four monomer(s) of this tetramer) retains the property of SA and avidin proteins to bind biotin. The other monomers of this tetramer can retain or not the property of SA and avidin proteins to bind biotin. Hence, a tetrameric fusion polypeptide of the invention can have one, two, three or four functional (or active) biotin binding subunits.

In a particular embodiment of the invention, the fusion polypeptide is in the form of a heterotetramer, wherein one, two or three monomers of this tetramer are non-functional, i.e., do not retain the property of SA and avidin proteins to bind biotin, for example due to one or several mutation(s) in the SA or avidin polypeptide. For example, the fusion polypeptide can include a SA or avidin tetramer which comprises only one functional biotin subunit that retains the property of SA or avidin to bind biotin, and in particular a monovalent SA tetramer as disclosed in Howarth et al., 2006.

Alternatively, in a particular embodiment of the invention, the fusion polypeptide of the invention is in the form of a monomer, which comprises or consists of a monomeric SA or avidin polypeptide and one or several effector molecule(s), preferably several effector molecules. This monomeric SA or avidin polypeptide can be for example a variant of a SA or avidin wild-type protein, which has an increased biotin binding affinity, an in particular a monomeric SA polypeptide as disclosed in Wu and Wong, 2005.

By "effector molecule", it is meant herein a molecule which has a biologically activity (*i.e*., a biologically active molecule) which comprises or consists of one or several polypeptidic structures, *i.e.,* a biologically active polypeptidic molecule. Said polypeptide may especially be chosen for its properties for the purpose of preparing prophylactic product or in a therapeutic product i.e., may have a prophylactic or a therapeutic activity, or may enhance a prophylactic or therapeutic activity.

In particular embodiments of the invention, the effector molecule(s) or some of the effector molecule(s) is(are) selected in the group comprising: polypeptides including peptides, glycopeptides and lipopeptides.

Additionally or alternatively, one or several elements chosen in the group of lipids, sugars, nucleic acids (in particular DNAs and RNAs and for example cDNA or siRNA), chemical moieties, and chemical molecules, for example radioelements, dyes or immunostimulant, for example Poly I:C (polyinosinic:polycytidylic acid or polyinosinic-polycytidylic acid sodium salt), can be grafted onto the fusion polypeptide and in particular onto one or several effector molecule(s) present in the fusion polypeptide. This(these) element(s) can be attached onto the fusion polypeptide either by chemical coupling, or by adding into the fusion polypeptide an aptamer or another recombinant ligand that would bind (especially with high affinity) said element(s). Said element(s) can be grafted for example on the polypeptidic structure of an effector molecule.

In particular embodiments of the invention, the effector molecule(s) or some of the effector molecule(s) is(are) a polypeptide and especially a peptide.

In a particular embodiment of the invention, the "effector molecule" does not interfere with the folding of the SA or avidin polypeptide, and in particular with tetramerization of these polypeptides. Alternatively, in case an effector molecule which interferes with tetramerization of the SA or avidin polypeptide is used, it is possible to insert, between the SA or avidin polypeptide and said effector molecule, one or several linker(s), in particular one or several flexible linker(s), in order that the SA or avidin polypeptide still forms a tetramer despite the presence of this effector molecule in the fusion polypeptide.

In a specific embodiment of the invention, the effector molecule(s) or some of the effector molecule(s) comprise or consist of 2 to 1000, preferably 5-800, 5 to 500, 5 to 200, 5 to 100, 8 to 50, 5 to 25, 5 to 20 or 8 to 16 amino acid residues.

In a particular embodiment of the invention, the effector molecule(s) or at least some of the effector molecule(s) is(are) chosen among the following group:
- polypeptides suitable for eliciting an immune response (also referred to as "immunogens") in particular a polypeptide which comprises or consists of an epitope or a plurality of epitopes, an antigen or a fragment thereof comprising at least one epitope;
- a cytokine, a polypeptidic drug, a toxin, a toxoid, an enzyme, an oncoprotein, a protein which regulates cell cycle or metabolism, a fluororescent polypeptidic marker, a polypeptide binding a nucleic acid, an aptamer, or a recombinant ligand capable of binding biologically active molecules, for example molecules capable of modulating activity on cells of the immune system and in particular on dendritic cells or on lymphocytes (for example B or T lymphocytes), such as cytokines.

As used herein, the term "epitope" refers to a polypeptide and especially a peptide that can elicit an immune response, when presented in appropriate conditions to the immune system of a host. In particular, such an epitope can comprise or consist of a stretch of 6, 7, 8, 9, 10, 11, 12, 13, 14, 15, 16, 17, 18, 19 or 20 amino acid residues.

The polypeptidic molecule suitable for eliciting an immune response is especially one eliciting a T-cell immune response, including a CTL response or a T helper response. The polypeptidic molecule suitable for eliciting an immune response can also be one eliciting a B-cell immune response.

In specific embodiments, the immunogen is derived from an allergen, a toxin, a tumor cell, or an infectious agent, in particular a bacteria, a parasite, a fungus or a virus.

In a particular embodiment of the invention, the effector molecule(s) or at least some effector molecule(s) comprise or consist of an antigen selected from the group consisting of a Chlamydia antigen, a Mycoplasma antigen, a Mycobacteria antigen (for example, an antigen from *Mycobacterium tuberculosis or Mycobacterium leprae),* a Plasmodia antigen (for example, an antigen from *Plasmodium berghei, Plasmodium vivax* or *Plasmodium falciparum*)*,* a hepatitis virus antigen, a poliovirus antigen, an HIV virus antigen (for example, a HIV protein), a HPV virus antigen, especially an antigen of HPV16 or HPV18 (for example, a E7 antigen of a HPV virus, especially the E7 antigen of HPV16), a CMV virus antigen (for example, the pp65 protein), an influenza virus antigen, a choriomeningitis virus antigen, or a tumor-associated antigen, or comprise or consist of a part of an amino acid sequence of any these antigens which comprises at least one epitope.

In a particular embodiment of the invention, the effector molecule(s) or at least some effector molecule(s) are from *Mycobacterium tuberculosis.* For example, they can comprise or consist of an amino acid sequence chosen from the ones of the following proteins:
- the ESAT-6 protein family (ESX family; Brodin P. et al., 2004; PMID: 15488391), in particular the proteins ESAT-6 (Rv3875; Sørensen et al., 1995; PMID: 7729876) ), CFP-10, (Rv3874; Berthet et al. 1998; PMID: 9846755), TB10.4 (Rv0288; Hervas-Stubbs S et al., 2006; PMID: 16714570) or TB10.3 (Rv3019c)
- the proteins Ag85A (Rv 3804 c (Ag85A)) and Ag85B (Rv1886c (Ag85B)) (Denis O, et al., 1998; PMID: 9529077); and
- proteins of PE and PPE family (Bottai D and Brosch R, 2009; PMID: 19602151)
or comprise or consist of a part of an amino acid sequence of any these proteins to the extent that it comprises at least one epitope.

In a particular embodiment of the invention, the effector molecule(s) or at least some effector molecule(s) are a tumor associated antigen (TAA). Tumor-associated antigens have been characterized for a number of tumors such as for example: Melanoma, especially metastatic melanoma; Lung carcinoma; Head & neck carcinoma; cervical carcinoma, Esophageal carcinoma; Bladder carcinoma, especially infiltrating Bladder carcinoma; Prostate carcinoma; Breast carcinoma; Colorectal carcinoma; Renal cell carcinoma; Sarcoma; Leukemia; Myeloma. For these various histological types of cancers, it has been shown that antigenic peptides are specifically expressed on tumor samples and are recognized by T cells, especially by CD8⁺ T cells or CD4⁺T cells.

A review of peptides found as tumor-associated antigens in these types of tumors is made by Van der Bruggen P. et al (Immunological Reviews, 2002, vol 188:51-64). Especially, the disclosure of the peptides contained in table 3 of said review is referred to herein as providing examples of such tumor-associated antigens and said table 3 is incorporated by reference to the present application.

The following antigens are cited as examples of tumor-associated antigens recognized by T cells, according to Kawakami Y. et al (Cancer Sci, October 2004, vol.95, no. 10, p784-791) that also provides methods for screening these antigens or further one: antigens shared by various cancers, including MAGE (especially in Melanoma), NY-ESO-1, Her2/neu, WT1, Survivin, hTERT, CEA, AFP, SART3, GnT-V, antigens specific for some particular cancers such as βbeta-catenin, CDK4, MART-2, MUM3, gp100, MART-1, tyrosinase for Melanoma; bcr-abl, TEL-AML1 for Leukemia; PSA, PAP, PSM, PSMA for prostate cancer; Proteinase 3 for myelogenous leukemia; MUC-1 for breast, ovarian or pancreas cancers; EBV-EBNA, HTLV-1 tax for lymphoma, ATL or cervical cancer; mutated HLA-A2 for Renal cell cancer; HA1 for leukemia/lymphoma. Tumor-associated antigens in animals have also been described such as Cycline D1 and Cycline D2 in tumors affecting cats or dogs.

Tumor-associated antigens recognized by T cells have also been disclosed in Novellino L. et al (Immunol Immunother 2004, 54:187-207).

More generally, TAA of interest in the present invention are those corresponding to mutated antigens, or to antigens that are overexpressed on tumor cells, to shared antigens, tissue-specific differenciation antigens or to viral antigens.

In a particular embodiment of the invention, the tumor-associated antigen is an antigen of papillomavirus (HPV) or is tyrosinase.

In a particular embodiment of the invention, the fusion polypeptide further comprises one or several ligand(s), in particular one or several recombinant ligand(s), for example one or several protein scaffold(s). Especially, protein scaffolds allowing binding and targeting of co-receptors of T cells or cytokines (for example IL-2 or IFNy), or delivery of nucleic acids (in particular RNAs and DNAs and for example cDNAs or siRNA) or of adjuvant molecules (for example CpG) could be used.

By "targeting molecule(s)" it is meant herein a molecule which is capable of targeting subset(s) of cells and/or cell surface molecule(s), and in particular capable of specifically interacting with targeted subset(s) of cells and/or cell surface molecule(s) and especially binding to such cells and/or cell surface molecule(s).

In a particular embodiment of the invention, the biotinylated targeting molecule(s) enable targeting of subset(s) of cells by interacting with surface molecule(s) of these cells.

By "subset(s) of cells", it is meant herein in particular antigen presenting cells (APC) and/or subset(s) of APC. In a particular embodiment of the invention, the terms "subset(s) of cells" and "APC" designate dendritic cells (DC) or subset(s) of DC and/or B lymphocytes or subset(s) of B lymphocytes. In a more particular embodiment of the invention, the term "subset(s) of cells" and "APC" designate DC or subset(s) of DC.

By "cell surface molecule(s)", it is meant herein any molecule which is expressed at the cell surface, and in particular cell surface receptor(s) and/or toll-like receptor(s) (TLR). These molecules include in particular the ones which are expressed at the surface of APC, and in particular at the surface of DC and/or B lymphocytes and/or T lymphocytes (more preferably at the surface of DC).

Hence, by "cell surface receptor(s)", it is meant herein in particular APC surface receptor(s), preferably DC and/or B lymphocytes and/or T lymphocytes surface receptor(s) and more preferably DC surface receptor(s).

DC subset(s) can be in particular chosen among the following group: plasmacytoid DC, blood-derived lymphoid tissue resident DC, peripheral migratory DC, monocyte-derived inflammatory DC.

In a particular embodiment of the invention, a biotinylated targeting molecule comprises or consists of one or several polypeptide especially peptidic structure(s) wherein the meaning of "polypeptide" is as disclosed herein.

In a particular embodiment of the invention, a biotinylated targeting molecule is a polypeptide.

Different biotinylated targeting molecule(s) can be used in the invention and in particular in the combination or the composition of the invention.

In a particular embodiment of the invention, the biotinylated targeting molecule(s) are capable of specifically targeting cells, and in particular of specifically interacting with, cells or subset of cells (in particular DC or B lymphocytes or subset(s) of DC or B lymphocytes), which induce a CD4+ T-cell immune response and/or a CD8+ T-cell immune response or which induce essentially a CD4+ or a CD8+ T-cell immune response.

In a particular embodiment of the invention, the biotinylated targeting molecule(s) or at least some of the biotinylated targeting molecule(s) present in the combination or the composition of the invention is(are) capable of specifically interacting with one or several cell surface receptor(s) chosen from the following group:
- major histocompatibility complex (MHC) molecules, and in particular MHC class I molecules (MHC-I) and more preferably MHC class II molecules (MHC-II);
- C-type lectins, in particular:
   - members of the mannose receptor family, for example CD205 endocytic C-type lectins (or DEC205),
   - members of the asialoglycoprotein receptor family, for example CD207 (Langerin, Clec4K), or CD209 (DC-Specific ICAM3-Grabbing Non-integrin, DC-SIGN),
   - members of the DC Immunoreceptor (DCIR) subfamily of asialoglycoproteoin receptor, for example DCIR-2 (Clec4A),
   - DC, NK lectin group receptor-1 (DNGR-1; also known as Clec 9A), or
   - Clec12A.
- PDCA-1;
- Integrins, for example β2 integrins, or α and β integrin subunits, for example CD11b and CD11c; and
- Dendritic cell inhibitory receptor 2 (DCIR-2).

Receptor CD207 enables to target in particular DC of the dermis and epidermis and in epithelium lining the human airways, and is thus particularly appropriate for use for example in the prevention or treatment tuberculosis.

An example of an antibody specific to the C-type lectin endocytic receptor CD205 is the monoclonal antibody NLDC-145 (Celldex Therapeutics; Needham, USA).

In a particular embodiment of the invention, the biotinylated targeting molecule(s) are chosen from:
- biotinylated antibodies or biotinylated antibody-like molecules; and
- biotinylated ligands, in particular biotinylated aptamers and protein scaffold ligands or biotinylated non-proteinaceous ligands,
- biotinylated polysaccharides, biotinylated nucleic acids (in particular DNAs or RNAs) or biotinylated lipids,
which biotinylated antibodies, antibody-like molecules, ligands, polysaccharides, nucleic acids or lipids are capable of specifically interacting with subset(s) of cells and/or cell surface molecule(s) as defined herein.

By "antibodies" it is meant herein any type of antibody and in particular monoclonal antibodies, which are specific to subset(s) of cells and/or cell surface molecule(s), as defined herein or antibody-like molecules.

The term "monoclonal antibody" encompasses:
- monospecific antibodies *i.e.,* molecules wherein the two antigen binding sites (domains formed by the VH regions or by the interaction of the VH and VL regions, and interacting with the immunogen) recognize and bind the same immunogen.
- trifunctional antibodies *i.e.,* bispecific molecules as disclosed hereinafter and further having an Fc region (CH2 and CH3 domains) of any origin, particularly of human origin.

The term "antibody-like molecule" refers to a molecule having all or part of the variable heavy and light domains of an antibody, but devoid of the conventional structure of a four-chain antibody, and conserving nevertheless the capacity to interact with and bind an immunogen. In a particular embodiment of the invention, an antibody-like molecule is a fragment of an antibody and in particular comprises the CDR1, CDR2 and CDR3 regions of the VL and/or VH domains of a full length antibody.

The term "antibody-like molecule" encompasses in particular:
- scFv, *i.e.,* a VH domain genetically associated (optionally via a linker) to a VL domain, as well as molecules comprising at least one scFv, such as Bis ScFv molecules (two ScFv having same or different antigen binding site(s) linked together (optionally via a linker));
- diabody molecules *i.e.,* the heavy chain variable domain derived from a first antibody (a first VH domain (VH1)) connected to the light chain variable domain derived from a second antibody (VL2) on the same polypeptide chain (VH1-VL2) connected by a peptide linker that is too short to allow pairing between the two domains on the same chain, interacting with the heavy chain variable domain of derived from a second antibody (VH2) connected to the light chain variable domain derived from a first antibody (a first VL domain (VL1)) on the same polypeptide chain (VH2-VL1), wherein VL1 and VH1 form a first antigen-binding site and VL2 and VH2 form a second antigen binding site (recognizing and/or binding a similar or a different immunogen from the first binding antigen binding site);
- bispecific molecules *i.e.,* molecules in which the two antigen binding sites of a Fab₂ fragment (variable and CH1 domains of light and heavy chains) interact with different immunogens).
- trispecific molecules *i.e.,* molecules in which the two antigen binding sites of a Fab₃ fragment (variable and CH1 domains of light and heavy chains) interact with different immunogens);
- VHH (VH domain of functional antibodies naturally devoid of light chains) *i.e.,* a VH domain which has the capacity to interact as such with an immunogen, without the presence of a variable light domain (VL).
- functional fragments of an antibody or an antibody-like molecule as defined herein, provided that these fragments retain the ability to specifically interact with subset(s) of cells and/or cell surface molecule(s). These fragments include Fv fragments (non-covalent association of the VH and VL domains of the invention) and Fab fragments.

In a particular embodiment of the invention, the combination or the composition further comprises one or several biotinylated, non-targeting molecule(s), which can be for example chosen from the following group: biotinylated immunogens as defined herein, biotinylated protoxins, biotinylated nucleic acids (in particular RNAs, DNAs or cDNAs), biotinylated adjuvant molecules and biotinylated cytokines (for example IL-2, IL-10, IL-12, IL-17, IL-23, TNFa or IFNy).

These biotinylated, non-targeting molecule(s), as well as the biotinylated targeting molecule(s) and the effector molecule(s) used to carry out the invention can be humanized, in particular for use *in vivo,* in a human host, or ex *vivo,* on a sample of human cells.

In a particular embodiment of the invention,
- a fusion polypeptide comprising or consisting in a SA polypeptide and at least one effector molecule which is an immunogen (as defined herein),
- is used in conjunction with biotinylated targeting molecules which are chosen from biotinylated antibodies (in particular biotinylated monoclonal antibodies), scFv, diabody molecules, aptamers, and recombinant ligands, such as protein scaffolds.

In a particular embodiment of the invention, the composition of the invention comprises or consists in a complex formed between the fusion polypeptide and biotinylated targeting molecule(s). Hence, in a particular embodiment, the invention relates to a composition (or complex) in which the fusion polypeptide is complexed to biotinylated targeting molecule(s) present in the composition, and optionally to biotinylated, non-targeting molecule(s) as defined herein.

By "complex", it is meant herein that the fusion polypeptide associates with biotinylated molecule(s) (in particular with biotinylated targeting molecule(s) and, when present, with biotinylated non-targeting molecule(s)) via non-covalent interactions that occur between the SA or avidin polypeptide and the biotin moiety of biotinylated molecule(s) present in the composition of the invention. This complex can include one, two, three or four biotins molecules, and in particular one, two, three or four biotinylated targeting molecule(s) as defined herein.

In a particular embodiment of the invention, this complex includes at least one biotinylated molecule(s) as defined herein, for example one, two, three or four biotinylated molecules.

In a particular embodiment of the invention, the fusion polypeptide and the complex comprising the fusion polypeptide are watersoluble.

In a particular embodiment of the invention, the effector molecule(s) or at least one effector molecule comprises or consists of the amino acid sequence of the ESAT-6 protein from *Mycobacterium tuberculosis.* In this case, a soluble fusion polypeptide is preferably produced by co-expression with the CFP-10 protein from *Mycobacterium tuberculosis,* in a cell, and in particular in a *E*. *coli* cell, for example an *E. coli* BL21 λDE3 cell, more preferably at 20°C.

In a particular embodiment of the invention, the composition of the invention or the composition(s) which are present in the combination of the invention is free or substantially free of biotinylated molecules (in particular of biotinylated targeting molecule(s)) not bound to the fusion polypeptide.

In a particular embodiment of the invention, the combination the invention and/or the composition of the invention further comprise(s) a pharmaceutically acceptable carrier, and optionally an adjuvant, an immunostimulant, for example Poly I:C, and/or another molecule which is therapeutically active or suitable to have a prophylactic effect, which is(are) combined with (*i.e*., present in the same composition as) the fusion polypeptide and/or the biotinylated targeting molecule(s), and/or, if present, biotinylated, non-targeting molecule(s).

In the context of the present invention a "therapeutically active molecule" can be one which may be beneficial to the condition of a human or non-human host to which it is administered. It is especially an active principle suitable for use in the manufacturing of a drug. It may be a compound suitable to either, potentiate increase or modulate the effect of an therapeutically active principle.

The invention is further directed to of a fusion polypeptide as defined herein,a composition of the invention, or a combination of the invention, for use in prophylaxis and/or in therapy, and in particular for use to elicit a T-cell immune response and/or a B-cell immune response *in vivo,* in a human or non-human host in need thereof.

The fusion polypeptide of the invention is in particular appropriate for use in prophylactic vaccination and/or in immunotherapy protocols or in diagnostic proliferative recall response assay, a T cell cytokine recall response assay, or a T cell cytotoxic recall response assay, respectively, detecting the presence of antigen-specific T cells. It can be used especially as priming reagent or as boosting reagent, *i.e.* after the host or the cells has(have) been primed with an effector molecule, for example using a construct comprising a CyaA protein and said effector molecule.

The invention relates in particular to a fusion polypeptide as defined herein or to a combination of the invention and in particular a composition of the invention, for use for the prevention or the treatment of a disease selected from neoplasia, cancers and infectious diseases selected from viral-, retroviral-, bacterial-, parasite- or fungal-induced diseases.

In a particular embodiment, the invention is intended for the induction of a protective anti-mycobacterial immunity, and in particular is intended for anti-tuberculosis vaccination.

In a particular embodiment, the invention is intended for the induction of a protective anti-viral immunity, and in particular is intended for vaccination against CMV.

The invention is also directed to the use of a fusion polypeptide as defined herein or a combination of the invention and in particular a composition of the invention, for the preparation of a vaccine or a medicament intended for the prevention and/or the treatment of a disease selected from neoplasia, cancers and infectious diseases selected from bacterial-, parasite-, fungus, viral- or retroviral-induced diseases especially resulting from infection with agents among those disclosed herein.

Another aspect of the invention relates to the use of a fusion polypeptide as defined herein or a combination of the invention and in particular a composition of the invention, in particular *in vitro, ex vivo* or *in vivo,* to select (or target) cell or subset(s) of cells, for use for diagnosing or immunomonitoring a disease in a mammal or for use in recall response assays from a sample (for example, from a sample of whole blood) from a human or a non-human mammal. Recall response assays can be performed for example in the case of following up the efficacy of an anti-tuberculosis or anti-CMV vaccine application.

The invention also relates to a fusion polypeptide as defined herein, for use *in vivo,* in combination with:
- one or several biotinylated targeting molecule(s) as defined herein; and
- optionally, one or several additional elements chosen from biotinylated, non-targeting molecule(s) as defined herein, a pharmaceutically acceptable carrier, an adjuvant, an immunostimulant (for example Poly I:C), and another therapeutically active molecule as defined herein,
for targeting of one or several effector molecule(s) (which are present in the fusion polypeptide) to subset(s) of cells and/or cell surface molecule(s) as defined herein, and in particular to DC, subset(s) of DC and/or DC surface molecule(s) (in particular DC surface receptor(s)).

By "recall assays", it is meant herein an *in vitro* stimulation of T lymphocytes present in a sample of PBMC or whole blood from a human or non-human host by one or several immunogens presented by APCs, to detect specific T cell responses.

The invention also relates to the use of a fusion polypeptide as defined herein, in combination with one or several biotinylated targeting molecule(s) as defined herein and, optionally, one or several additional elements as defined above, for targeting, in particular *in vitro* or *ex vivo,* one or several effector molecule(s) of the fusion polypeptide to subset(s) of cells (in particular to DC or subset(s) of DC) and/or to cell surface molecule(s), in particular to cell surface receptor(s) (including DC surface receptor(s)).

The invention enables direct *in vivo, ex vivo* or *in vitro,* targeting of cells or subset(s) of cells and in particular DC subset(s) through their specific surface markers (particularly surface receptors).

Indeed, the use of a fusion polypeptide as defined herein in combination (for example in a composition or a composition of the invention) with one or several biotinylated targeting molecule(s) as defined herein and optionally, one or several additional elements as defined above, enables the delivery (or transfer) of one or several effector molecule(s) to the surface of cells or of subset(s) of cells, which have been selectively targeted via the biotinylated targeting molecule(s). In a preferred embodiment of the invention, these effector molecule(s) are then delivered into target cells, for example via endocytosis. Optionally they can be processed for MHC (in particular MHC-I or II) molecule-mediated antigen presentation.

Hence the invention enables the delivery, *in vivo, ex vivo* or *in vitro,* of one or several effector molecule(s) (in particular one or several immunogen(s)) onto and/or into (preferably into) subset(s) of cells, by the use of individual biotinylated targeting molecule of specificity against subset(s) of cells and/or against surface receptor(s) expressed on said subset(s) of cells.

In a particular embodiment, the invention enables to raise, especially to prime or to boost, or to enhance antibody responses and/or T-cell responses, especially CD4+ and/or CD8+ systemic responses and/or mucosal T-cell responses, and/or lymphoproliferative responses, and/or to enhance resistance to tumor growth or to viral, parasitic or bacterial infection, in a cell or in a host (*in vitro, ex vivo* or *in vivo*)*.*

T-cell responses as used herein can include CD4+ and/or CD8+ T cells responses, and in particular Th1, Th2, Th17, Treg and/or CD8+ T-cell responses.

The invention is also directed to a method for targeting one or several effector molecule(s) to cells, subsets of cells and/or to cell surface molecule(s) as defined herein, said method comprising:
(i) contacting said cells with one or several biotinylated targeting molecule(s) as defined herein, and
(ii) contacting cells with a fusion polypeptide as defined herein; and
(iii) optionally, contacting said cells with one or several additional elements chosen from biotinylated, non-targeting molecule(s) as defined herein, a pharmaceutically acceptable carrier, an adjuvant, an immunostimulant (for example Poly I:C), and another therapeutically active molecule as defined herein.

These two or three steps can be replaced by a single step consisting of contacting the cells with a composition of the invention.

Alternatively, steps (i) and (ii) can be performed separately, the fusion polypeptide and the biotinylated targeting molecule(s) being present in different compositions.

This method, which can be used for the delivery of one or several effector molecule(s) onto and/or into (preferably into) cells or subsets of cells as defined herein, can be performed in particular *in vivo, in vitro* or *ex vivo.*

In a particular embodiment, this method is performed *in vitro* or *ex vivo,* and cells are contacted either first with one or several biotinylated targeting molecule(s) as defined herein, and then with a fusion polypeptide as defined herein, or preferably with a composition of the invention.

In another particular embodiment, this method is performed *in vivo.* In this case, the fusion polypeptide, the biotinylated targeting molecule(s) and optionally, additional elements as defined herein, are contacted to cells of a human or non-human host by administration of these compounds or of composition(s) comprising them to said host. Preferably, a composition of the invention is administered to the host. Alternatively, the fusion polypeptide and the biotinylated targeting molecule(s) can be administered as separate compositions, but preferably extemporaneously.

The invention also provides a method for preventing or treating a human disease, by contacting one or several effector molecule(s) with human cells, or subset(s) of human cells, *in vivo* or *ex vivo.* This method, which requires the use of a fusion polypeptide as defined herein, one or several biotinylated targeting molecule(s) as defined herein, and optionally one or several additional elements as defined herein, can be performed by the method for targeting one or several effector molecule(s) to cells, subsets of cells and/or to cell surface molecule(s) disclosed herein.

In a particular embodiment of the invention, one or several pico moles (for example 2, 3, 4, 5, 6, 7, 8, 9 or 10 pico moles) of an effector molecule are administered to a human or non human host.

In a particular embodiment of the invention, the cells (for example human or non human cells) which are contacted with the fusion polypeptide and the effectors molecule(s) or the host (for example the human or non human host) to which the fusion polypeptide and the effectors molecule(s) are administered have(has) been previously primed with effector molecule(s) which is (are) identical to effector molecule(s) that are present in the fusion polypeptide.

In a further aspect, the invention relates to the use of a SA or avidin polypeptide as defined herein for preparing a fusion polypeptide as defined herein.

The invention is also directed to a method for the production of a polypeptide comprising a SA or avidin polypeptide, and in particular a fusion polypeptide as defined herein. Said method comprises: expressing said polypeptide in a cell, for example an *E. coli* cell, at a temperature of 20°C or less than 20°C, from a gene construct (in particular a polynucleotide, a plasmid or a vector) encoding said polypeptide. The *E. coli* cell can be for example an *E. coli* BL21 λDE3 cell or preferably an *E. coli* Artic Express DE3 cell.

*E. coli* Artic Express DE3 cell enables production of a polypeptide, and in particular of a polypeptide (for example a tetrameric fusion polypeptide) at a temperature of less than 20°C, for example at a temperature ranging from 10 to 15°C or from 10 to 20°C, and in particular at 10°C or 15°C. The produced polypeptide can then be solubilized in a 2M urea buffer, without having to use denaturing urea concentrations (which are above 4M).

This method enables direct production of a soluble tetrameric polypeptide or fusion polypeptide comprising a SA or avidin polypeptide, in the cytoplasm of *E*. *coli.* In contrast, methods disclosed in the prior art only allow production of a fusion polypeptide comprising a SA or avidin polypeptide as inclusion bodies, from which fusion polypeptide has to be extracted under denaturing conditions, for example with urea or guanidine solutions, and subsequently renaturated and refolded to form tetramers *in vitro.* Other methods disclosed in the prior art enable production of a fusion polypeptide comprising a SA or avidin polypeptide as a soluble fusion polypeptide but which is exported into periplasmic space of *E*. *coli* cells, which results in reduced yields.

In a particular embodiment of the invention, the method for the production of a polypeptide comprising a SA or a avidin polypeptide further comprises a step wherein the expressed polypeptide, in particular the expressed fusion polypeptide is purified using one or several IminoBiotin-Agarose columns (from Sigma).

By way of illustration, the affinity purification step can be performed as follows:
- the polypeptide is bound to a IminoBiotin-Agarose column (from Sigma), which can be for example equilibrated in 50 mM CH₃COONH₄ buffered with NH₄OH to pH 9.
- the column is then washed with 0.1 M acetic acid, 0.5 M NaCl pH 2.9 or pH 3,
- elution of the polypeptide is then performed using 0,1 M acetic acid without addition of salt, for example at pH3, preferably with immediate neutralization of acetic acid by addition of NH₄OH (for example 1/50 of fraction volume of 25% NH₄OH) to reach a final pH of about 9 or 9.3.

The invention is also directed to a method for the production of a composition or complex as defined herein, which comprises or consists in the following steps:
- contacting a fusion polypeptide as defined herein with one or several biotinylated targeting molecule(s) as defined herein; and
- optionally, contacting said fusion polypeptide with non-targeting molecule(s) as defined herein.

In a particular embodiment of the invention, this method comprises or consists in the following steps:
- producing a fusion polypeptide as defined herein by the method disclosed above; and
- contacting a fusion polypeptide as defined herein with one or several biotinylated targeting molecule(s) as defined herein; and
- optionally contacting said fusion polypeptide with non-targeting molecule(s) as defined herein.

In a particular embodiment of the invention, the fusion polypeptide is contacted first with one or several biotinylated targeting molecule(s) in condition enabling said biotinylated targeting molecules to interact and complex with the fusion polypeptide and, optionally, with non-targeting molecule(s) as defined herein.

In a particular embodiment of the invention, the fusion polypeptide is contacted with biotinylated targeting or non-targeting molecules by mixing a composition comprising said fusion polypeptide with a composition comprising said biotinylated targeting or non-targeting molecules.

The invention also relates to a method for the stimulation of specific T lymphocytes by targeting an antigen or fragment thereof comprising at least one T-cell epitope to antigen presenting cells, wherein said method comprises the steps of:
- exposing T cells, in particular CD8+ or CD4+ T cells, present in PMBC or whole blood to a fusion polypeptide as defined herein, which comprises said antigen or fragment thereof, and to a biotinylated targeting molecule as defined herein, which is capable of targeting one or several cell receptor(s) of antigen presenting cells, and wherein optionally said fusion polypeptide has been previously produced by the method of the invention; and
- detecting *in vitro* a change in activation of the T cells.

Said method can be performed *in vivo, in vitro* or *ex vivo,* but it is preferably performed *in vitro or ex vivo.*

The invention also relates to a method for the *in vitro or ex vivo* selection of a subset of APC to which the targeting of an antigen or a fragment thereof comprising at least one T-cell epitope can induce a T-cell immune response directed against said antigen or fragment thereof, wherein said method comprises the steps of:
- exposing *in vitro* or *ex vivo* T cells, in particular CD8+ or CD4+ T cells, to a subset of APC binding a fusion polypeptide as defined herein through biotinylated targeting molecule(s) as defined herein, wherein the fusion polypeptide comprises said antigen or fragment thereof; and
- detecting a change in activation of the T cell.

In a particular embodiment of the invention, the above-mentioned method comprises the steps of:
a) exposing a subset of APC to (i) biotinylated targeting molecules as defined herein, which are capable of targeting these APCs and in particular of interacting with one or several cell receptor(s) present on the surface of these subset of APCs, and to (ii) a fusion polypeptide as defined herein, which comprises the antigen or fragment thereof,
   wherein optionally said fusion polypeptide has been previously produced by the method of the invention; and
b) exposing T cells, in particular CD8+ or CD4+ T cells, to the subset of APCs provided by step a); and
c) detecting *in vitro* a change in activation of the T cells.

Step a) provides a subset of APC binding the fusion polypeptide through the biotinylated targeting molecule.

A "change in activation of the T cell(s)" as used herein can be for example a change in IL-2, IL-4, IL-5, IL-17 or IFN-y production.

In a particular embodiment of the invention, the detection of a change in T cell activation is achieved with the EPLISPOT assay, ELISA, or other assay to detect T cell activation, for example a proliferation assay.

In a particular embodiment of the invention, the test sample used in theses methods is peripheral blood mononuclear cells (PBMC), whole blood, or a fraction of whole blood.

The invention also relates to a polynucleotide encoding a fusion polypeptide as defined herein, and to a plasmid or a recombinant vector (in particular a recombinant expression vector) comprising said polynucleotide.

In a particular embodiment of the invention, the polynucleotide of the invention or the plasmid or a recombinant vector of the invention comprises or consists of SEQ ID NO.: 41.

The invention also relates to a cell comprising the polynucleotide of the invention or a plasmid or a recombinant vector of the invention.

The invention is also directed to a kit, in particular a kit for a diagnostic test of a disease in a mammal, for immonomonitoring a disease in a mammal and/or for the prevention and/or the treatment of a disease in a mammal, which comprises:
- a fusion polypeptide as defined herein, a polynucleotide or a plasmid or a recombinant vector encoding said fusion polypeptide or a cell able to express said fusion polypeptide; and
- instructions explaining how to use said fusion polypeptide in conjunction with biotinylated targeting molecule(s) in order that the effector molecule(s) comprised in said fusion polypeptide be delivered into or onto (preferably onto) subset(s) of cells targeted via said biotinylated targeting molecule(s); and
- optionally, biotinylated targeting molecule(s) as defined herein and/or one or several additional elements as defined herein.

Other characterizing features of the invention will become apparent from the examples and from the figures and they apply, individually or in combination, to the above disclosed elements of the invention.

### BRIEF DESCRIPTION OF THE DRAWINGS

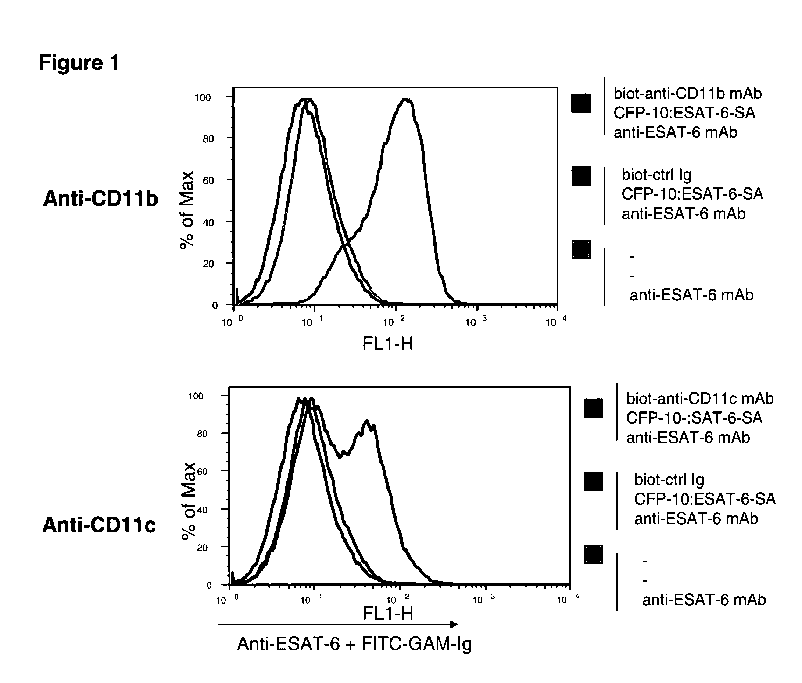

**Figure 1****.** *In vitro* binding of CFP-10:ESAT-6-SA, delivered to BM-DC by use of biot-mAbs specific to different DC surface receptors. Cells were first incubated at 4°C with 1.5 µg/ml of biot-conjugated control Ig isotypes (clone R187) or biot-mAbs specific to CD11b (clone M1/70.15.11.5.HL) or to CD11c (clone N418) integrins, prior to incubation with 20 µg/ml of CFP-10:ESAT-6-SA. The presence of ESAT-6 at the cell surface was detected by cytofluorometry, by use of the mouse anti-ESAT-6 mAb (clone 11 G4), followed by a goat anti-mouse Ig polyclonal Ab conjugated with FITC.

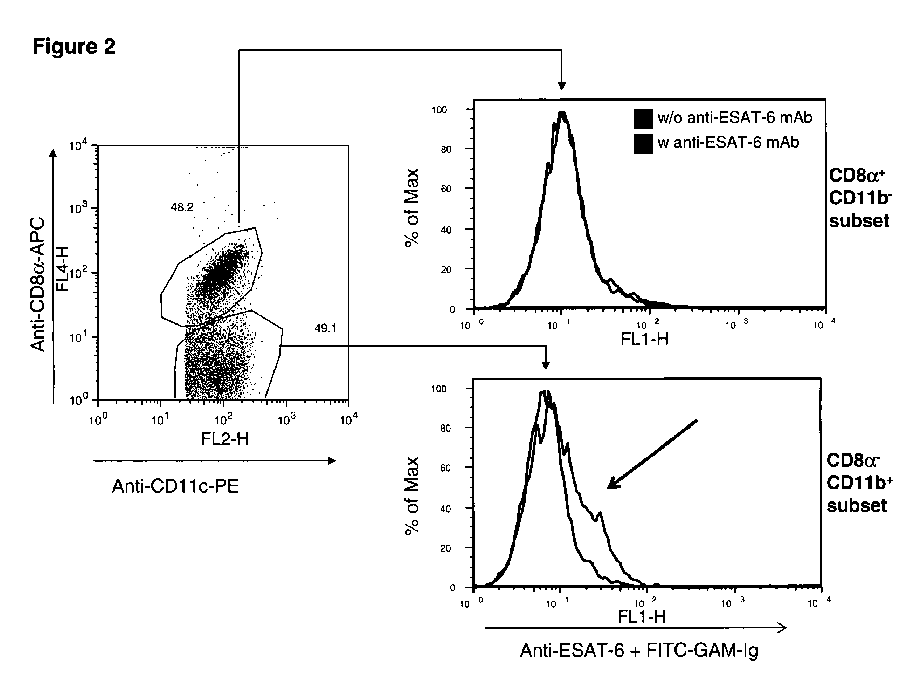

**Figure 2****.** *In vivo* tracking of CFP-10:ESAT-6-SA at the surface of spleen CD11b⁺ DC, subsequent to i.v. injection of CFP-10:ESAT-6-SA complexed to biot-anti-CD11b mAb. Detection of ESAT-6 at the surface of spleen DC of C57BU6 mice injected with CFP-10:ESAT-6-SA complexed to biot-anti-CD11b mAb. Mice were injected i.v. with 500 pmoles/mouse of CFP-10:ESAT-6-SA, complexed at a molar ration of 2:1, to biot-control Ig or to biot-anti-CD11b mAb, in the presence of 25 µg/mouse of Poly I:C This TLR3 agonist has been used here as in further immunization assays it has been used to activate DC in the developed model. At 2h post injection, spleen low density cells were analyzed by cytofluorometry. Cells were gated on CD11c⁺ CD8a⁺ (CD11b⁻) or CD11c⁺ CD8a⁻ (CD11b⁺) cells and ESAT-6 surface signal was analyzed by cytoflurometry, by use of the mouse anti-ESAT-6 mAb (clone 11 G4), followed by a goat anti-mouse Ig polyclonal Ab conjugated with FITC.

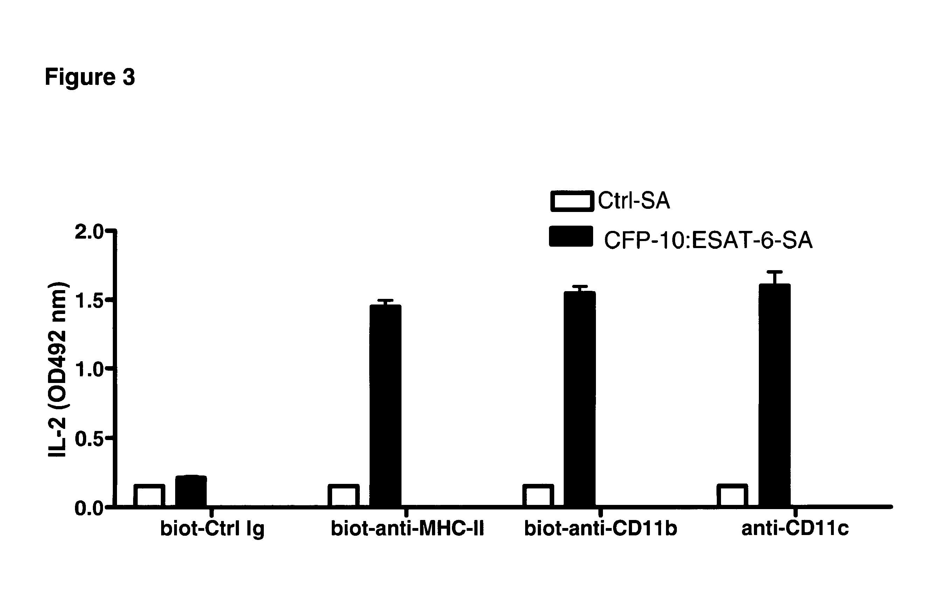

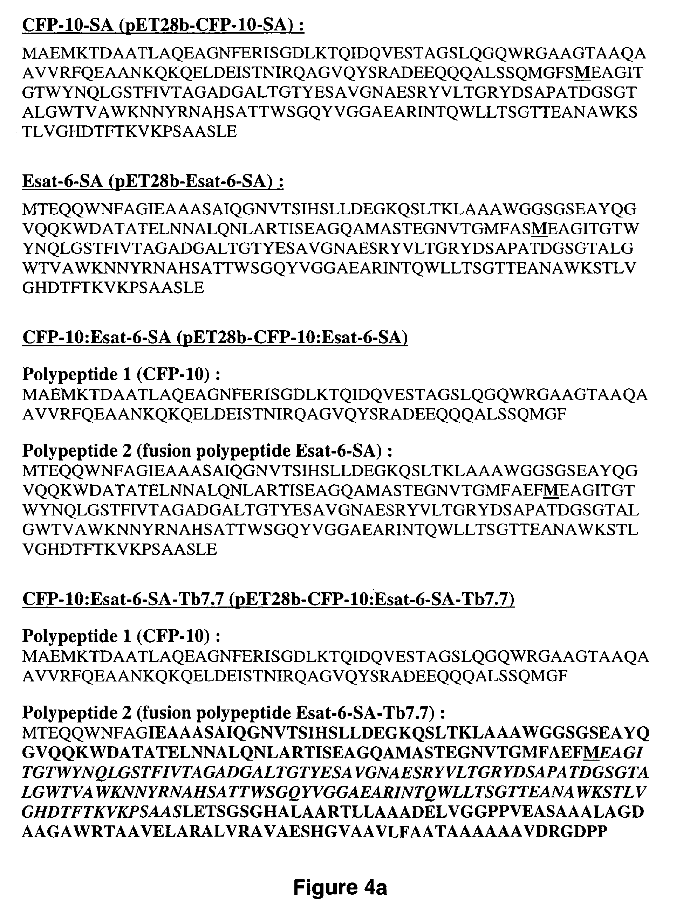

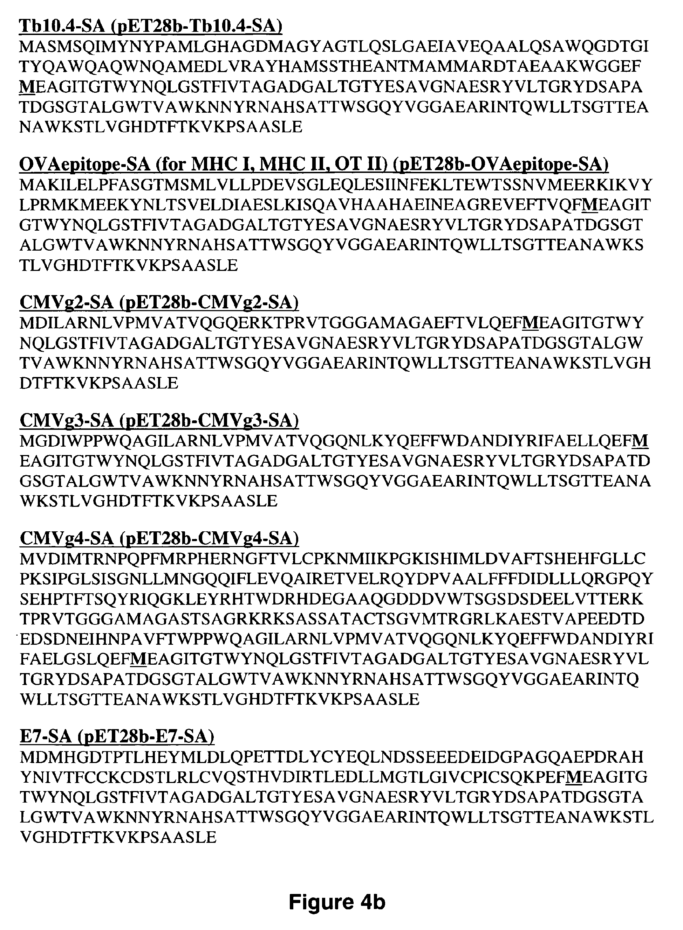

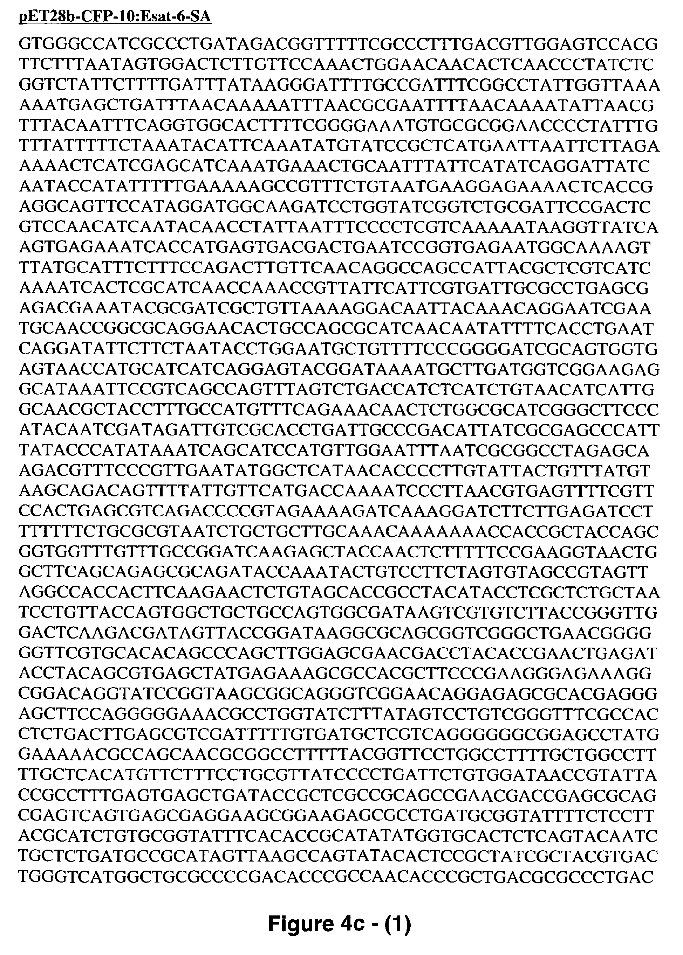

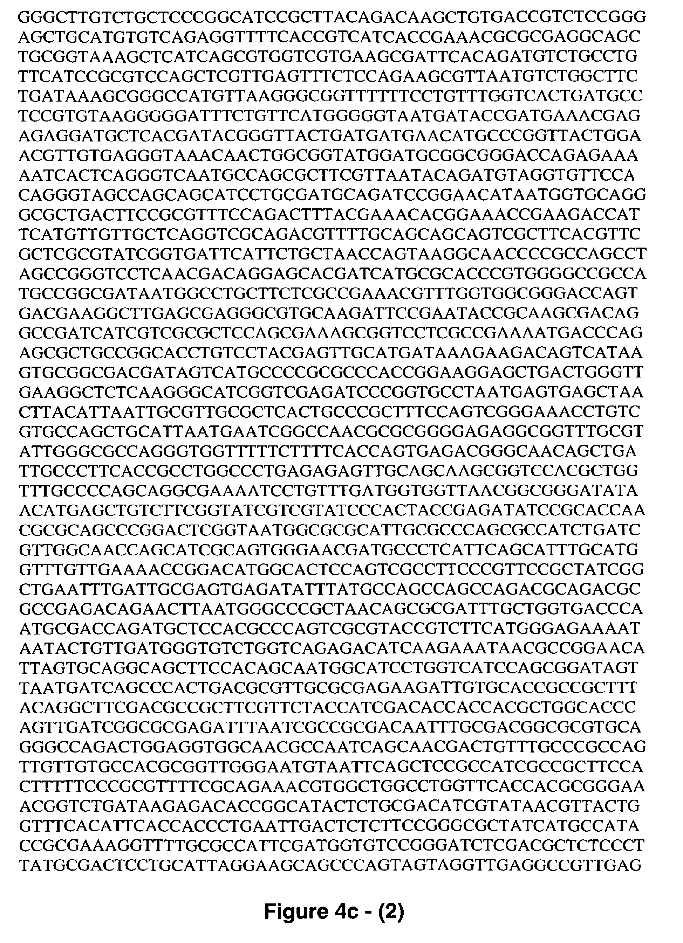

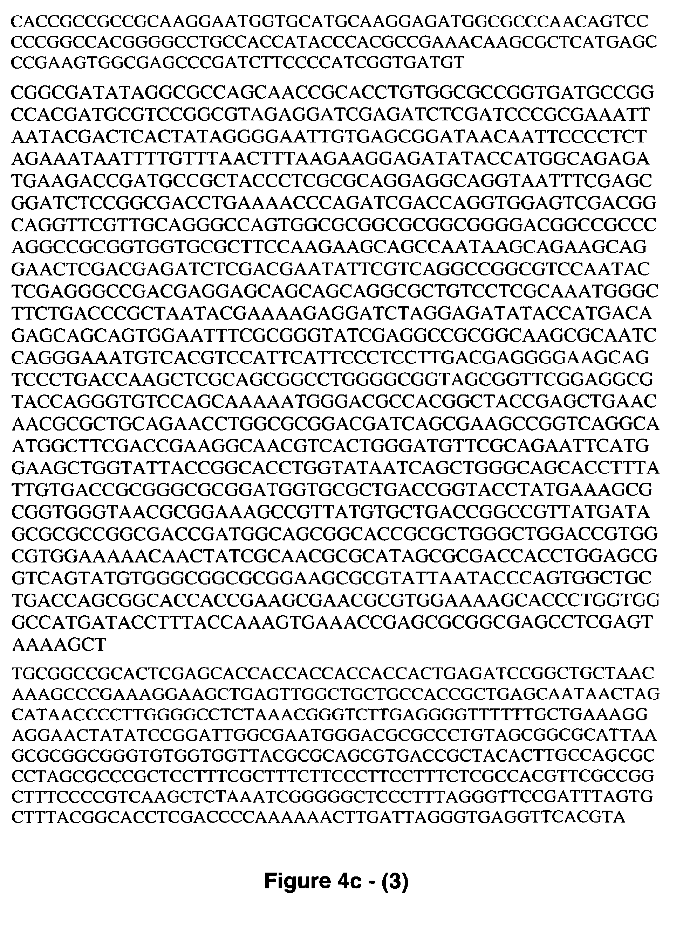

**Figure 3**. MHC-II-restricted presentation of ESAT-6 by BM-DC targeted with CFP-10:ESAT-6-SA via biot-mAbs specific to MHC-II, CD11b or CD11c. BM-DC from C57BU6 (H-2^{b}) mice were incubated at 4°C with biot-control Ig or biot-mAbs specific to MHC-II, CD11b or CD11c, washed and incubated at 4°C with 1 µg/ml of CFP-10:ESAT-6-SA or CFP-10-SA. Cells were then washed and co-cultured at 37°C, 5% CO2, with anti-ESAT-6:1-20 NB11 T-cell hybridoma, restricted by I-A^{b}. The recognition of immunodominant ESAT-6:1-20 epitope in the context of I-A^{b} by NB11 T-cell hybridoma leads to the production of IL-2 which has been assessed by ELISA in the co-culture supernatants at 24h.
**Figure 4****.**
   **A**. The amino acid sequences of the following constructs are given:
      ● CFP-10-SA (pET28b-CFP-10-SA) (SEQ ID NO.: 28) ;
      ● Esat-6-SA (pET28b-Esat-6-SA) (SEQ ID NO.: 29) ;
      ● CFP-10:Esat-6-SA (pET28b-CFP-10:Esat-6-SA) (SEQ ID NOs.: 30 and 31);
      ● CFP-10:Esat-6-SA-Tb7.7 (pET28b-CFP-10:Esat-6-SA-Tb7.7) (SEQ ID NOs.: 32 and 33) ;
      ● Tb10.4-SA (pET28b-Tb10.4-SA) (SEQ ID NO.: 34) ;
      ● OVAepitope-SA (for MHC I, MHC II, OT II) (pET28b-OVAepitope-SA): synthetic polyepitope derived from hen egg ovalbumin (SEQ ID NO.: 35) ;
      ● CMVg2-SA (pET28b-CMVg2-SA): synthetic oligoepitope derived from human cytomegalovirus pp65 (SEQ ID NO.: 36) ;
      ● CMVg3-SA (pET28b-CMVg3-SA) : synthetic oligoepitope derived from human cytomegalovirus pp65 (SEQ ID NO.: 37) ;
      ● CMVg4-SA (pET28b-CMVg4-SA): synthetic oligoepitope derived from human cytomegalovirus pp65 (SEQ ID NO.: 38) ; and
      ● E7-SA (pET28b-E7-SA): polypeptide of the E7 oncoprotein of human papillomavirus 16 (SEQ ID NO.: 39).
      In these sequences, the methionine residue which is underlined corresponds to the one which is located at the junction between the antigen and linker-encoded sequences (these sequences are N-terminal to the methionine residue) and the streptavidin polypeptide (residues 14-139 of the streptavidin preprotein from *Streptomyces avidinii*) (these sequences are C-terminal to the methionine residue). In addition, a linker of sequence leucine ― glutamic acid (L ― E), which is optional, is located at the C terminal end of these sequences.
   **B.** Sequence of pET28b-CFP-10:Esat-6-SA (vector for co-expression of CFP-10 with ESAT6-SA) (SEQ ID NO.: 40). The sequences indicated (i) in bold, (ii) in italics and underlined, (iii) in bold and italics and (iv) the sequence which is underlined correspond respectively to sequences of the (i) T7 promoter, (ii) the lac operon, (iii) the ribosome binding site, and (iv) the M. *tuberculosis* antigens CFP-10 and Esat-6 and to the SA polypeptide (residues 14-139).

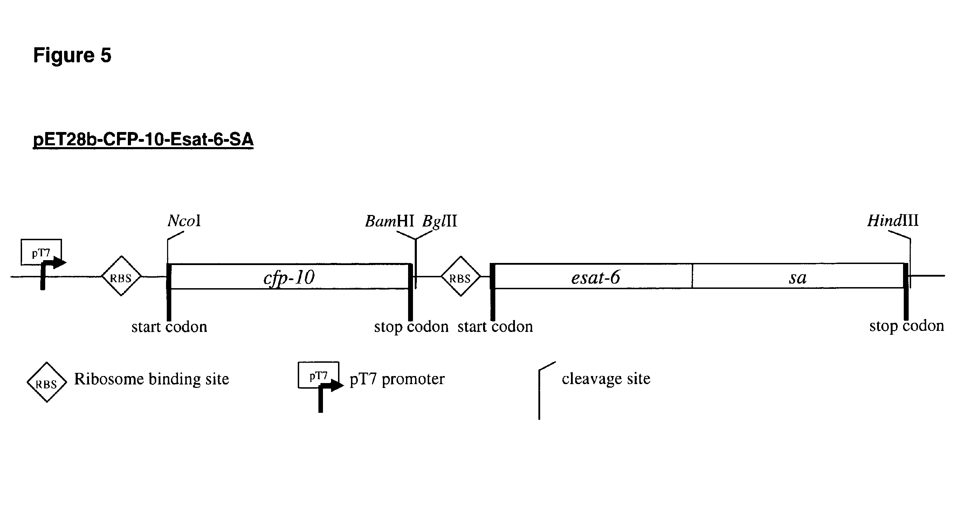

**Figure 5****.** pET28b-CFP-10-Esat-6-SA. Vector for coexpression of CFP-10 with ESAT6-SA. Schematic drawing of the key elements of the expression vector determining production of the CFP-10:ESAT-6-SA fusion complex in a bacterial cell.

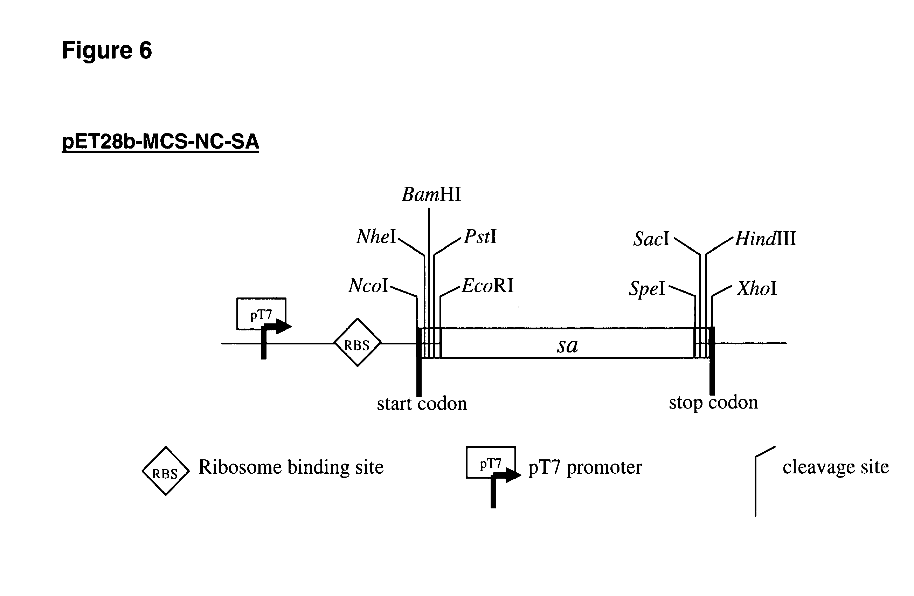

**Figure 6****.** pET28b-MCS-NC-SA. Expression vector for streptavidin with N- and C-terminal multi cloning sites. Schematic drawing of the key elements of the expression vector determining production of the natural core streptavidin protein in a bacterial cell, including restriction sites in the multiple cloning sites that can be used for fusions with antigens and effector molecules.

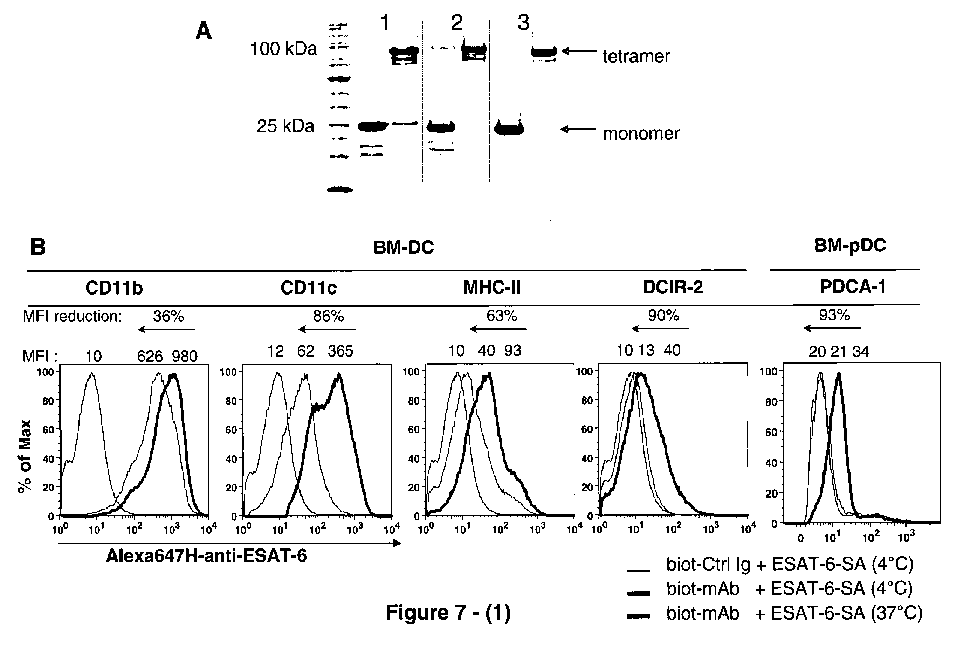

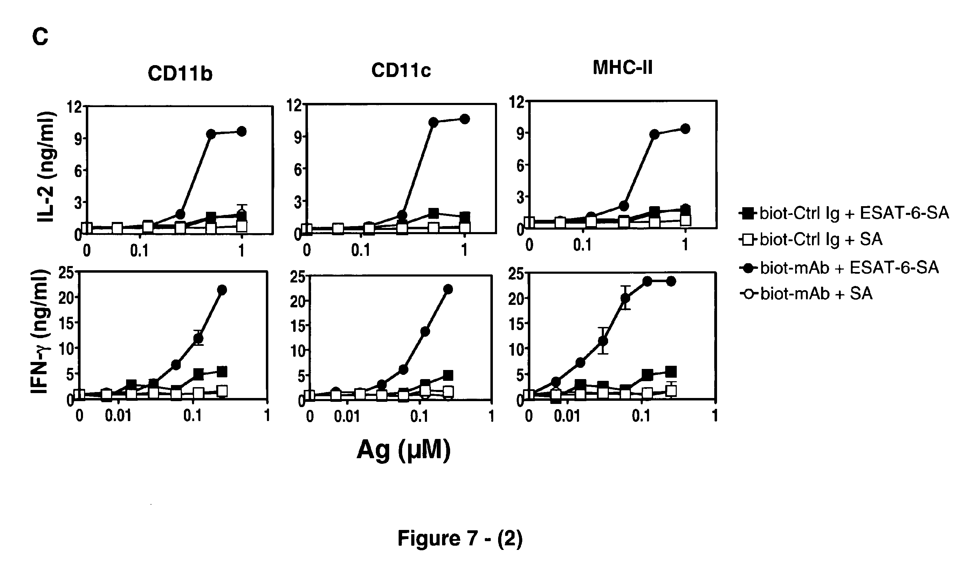

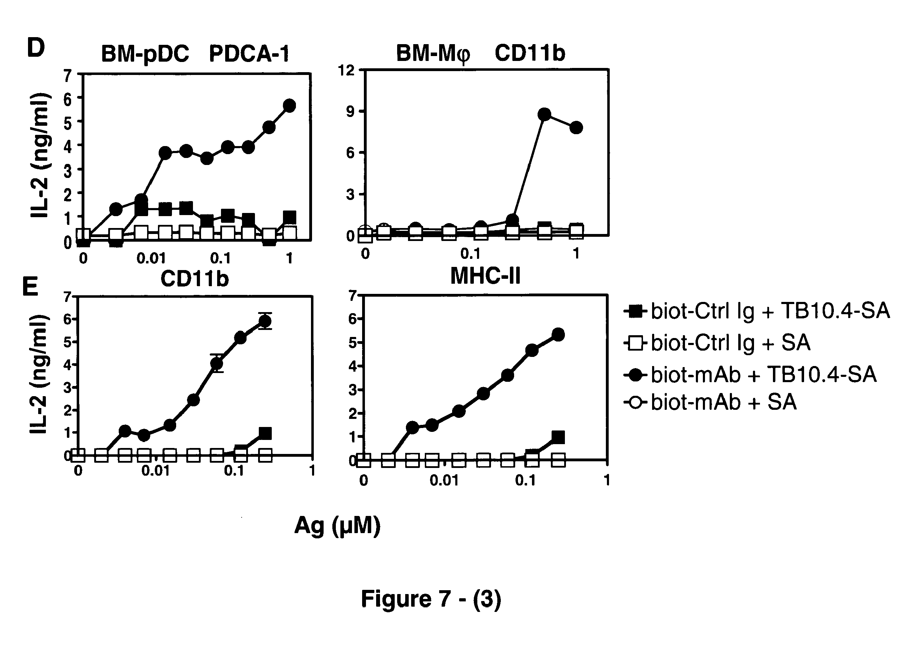

**Figure 7****.** ESX-SA fusion proteins, their Ab-mediated binding to DC surface receptors, followed by their internalization and delivery to the MHC-II presentation pathway. (A) 15% Tris-Tricine SDS-PAGE of ESX-SA fusion proteins. Monomers of the fusion of streptavidin with antigen were obtained by heating the sample in SDS-PAGE loading buffer at 100°C for 5 minutes. Tetramers of the antigen-SA fusions were stable in sample buffer at room temperature. 10 micrograms of each protein sample were loaded and separated on 15% Tris-Tricine SDS-PAGE gels stained with Coomassie Blue. 1: TB10.4-SA (24.5 kDa). 2: CFP-10-SA (24.5 kDa). 3: ESAT-6-SA (23.5 kDa). (B) *In vitro* binding of ESAT-6-SA delivered to conventional or plasmacytoid BM-DC by use of biot-mAbs specific to different DC surface receptors. Cells were first incubated at 4°C with biot-control Ig or each of the biot-mAbs specific to the DC surface receptors, prior to incubation with ESAT-6-SA. Cells were then kept at 4°C or incubated at 37°C for 3h in order to evaluate the possible internalization. Surface ESAT-6 signal was detected by cytofluorometry by use of Alexa647H-conjugated anti-ESAT-6 mAb. ESAT-6 signal MFI are indicated at the top of each peak, arrows and percentages of MFI reduction at 37°C are indicated above each histogram. (C) MHC-II-restricted presentation of ESAT-6 by BM-DC targeted with ESAT-6-SA via different biot-mAbs. BM-DC from C57BU6 (H-2^{b}) mice were incubated at 4°C with biot-control Ig or biot-mAbs specific to diverse DC surface receptors, washed and incubated at 4°C with various concentrations of ESAT-6-SA or SA alone. Cells were then washed and co-cultured with anti-ESAT-6 NB11 T-cell hybridoma (C, top) or with Thy-1.2⁺ splenocytes (C, bottom) from C57BU6 mice chronically infected with M. *tuberculosis* H37Rv. IL-2 (C, top) or IFN-y (C, bottom) produced by T cells were assessed by ELISA in the co-culture supernatants at 24h or 72h, respectively. (D) MHC-II-restricted presentation of ESAT-6 by BM-derived plasmacytoid DC or BM-derived M targeted with ESAT-6-SA, as evaluated with anti-ESAT-6 NB11 T-cell hybridomas, as explained in the legend to (C). (E) MHC-II-restricted presentation of TB10.4 by BALB/c (H-2^{d}) BM-DC targeted with TB10.4-SA, as evaluated by use of 1 H2 T-cell hybridoma. Results are representative of at least three independent experiments.

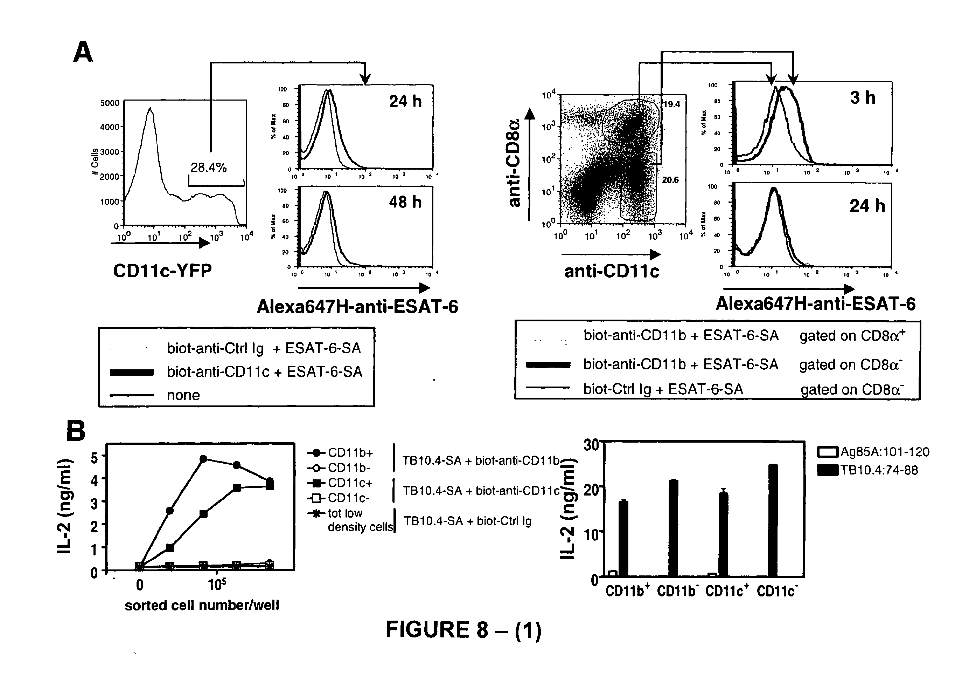

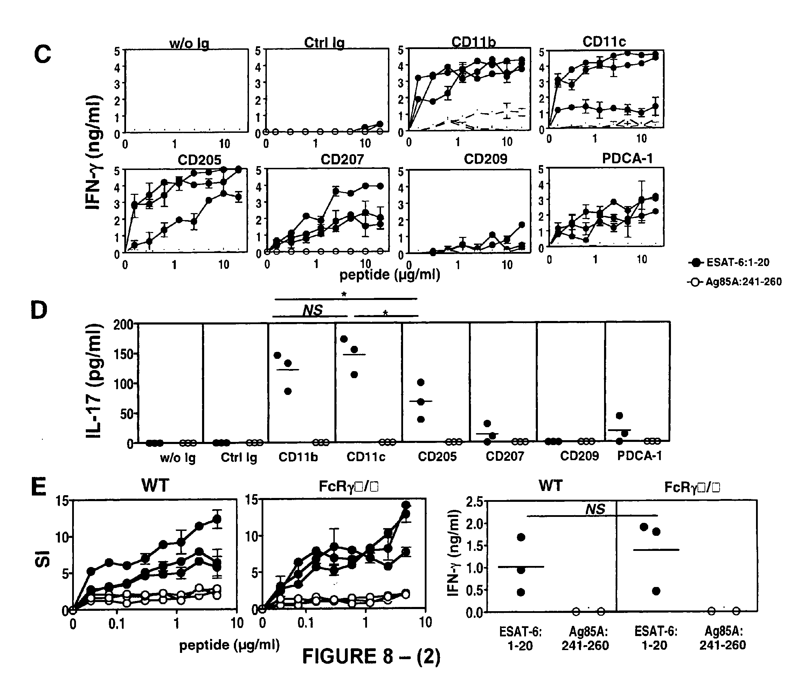

**Figure 8****.** *In vivo* binding and presentation of ESX antigens by different DC subsets, followed by induction of CD4⁺ T-cell responses by ESX antigen targeting to different DC surface integrins, C-type lectins or PDCA-1. (A) Tracking of ESAT-6 at the surface of spleen DC of mice immunized with ESAT-6-SA complexed to different biot-mAbs. C57BU6 mice, either CD11c YFP (left) or WT (right), were injected i.v. with 500 pmoles/mouse of ESAT-6-SA, complexed at a molar ration of 2:1, to biot-control Ig or biot-mAbs specific to CD11 c (left) or CD11 b (right), in the presence of Poly I:C. At different time points post-injection, spleen low density cells were gated on CD11c YFP cells (left) or on CD11c⁺ CD8a⁺ or CD11c⁺ CD8a⁻ cells (right) and ESAT-6 surface signal was analyzed by cytoflurometry by use of A!exa647H-conjugated anti-ESAT-6 mAb. (B) *Ex vivo* presentation of TB10.4 by targeted DC. BALB/c mice were injected with 50 pmoles/mouse of TB10.4-SA, complexed, at a molar ratio of 1:1, to mAbs specific to DC surface receptors, in the presence of Poly I:C. Spleen low density cells were then positively selected by use of anti-biot mAb conjugated to magnetic beads and sorted at 3h post-injection. Various numbers of cells from positive or negative sorted fractions were co-cultured with 1 H2 T-cell hybridomas and IL-2 assessed by ELISA (left) As a functional control, the positive and negative fractions were tested for their capacity to present the synthetic TB10.4:74-88 peptide added *in vitro.* (C) Induction of CD4⁺ T-cell responses by ESAT-6 targeting to different DC surface integrins, C-type lectins, or PDCA-1. C57BU6 mice were immunized i.v. with a single injection of 50 pmoles/mouse of ESAT-6-SA, without biot-Ig or complexed to biot-control Ig or to mAbs specific to CD11 b, CD11 c integrins or to CD205, CD207, CD209, DCIR-2 C-type lectins or to PDCA-1, in the presence of 25 µg/mouse of Poly I:C. Eleven days post injection, total splenocytes from three individual immunized mice/group were stimulated *in vitro* with various concentrations of ESAT-6:1-20 peptide or Ag85A:241-260, as a negative control peptide. IFN-y response was measured by ELISA in the culture supernatants at 72h. (D) ESAT-6-specific IL-17 response was measured in the supernatants of the cultures stimulated *in vitro* with 10 µg/ml of peptide. (E) Ab-mediated ESX antigen targeting to DC is independent of Ig Fc interaction with FcR. Proliferative response, expressed as stimulation index (SI) (left), or IFN-y CD4⁺ T-cell responses, following *in vitro* stimulation with 10 µg/ml of peptides (right). Results are from individual WT or FcRy°/° mice determined at day 11 post-immunization. Horizontal bars indicate the mean values. Results are representative of at least two independent experiments. * = statistically significant, as determined by Student's *t* test, p < 0.05, *ns* = not significant.

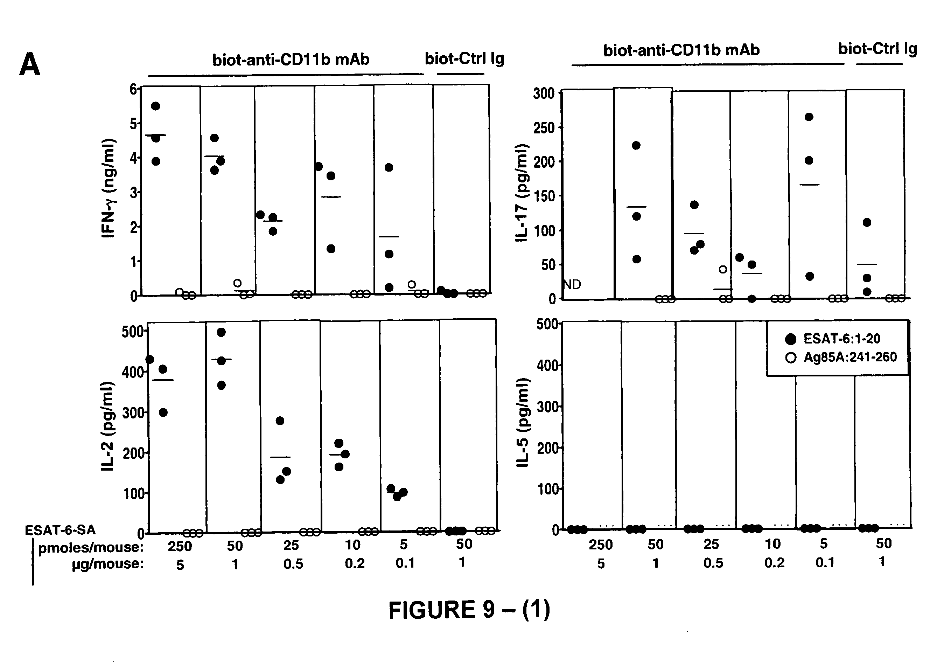

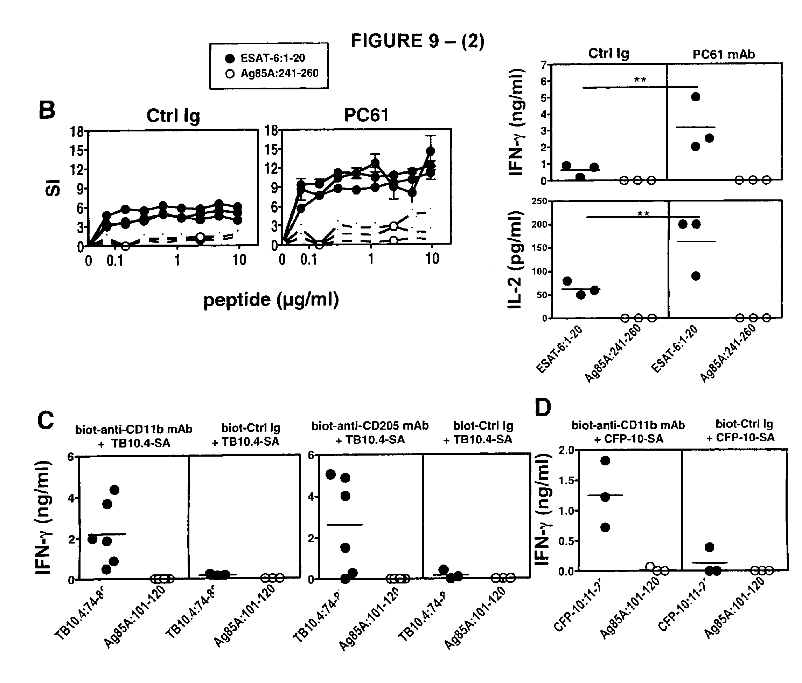

**Figure 9****.** Dose-response effect of *in vivo* ESAT-6 targeting on Th1, Th2 and Th17 responses, Treg-mediated control of the induced CD4⁺ T-cell responses and extension of the immunization approach to other ESX antigens. (A) Determination of dose-response effect of ESX antigen targeting on T-cell responses. C57BL/6 (H-2^{b}) mice were immunized with various doses of ESAT-6-SA, complexed to biot-CD11b mAb, at 2:1 ratio at molar basis in the presence of Poly I:C. IFN-y, IL-2, IL-17 and IL-5 responses were quantified at day 11 post injection. (B) The CD4⁺ T-cell responses induced by *in vivo* ESAT-6-SA targeting are under the negative control of Treg. Proliferative (left), IFN-y or IL-2 (right) responses in C57BL/6 mice treated with 1 mg/mouse of a control lg or of anti-CD25 mAb (clone PC61), 2 days before the injection of 50 pmoles/mouse of ESAT-6-SA, complexed to biot-anti-CD11 b mAb, in the presence of 25 µg/mouse of Poly I:C. (C, D) Induction of IFN-y CD4⁺ T-cell response to immunodominant epitopes of TB10.4 (C) or CFP-10 (D) by targeting TB10.4-SA to CD11b or to CD205 in BALB/c (H-2^{d}) mice (C) or by targeting CFP-10-SA to CD11b, in C3H (H-2^{k}) mice (D). Results are representative of two independent experiments.** = statistically significant, as determined by Student's *t* test, *p* < 0.02.

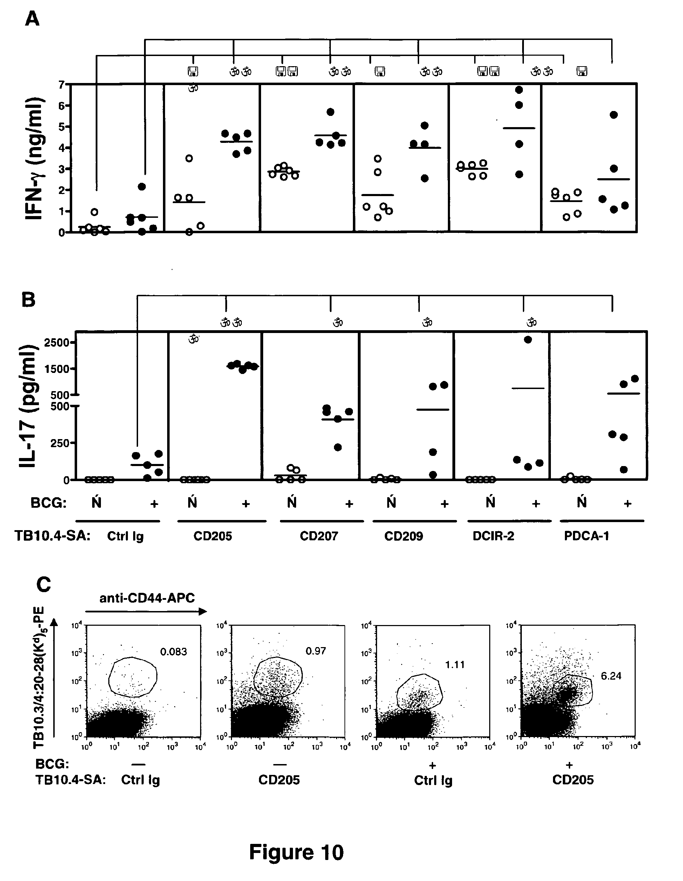

**Figure 10****.** Boost effect of ESX antigen targeting to DC surface receptors subsequent to BCG priming. BALB/c mice, unprimed or primed s.c. with 1 x 10⁶ CFU of BCG at day 0, were boosted twice at days 14 and 21, with 50 pmoles (= 1 µg)/mouse of TB10.4-SA, complexed to biot-control lg or biot-anti-CD205 mAb, at molar ration of 2:1, in the presence of Poly I:C. TB10.4-specific IFN-y (A) and IL-17 (B) CD4⁺ T-cell responses were studied at day 28. Statistical analyses were performed by Student's *t* test. ⊹ and ⊹⊹ = differences statistically significant, respectively, p < 0.05 and 0.005, between BCG-unprimed mice and mice immunized with TB10.4-SA targeted to different DC markers. ∗ and ∗∗ differences statistically significant, respectively, p < 0.05 and 0.005, between BCG-primed mice and BCG-primed mice and boosted with TB10.4-SA targeted to different DC markers. (C) CD8⁺ T-cell cross priming in BCG-primed BALB/ mice boosted with TB10.4-SA targeted to CD205. Representative CD8⁺ T-cell responses to H-2K^{d}-restricted GYAGTLQSL epitope, shared by TB10.3 and TB10.4, detected by cytofluorometry by use of a combination of FITC-anti-CD8_{α}, allophycocyanine-anti-CD44 and PE-conjugated H-2K^{d} pentamer complexed with TB10.3/4:20-28 peptide. Results are representative of at least two independent experiments.

### EXAMPLES

### I. Construction and production of recombinant Ag-SA fusion proteins

The codon-optimized synthetic gene encoding for expression in *E. coli* of residues 14-139 of the streptavidin protein from *Streptomyces avidinii* (Sano et al, 1995) was obtained from GenScript (NJ, USA) and inserted into the pET28b expression vector (Novagen, Darmstadt, Germany). The reason for using only the natural core of strepavidin without N- and C- terminal part was the previously reported proteolytical processing of streptavidin during the cultivation and purification of SA from *E. coli* (Pahler A. et al 1987, Bayer E. A. et al, 1989). To avoid cleavage of the streptavidin fusion proteins, the natural core SA without the processed sequences was used, where the truncation of SA did not perturb tetramerization capacity of the fusion constructs.

The genes for appropriate antigens were PCR-amplified using pairs of PCR primers indicated in Table 1 and genetically fused to the 5'- or 3' end of the streptavidin gene by insertion into appropriate restriction sites (Table 1). The exact sequence of the cloned inserts was verified by DNA sequencing. The plasmids were transformed in to *E. coli* cells for IPTG inducible production of proteins.

CFP-10 fusion proteins (CFP-10-SA, CFP-10-Esat-6-SA, CFP-10-Esat-6-SA-Tb7.7) were produced in *E. coli* BL21(λDE3) cells (Stratagene, La Jolla, USA) at 20 °C, while *E. coli* Artic Express DE3 cells (Stratagene, La Jolla, USA) was used for production of other SA fusion proteins. To express the CFP-10-SA fusion proteins the transformed *E. coli* strain BL21(λDE3) was grown at 20 °C in LB medium (containing 60 µg/ml kanamycin), which was inoculated with O/N culture to the OD₆₀₀ ∼ 0,8 and subsequently induced with IPTG to final concentration of 0,5 mM. The cells were harvested 8 hours later, washed one time in 50 mM CH₃COONH₄ buffered to pH 9 by 25% NH₃*H₂O (AC buffer) and stored at -20 °C.

The ESAT-6-SA fusion polypeptide on its own is rather poorly soluble in *E. coli* cells. However, when CFP-10, which is a chaperon for ESAT-6, is co-expressed with ESAT-6, or with a fusion polypeptide comprising ESAT-6 (for example the fusion polypeptide ESAT-6-SA), it enhances the solubility of ESAT-6 or of said fusion polypeptide in *E. coli* cytoplasm.

**Table 1: sequence of the primers used in PCR cloning (SEQ ID NOs.: 6-27).**

| name of primer | sequence | restriction site | used to |
|---|---|---|---|
| ESAT-6-1 | ATTACCATGACAGAGCAGCAGTGG | *Nco* I | fusion protein with SA |
| ESAT-6-II | ATTTTCCATGGATGCGAACATCCCAGTGAC | *Nco* I | |
| TB10 4-I | ATGCTAGCATGTCGCAAATCATGTACAA | *Nhe* I | fusion protein with SA |
| TB10.4-II | ATGAATTCGCCGCCCCATTTGGCG | *Eco* RI | |
| MCS-N-ter.I | CATGGCTAGCGGATCCCTGCAGG | *Nco* I | multi clonning site on N terminus SA |
| MCS-N-ter.II | AATTCCTGCAGGGATCCGCTAGC | *Eco* RI | |
| MCS-C-ter.I | TCGAAACTAGTGAGCTCAAGCTTTAACTCGAGA | *Xho* I | multi clonning site on N terminus SA |
| MCS-C-ter.II | AGCTTCTCGAGTTAAAGCTTGAGCTCACTAGTT | *Hind* III | |
| OVA-I | CTACCATGGCTAAGATCCTGGAGCTTCCAT | *Nco* I | multi clonning site on N terminus SA |
| OVA-II | TACGAATTCGACAGATGTGAGGTTGTATT | *Eco* RI | |
| E7-I | TACCATGGATATGCATGGAGATACACCTAC | *Nco* I | multi clonning site on N terminus SA |
| E7-II | TCGAATTCAGGTTTCTGAGAACAGATGG | *Eco* RI | |
| CMV-G2-I | TA CCC ATG GAT ATC CTG GCT CGT AAC CTG GTT | *Nco* I | multi clonning site on N terminus SA |
| CMV-G2-II | CGCTGCAGTACGGTGAATTCAGCACC | *Pst* I | |
| CMV-G3-I | ATCCATGGGTGATATCTGGCCGCCGTGGCAGG | *Nco* I | multi clonning site on N terminus SA |
| CMV-G3-II | CTCTGCAGCAGTTCAGCGAAGATACG | *Pst* I | |
| CMV-G4-I | TACCATGGTAGATATCATGACCCGTAACCCGCA G | *Nco* I | multi clonning site on N terminus SA |
| CMV-G4-II | TAGGATCCCAGTTCAGCGAAGATACGG | *Pst* I | |
| OTII-I | AATTGGATATCGCTGAATCTCTGAAAATCTCTC AGGCTGTTCACGCTGCTCACGCTGAAATCAACG AAGCTGGTCGTGAAGTTGAATTTACCGTAC | *Eco* RI | OT II epitope inserted into pET28b OVAepitope-SA |
| OTII-II | AATTGTACGGTAAATTCAACTTCACGACCAGCT TCGTTGATTTCAGCGTGAGCAGCGTGAACAGCC TGAGAGATTTTCAGAGATTCAGCGATATCC | *Eco* RI | |
| TB7.7-I | ATACTAGTGGCAGCGGCCACGCG | *Spe* I | multi clonning site on C terminus SA |
| TB7.7-II | AGTAAGCTTCTACGGCGGATCACCCCGG , | *Xho* I | |

Other SA-fusion expression vectors were transformed to *E. coli* strain Artic Express DE3, where the newly synthesized proteins were stabilized by chaperons *cpn* 10 and *cpn 60,* induced at low growth temperatures. The proteins of interest were produced in soluble form in bacterial cytosol. 500 ml LB medium (60 µg/ml kanamycin and 20 µg/ml gentamycin) was inoculated with O/N culture, cells were grown at 28 °C to A₆₀₀ ∼0.8 before temperature was lowered to 10°C and production of proteins was induced by addition of IPTG to 0.5 mM final concentration. The culture was harvested 24 hours later, washed one time in 50 mM AC buffer and stored at -20 °C.

*E. coli* Arctic express DE3 cells have cold-inducible expression of cpn10 and cpn60 chaperons, which assist protein folding at low temperatures (around 10°C) and help maintain recombinant proteins soluble. Use of these cells enables to obtain folded tetramers of fusion polypeptides, which only precipitate in *E. coli* cytoplasm, without forming true inclusion bodies, and can be extracted as in native tetramers forms, for example using 2 M urea, which is not denaturing the tetramers. Hence, no *in vitro* refolding of the produced fusion polypeptides is necessary under these conditions.

The frozen cells were resuspended in AC buffer and lyzed by ultrasonic disruption. CFP-10-SA fusion proteins were purified directly from soluble cytosolic extract, while the other SA fusion tetramers were solubilized from cell debris by extraction with 2 M Urea in AC buffer without tetramer disruption, respectively. The extracts were loaded on IminoBiotin-Agarose (Sigma) columns equilibrated in AC buffer with 0.5 M NaCl (pH 9). The columns were first washed with several bed volumes of equilibration buffer, followed by 0.1 M acetic acid pH 2,9 with 0.5 M NaCl. Elution was achieved with 0.1 M acetic acid pH 2.9 without salt. Eluted fractions were immediately buffered by addition of 1/50 of fraction volume of 25% NH₃*H₂O to reach a final pH 9 and a soluble stable protein was obtained. In turn, elution with 50 mM ammonium or sodium acetate pH 4.0 recommended within IminoBiotin agarose datasheet of Sigma-Aldrich, resulted in elution of precipitated Ag-SA fusion proteins.

The unfused core streptavidin eluted already in 0.1 M acetic acid 0.5 M NaCl. In contrast, the washing step with 0,1 M acetic acid 0.5 M NaCl pH 2,9 was crucial for stabilization and retention of the Ag-SA fusion tetramers on the column during the decrease of pH from equilibration buffer (pH 9) to elution buffer (pH 2.9) to prevent precipitation. Subsequent elution with 0,1 M acetic acid pH 2.9 without salt allowed to recover soluble proteins.

The Ag-SA fusion proteins were concentrated on spin columns (Millipore, Bedford, MA, USA) and contaminating lipopolysacharide was removed by passage through EndoTrap column (Profos, Regensburg, Germany), to reduce LPS levels below 50 EU/mg of protein, as assessed using the endotoxin chromogenic LAL test assay kit (Lonza, Walkersville, MD, USA). Formation of Ag-SA tetramers was controlled using Tris-Tricine SDS-PAGE gels (15%). Biotin binding was controlled in Western blots by detection of biotinylated marker proteins with antigen-SA fusions that were themselves detected by a sandwich of antigen-specific polyclonal sera and anti-rabbit-peroxidase conjugate.

### II. Data obtained with construct CFP-10:ESAT-6-SA

Construct CFP-10:ESAT-6-SA enables co-expressing the CFP-10 protein and the ESAT-6-SA fusion polypeptide in the same cell (for example in *E. coli* cell), from the same expression vector. CFP-10, which is a chaperon for ESAT-6, associates with the ESAT-6-SA fusion polypeptide and hence enhances its solubility in *E. coli* cytoplasm.

CFP-10:ESAT-6-SA fusion protein binds efficiently to the surface of mouse bone-marrow-derived dendritic cells (BM-DC) via biotinylated mAbs specific to DC surface markers (see figure 1).

Following injection of CFP-10:ESAT-6-SA, complexed to biot-mAbs specific to DC surface markers, it is possible to detect this fusion protein specifically at the surface of the targeted DC subset (see figure 2).

CFP-10:ESAT-6-SA fusion protein, targeted in vitro to the surface of BM-DC via biot-mAbs specific to different DC surface markers, gains access to the MHC-II processing/presentation pathway, leading to the presentation of immunodominant epitopes by MHC-II molecules to specific TCR (see figure 3).

### III. Targeting of Mycobacterial ESX Antigens to Diverse Dendritic Cell Subsets for the Induction of Immunity against Mycobacterium tuberculosis

### A. Introduction

One-third of the Earth's population is infected with *Mycobacterium tuberculosis,* making the pulmonary tuberculosis the most widely spread infectious disease, leading to 1.6 million deaths annually. The only vaccine in use against infection with M. *tuberculosis,* the live attenuated *M. bovis* BCG (Bacillus Calmette-Guérin), is not able to protect efficiently against the adult pulmonary tuberculosis in endemic zones. Moreover, with the resurgence of tuberculosis in immuno-compromised individuals and the rapid expansion of multi-drug resistant and extensively drug-resistant tuberculosis, the need of a better rational design of new strategies of anti-tuberculosis vaccines is reinforced (WHO, 2007). Despite intense research on live attenuated and/or sub-unit anti-tuberculosis vaccines, a very few vaccine candidates display only slightly improved protective effect, with limited success compared to BCG (Kaufmann, 2006).

In the present study, we sought to drive the extensive knowledge available on the properties of DC and antigen targeting to DC subsets, towards the practical *in vivo* antigen delivery to DC in anti-tuberculosis vaccination. Indeed, so far, addressing M. *tuberculosis-derived* protein antigens to DC subset(s) and/or DC surface receptor(s), for the induction of protective anti-mycobacterial immunity, has not been investigated.

Our strategy for the rational design of a new anti-mycobacterial vaccine was to target prominent mycobacterial antigens, *i.e.,* proteins of ESX family, to diverse DC subsets with specialized activities. Indeed, mobilization of the latter by their direct *in vivo* targeting through their specific surface markers represents a promising pathway to dictate and to control differentiation of T cells. To this end, we developed a versatile *in vivo* approach by genetic fusion of selected ESX antigens to streptavidin, thus able to be complexed to individual biotin-conjugated mAbs of a wide-ranging panel of specificities against DC surface receptors and to be readily carried *in vivo* to different DC subsets. By use of this strategy, we showed that minute amounts, i.e, several pmoles, of ESX antigens targeted to β2 integrins, PDCA-1 or diverse C-type lectins were highly efficiently captured, endocytosed and presented by MHC molecules *in vitro* and *in vivo* and induced ESX-specific Th1 and Th17 - but not Th2 - responses. Moreover, in BCG-primed mice, boosting with ESX antigen targeting to DC subsets led to a remarkable improvement of Th1 and Th17 responses in the case of targeting to C-type lectins or to PDCA-1. In BCG-primed mice, TB10.4 targeting to CD205 endocytic C-type lectin also induced a significant cross-priming of specific CD8⁺ T cells.

Despite their shared morphology, abundance in T-cell areas of lymphoid tissues, high MHC-II expression and outstanding potential to continuously probe the environment, process and present antigens to T cells, DC are divided into different subsets, according to their ontogenic origin, phenotype, maturation programs and specialized functions. Although the well-established classification of the mouse DC subsets cannot been directly transposed to the human DC populations, plasmacytoid DC, blood-derived lymphoid tissue resident DC, peripheral migratory DC and monocyte―derived inflammatory DC have been distinguished in both mice and humans (Reis e Sousa, 2006, Shortman, 2007, Randolph, 2008). In the mouse spleen, three major DC subsets are distinguished: (i) CD11c⁺ B220⁺ Plasmacytoid DC Antigen (PDCA)-1 ⁺ plasmacytoid DC, specialized in the production of type-I IFNs, (ii) CD11c⁺ CD11b⁻ CD8ₐ⁺ conventional DC, with high potential to take up notably dead cells, to process and to cross-present the derived antigens and to activate T cells via IL-12p70 production, and (iii) CD11c⁺ CD11b⁺ CD8ₐ⁻ conventional DC, considered as potent inducers of MHC-II-restricted T-cell responses against exogenous antigens (Reis e Sousa, 2006). In the mouse intestine-associated lymphoid organs, at least two functionally distinct DC subsets have been described, according to their expression of the integrin CD103. Only the CD103⁺ DC population displays properties at inducing Foxp3⁺ Treg from FoxP3⁻ T cells via a TFG-β- and retinoic acid-dependent mechanism (Coombes, 2007; Sun, 2007). Another level of specialization of DC subsets has been recently evidenced in the mouse skin. Indeed, in addition to the resident DC of the lymph nodes, the skin contains epidermis-derived CD205^{hi} CD8ₐ⁻ Langerhans cells, CD207⁺ CD205 ^{int} CD8ₐ⁻ conventional dermal DC and a CD207⁺ CD103⁺ dermal DC population. Only the latter is able to cross-present viral and self antigens to naive CD8⁺ T cells (Bedoui, 2009). In the mouse lungs and conducting airways, different DC subsets with functional specialization have been identified, as well. At the steady state, the trachea contains intraepithelial CD11^{b-} CD207⁺ CD103⁺ DC. Under conditions of inflammation, the submucosa of the airways may contain CD11b⁺ CD103-conventional DCs with potential capacity to prime and/or restimulate effector CD4⁺ T cells. In the lung parenchyma CD11c⁺ CD11b⁺ and CD11c⁺ CD11b⁻ DCs are present, can migrate to the alveolar lumen or to mediastinal lymph nodes. Like in the spleen, in the lung parenchyma plasmacytoid DC are detectable, display a CD11c^{int} CD11b⁻ PDCA-1⁺ phenotype and produce large amounts of IFN-a upon *in vitro* TLR triggering (de Heer, 2005).

Numerous evidences argue that the magnitude of adaptive immune responses, as well as differentiation and specialization of CD4⁺ T cells into Th1, Th2 or Th17, are dictated by different DC subsets with specialized activities (Villadangos, 2007) (Steinman, 2008) (Steinman, 2007). Mobilization of different DC subsets by their direct *in vivo* targeting through their specific surface markers represents a promising pathway to design well-controlled immunization strategies for the development of preventive and/or therapeutic vaccines (Steinman, 2008; Shortman, 2009). In this domain, the most significant strategy has been elegantly developed by the teams of Steinman and Nussenzweig, through antigen coupling to antibodies specific for DC surface receptors. In this approach, the ovalbumin (OVA) model antigen or pathogen-derived antigens are coupled or genetically inserted to the NLDC-145 mAb, specific to the C-type lectin endocytic receptor CD205. A single, low dose of this vector, together with an appropriate DC maturation signal, are able to induce robust and long-lasting antibody responses, CD4⁺ and CD8⁺ systemic and mucosal T-cell responses, correlated with an enhanced resistance to tumor growth or to viral infection (Bonifaz, 2002; Boscardin, 2006; Dudziak, 2007) (Trumpfheller, 2006). More recently, another endocytic C-type lectin, i.e., DC, NK lectin Group Receptors-1 (DNGR-1, Clec9A), has been identified by two independent teams. DNGR-1, specifically expressed on mouse CD8ₐ⁺ splenic DC, has been used as targeted DC surface marker, (i) in the absence of adjuvant, for efficient induction of humoral immunity (Caminschi, 2008) and, (ii) in the presence of anti-CD40 agonistic mAb as adjuvant, for induction of OVA-specific CD4⁺ and CD8⁺ T cells, with successful preventive and therapeutic effect against OVA-expressing melanoma tumor cells (Sancho, 2008). Another C-type lectin Clec12A, highly expressed on splenic CD8⁺ DC and plasmacytoid DC has been used as targeted DC surface receptor and induces Ab responses, in the presence of minimal amounts of adjuvant (Lahoud, 2009).

So far, the antigen targeting strategy is limited by the requirement of individual chemical coupling or genetic insertion of each immunogen of interest to mAbs specific to each of the numerous DC surface receptors, candidate for antigen addressing. Even though it is largely admitted that DC translate information from different surface receptors into an activation program that orients the Th cell differentiation, since all the rules governing functions of DC subsets are not yet understood, it remains difficult to predict which DC subsets/DC surface receptors are the most appropriate to be targeted in order to optimize the protective immunity against a given pathogen. Therefore, comparison of the properties and impacts of various DC subsets on the generation of pathogen-specific adaptive responses may help identify the most adapted DC subset(s), able to tailor the most adapted and protective adaptive immunity. To this end, here we designed a versatile approach to identify the most appropriate DC receptor(s) to which the targeting of relevant *M. tuberculosis-de rived* immunogens can induce optimized immune responses with anti-tuberculosis protective potential. In this approach, prominent mycobacterial immunogens are genetically fused to streptavidin (SA). The resulting fusion proteins are tetramerized to optimize their high affinity interaction with biotin (biot). Such SA fusion tetramers are then complexed to biot-conjugated mAbs, specific to diverse DC surface receptors. Therefore, in this flexible model, once such antigen-SA fusion proteins are produced, they can be readily carried and delivered to different DC subsets by simple use of individual biot-mAbs of a large panel of specificities against DC surface receptors, with expression profiles restricted to given DC subsets.

Potent mycobacterial antigens included in this study were selected among highly-conserved, low-molecular weight immunogens belonging to the Early Secreted Antigenic Target, 6 kDa (ESAT-6) protein family (ESX) of *M. tuberculosis* (Brodin, 2004). These proteins, actively secreted by the type VII secretion system of mycobacteria (Simeone, 2009), are known for their marked immunogenicity in mice, guinea pig and in ethnically different human populations, and for their protective potential in animal tuberculosis models (Brodin, 2004). Moreover, the presence of CD4⁺ and CD8⁺ effector T cells specific to such proteins is directly correlated to the natural anti-mycobacterial protection in *M. tuberculosis-infected* humans. We characterized the immunogenicity of several ESX proteins fused to SA (ESX-SA), targeted to different DC surface receptors by complexing them to biot-mAbs specific to MHC-II molecules, CD11b or CD11c β2 integrins, PDCA-1 or diverse C-type lectins. The latter were chosen from: (i) mannose receptor family, i.e., CD205 (DEC205), (ii) asialoglycoprotein receptor family, i.e., CD207 (Langerin, 37 Clec4K), or CD209 (DC-Specific ICAM3-Grabbing Non-integrin, DC-SIGN), or (iii) DC Immunoreceptor (DCIR) subfamily of asialoglycoproteoin receptor, i.e., DCIR-2 (Clec4A) (Geijtenbeek, 2009). We explored this model to select the most appropriate DC subsets or DC surface receptors to target in anti-tuberculosis vaccination on the basis of capture/endocytosis/processing and presentation of ESX antigens by MHC molecules, *in vivo* outcome of the ESX-specific Th1, Th2, Th17 Treg or CD8⁺ T-cells responses, boost effect of such immunization subsequent to BCG priming and protective potential in the mouse model of M. *tuberculosis* infection.

### B. Material and Methods

### Recombinant ESX-SA fusion proteins, Peptides

The *E. coli* codon-optimizeds synthetic gene encoding residues 14-139 of streptavidin from *Streptomyces avidinii* was obtained from GenScript (NJ, USA) and inserted into the pET28b expression vector (Novagen, Darmstadt, Germany). The genes for TB antigens *cfp-10, esat-6* and *tb10.4* were PCR-amplified using pairs of PCR primers indicated in Table 1 and genetically fused to the 5'-end of the streptavidin gene by insertion into the *Nc*oI, *Nhe*I and *Eco*RI sites. The exact sequence of the cloned inserts was verified by DNA sequencing. The plasmids were transformed in to *E. coli* cells for IPTG inducible production of proteins.

CFP-10-SA protein was produced in *E. coli* BL21 λDE3 cells (Stratagene, La Jolla, Canada) at 20 °C, while *E. coli* Artic Express DE3 cells (Stratagene, La Jolla, Canada) was used for production of ESAT-6-SA and TB10.4-SA proteins. In the latter case, cells were grown at 28 °C to A₆ₒₒ 0.8 before temperature was lowered to 10°C and production of proteins was induced by addition of IPTG to 0.5 mM final concentration. Cells were grown in LB medium containing 60 µg/ml kanamycin and 20 µg/ml gentamycin (for *E. coli* Arctic express only).

The cells were harvested and lyzed by ultrasonic disruption. CFP-10-SA was purified directly from soluble cytosolic extract, while ESAT-6-SA and TB10.4-SA tetramers were solubilized from cell debris by extraction with 2 M Urea, respectively. The extracts were loaded on IminoBiotin-Agarose (Sigma) columns equilibrated in 50 mM CH₃COONH₄ buffered with NH₃*H₂O to pH 9. The columns were washed with several bed volumes of 0.1 M acetic acid, 0.5 M NaCl pH 3 and eluted using 0.1 M acetic acid without salt pH 3, with immediate neutralization of acetic acids by addition of 1/50 of fraction volume of 25% NH₃*H₂O to reach a final pH 9. The proteins were concentrated on spin columns (Millipore, Bedford, MA, USA) and contaminating lipopolysacharide was removed by passage through EndoTrap column (Profos, Regensburg, Germany) to reduce its level below 50 EU of LPS/mg of protein, as assessed using the endotoxin chromogenic LAL test assay kit (Lonza, Walkersville, MD, USA). Formation of the tetramers was controlled using Tris-Tricine SDS-PAGE gels (15%). Biotin binding was controlled in Western blots by detection of biotinylated marker proteins with antigen-SA fusions that were themselves detected by a sandwich of antigen-specific polyclonal sera and anti-rabbit-peroxidase conjugate.

The synthetic peptides ESAT-6:1-20 (Brandt, 1996), Culture Filtered Protein, 10 kDa (CFP-10):11-25 (Kamath, 2004), TB10.3/4:20-28 (Majlessi, 2003), and TB10.4:74-88 (Hervas-Stubbs, 2006) peptides were all synthesized by NeoMPS (Strasbourg, France).

### Biotinylated mAbs specific to DC surface receptors

mAbs specific to CD11b (clone M1/70.15.11.5.HL, rat IgG_{2b}, ATTC-TIB-12), CD11c (clone N418, Armenian hamster IgG, ATTC-HB-224), DCIR-2 (clone 33D1, rat IgG_{2b}, ATCC-TIB-227) or to MHC-II (I-A/I-E) (clone M5/114.15.2, rat IgG_{2b}) or the control lg (clone R187, rat IgG, ATCC-CRL-1912) were prepared from supernatants of B-cell hybridomas, cultured in serum-free, synthetic HL-1 medium (Lonza BioWhittaker, Walkersville, MD) complemented with 2 mM L-glutamax, 5 x 10⁻⁵ M ß-mercapto-ethanol, 100 IU/ml penicillin and 100 µg/ml streptomycin. Supernatants were treated with (NH₄)₂SO₄, prepared in sterile water for injection (Baxter, Maurepas, France), at 50% final concentration at 4°C in endotoxin-free conditions, as described elsewhere (Jaron, 2008). The precipitated proteins were extensively dialyzed against PBS and sterilized by filtration through 0.2 µm filters. Absence of endotoxins in the lg preparations was then checked by use of "Limulus Amebocytes Lysate" kit (Cambrex, Emerainville, France), with a detection limit of 0.01 IU/ml. lg were biotinylated by use of EZ-Link Sulfo-NHS-LC kit (Pierce, Rockford, IL), according to the manufacture's protocol, and under stoechiometric conditions leading to fixation of 2 moles of biotine per mole of lg. Biot-anti-CD205 mAb (clone NLDC-145, rat IgG₂ₐ) was purchased from Celldex Therapeutics (Needham, USA). (Czech Republic). Biot-mAbs specific to CD207 (Langerin) (clone eBioL31, rat IgG₂ₐ), CD209 (DC-SIGN) (clone LWC06, rat IgG₂ₐ) or CD317 (PDCA-1) (clone eBio927, rat IgG_{2b}) were purchased from eBioscience (San Diego, CA).

### Detection of ESAT-6 binding to DC surface receptors

Conventional or plasmacytoid DC were generated from femur-derived hematopoietic precursors, respectively, in the presence of GM-CSF or Flt3L, as previously described (Mouries, 2008). BM-DC (1 x 10⁶ cells/well) were incubated at 4°C with 1.5 µg/ml of biot-mAbs specific to DC surface markers or of biotin-conjugated control lg isotypes. Cells were then washed at 4°C and incubated with 1 µM (= 21 µg/ml) of ESAT-6-SA for 1 h at 4°C. Cells were washed three times at 4°C and were then either left at 4°C or incubated for 3 h at 37°C to evaluate the possible internalization. The presence of ESAT-6 at the cell surface was detected by cytofluorometry, by use of the anti-ESAT-6 mAb (clone 11 G4) (Antibody Shop, Gentoft, Denmark), labeled with the pH sensitive Alexa647H, by use of FluoProbs protein labeling kit (Interchim, Montluçon, France). Percentages of the reduction in the MFI of the cell surface bound ESAT-6 signal was calculated as 100 - [(MFI biot-mAb + ESAT-6-SA 37°C) - (MFI biot-control lg + ESAT-6-SA 4°C) / (MFI biot-mAb + ESAT-6-SA 4°C) - (MFI biot-control lg + ESAT-6-SA 4°c)] x 100.

*In vivo* binding of ESAT-6 to spleen DC subsets was studied at different time points after i.v. injection of ESAT-6-SA, complexed to biot-mAbs or to biot-control lg, in the presence of Poly Inosinic:Poly Cytidylic acid (Poly I:C). Spleen low density cells were prepared by use of iodixanol gradient medium (OptiPrep, Axis-Shield, Dundee, UK). Briefly, collagenase-DNase-treated spleens were homogenized and splenocytes were suspended in 15% iodixanol and layered with 11.5% iodixanol. After centrifugation, low density cells recovered from the top of the gradient were stained with a combination of DC-specific and Alexa647H-anti-ESAT-6 mAbs, prior to analysis by cytoflurometry.

### T-cell hybridomas specific to ESX antigens

ESAT-6:1-20-specific, I-A^{b}-restricted, NB11 T-cell hybridoma has been recently described (Frigui, 2008). TB10.4:74-88-specific T-cell hybridomas were generated from BALB/c (H-2^{d}) mice, immunized s.c. with 1 x 10⁷ CFU of BCG. Two weeks after the immunization, total splenocytes and inguinal lymph node cells were pooled and stimulated *in* vitro with 10 µg/ml of TB10.4:74-88 peptide. At day 4, viable cells were harvested on Lympholyte M (Cedarlane Laboratories) and were fused, at 1:1 ratio, with BW51-47 thymoma cells by use of polyethylene glycol 1500 (Roche Diagnostics), as previously described (Majlessi, 2006). T-cell hybridomas were first individually expanded and screened for their capacity to release IL-2 upon recognition of TB10.4:74-88 peptide, presented by syngenic BM-DC. The positive T-cell hybridomas were then screened for their capacity to recognize BM-DC incubated with the recombinant TB10.4 protein (Hervas-Stubbs, 2006) or BM-DC infected with BCG for 24h at m.o.i of 1, in antibiotic-free conditions. The presence of IL-2 in the supernatants of the co-cultures of BM-DC and T-cell hybridomas was assessed by a standard IL-2-specific ELISA. L fibroblasts, transfected with I-A^{d}, I-E^{d} or I-A^{b} restricting elements, were used as peptide presenting cells, in the same type of assay to determine the H-2 restriction of the presentation to the T-cell hybridomas. A selected T-cell hybridoma (1H2), specific to TB10.4:74-88 and restricted by I-A^{d}, was used in *in vitro* and *ex vivo* presentation assays of TB10.4 antigen delivery to DC.

### In vitro and ex vivo antigen presentation assays

BM-derived macrophages (fully adherent CD11 c-CD11 b+ cells), BM-derived conventional DC (semi-adherent CD11c⁺CD11b⁺ cells) or BM-derived plasmacytoid DC (CD11c^{int}B220⁺PDCA-1⁺), as previously described (Mouries, 2008), (1 x 10⁵ cells/well) were incubated with 1.5 µg/ml of biot-mAbs specific to diverse markers of DC for 30 min at 4°C. Cells were then washed and incubated with various concentrations of ESX-SA fusion proteins. Cells were then washed again extensively at 4°C and co-cultured overnight with 1 x 10⁵ cells/well of appropriate T-cell hybridomas. The efficiency of antigen presentation was judged by the evaluation of IL-2 produced in the co-culture supernatants by ELISA. When indicated, the efficiency of ESX antigen presentation was measured by use of polyclonal T cells from M. *tuberculosis* infected C57BU6 mice, prepared by positive magnetic sorting of Thy-1.2⁺ T splenocytes by use of anti-Thy-1.2-mAb-conjugated magnetic microbeads and AutoMacs Pro (Miltenyi Biotec, Bergisch-Gladbach, Germany) and by use of Possel-D program. In this case, the supernatants of co-cultures were assessed for IFN-γ by ELISA.

For *ex vivo* antigen presentation assays, BALB/c mice were injected i.v. with 50 pmoles (= 1 µg)/mouse of TB10.4-SA, complexed to biot-mAbs, in the presence of 25 µg/mouse of Poly I:C. At different time points post-injection, low density cells were prepared from the spleen of the injected mice, as detailed above, and were stained with anti-biot mAb-coupled to magnetic microbeads (Miltenyi Biotec) for further positive selection of cells targeted *in vivo* by TB10.4-SA-biot-mAb complex. Cells were then magnetically sorted on AutoMacs Pro by use of Possel-S program. Various numbers of cells contained in positive or negative fractions were co-cultured with anti-TB10.4:74-88 1H2 T-cell hybridoma and IL-2 was assessed in their co-culture supernatants after 24h incubation.

### Mice and immunization

Female BALB/c (H-2^{d}), C57BU6 (H-2^{b}) and C3H (H-2^{k}) mice were purchased from Charles Rivers (Arbresle, France) and were immunized at 6-12-week-old. Tetramers of ESX-SA fusion proteins and biot-conjugated mAbs or appropriate biot-conjugated control lg were mixed at a ratio of 2:1 at molar basis, at the indicated doses, and were complexed by incubation at 4°C for 1 h. The final mixture was injected i.v. in 200 µl/mouse in the presence of 25 µg/mouse of Poly I:C. Immunization with BCG (Pasteur 1173P2 strain) was performed by s.c. injection at the basis of the tail. C57BU6 FcyR^{%} mice, deficient for activating FcγRl, III, IV receptors (Takai, 1994), were kindly provided by Pierre Bruhms and Marc Daeron (Institut Pasteur, Paris). C57BU6 CD11c YFP mice (Lindquist, 2004) were kindly provided by Philippe Bousso (institut Paster, Paris). Treg attenuation was performed by an i.p. injection of 1 mg/mouse of anti-CD25 mAb (clone PC61) or of control lg at day -2 before immunization. All animal studies were approved by the Institut Pasteur Safety Committee, in accordance with the national law and European guidelines.

### T-cell assays

CD4⁺ T-cell assays were performed on splenocytes from individual immunized mice. Cells were cultured in complete HL-1 medium in the presence of various concentrations of appropriate ESX-derived peptides, harboring MHC-11-restricted immunodominant T-cell epitopes or mycobacterial-derived Ag85A:101-120 or Ag85A:241-260, as negative control peptides, respectively in H-2^{d} or H-2^{b} haplotype. Supernatants of such cultures were assessed at 24 h post-incubation for the presence of lL-2 and at 72 h for lL-5 and IFN-γ, as previously described (Jaron, 2008). IL-17A was also quantified at 72 h by ELISA by use of anti-IL-17 mAb (clone 50104) for coating and biot-anti-IL-17 mAb (clone BAF421) from R&D system for the detection.

To evaluate CD8⁺ T-cell responses, total splenocytes from immunized mice were stimulated *in vitro* with 10 µg/ml of TB10.3/4:20-28 peptide in RPMI, complemented with 2 mM L-glutamax, 5 x 10⁻⁵ M ß-mercapto-ethanol, 100 IU/mi penicillin, 100 µg/m) streptomycin and 10% FCS. At day 6, detection of CD8⁺ T lymphocytes, specific to the TB10.3/4:20-28 epitope, was performed by cytofluorometry, by use of a PE-conjugated pentamer of H-2K^{d}, complexed to TB10.3/4:20-28 peptide (Proimmune, Oxford, UK), in the presence of FITC-conjugated anti-CD8_{α} (clone 53-6.7) and allophycocyanin-conjugated anti-CD44 (clone IM7) mAbs, purchased from BD/PharMingen, (Le Pont de Claix, France). Dead cells were excluded by gating out the Pl⁺ cells. Cells were analyzed in a FacsCalibur system (Becton Dickinson, Grenoble, France) by use of FlowJo program.

### Protection assay against infection with M. tuberculosis

*M. tuberculosis* H37Rv was grown at 37°C in Dubos broth (Difco, Becton Dickinson, Sparks, MD), complemented with albumin, dextrose and catalase (ADC, Difco). BALB/c (H-2^{d}) mice (n = 6) were primed by BCG (1 x 10⁴ CFU/mouse, s.c.) at day 0 and then boosted at day 14 and 21 by 50 pmoles (=1 µg)/mouse of TB10.4-SA complexed with 25 pmoles (=3.6 µg)/mouse of biot-control lg or biot-mAbs specific to various DC surface receptors, in the presence of 25 µg of Poly I:C. Mice were challenged at day 28 with M. *tuberculosis* H37Rv, via the aerosol route, by use of a home-made nebulizor. Five ml of a suspension containing 5 x 10⁶ CFU/ml were aerosolized to obtain an inhaled dose ranged from 100 to 200 CFU/mouse. Four weeks post-challenge, lungs and spleens were homogenized by use of 2.5-mm diameter glass beads and an MM300 organ homogenizer (Qiagen, Courtaboeuf, France). Serial 5-fold dilutions of homogenates were seeded on 7H11 Agar, supplemented with Ovalbumin ADC (OADC, Difco). CFU were counted after 18 days of incubation at 37°C. Mice infected with M. *tuberculosis* H37Rv were housed in isolator and manipulated in A3 animal facilities at Institut Pasteur.

### C. Results

### C.1. Construction of ESX proteins genetically fused to SA

The complete polyeptide sequences of mycobacterial antigens from the ESX protein family, i.e., ESAT-6 (ESX A, Rv3875), CFP-10 (ESX B, Rv3874) or TB10.4 (ESX H, Rv0288), were genetically fused to SA to generate protein that formed tetramers and could be combined with biot-mAbs specific to DC surface markers. For our purpose we used only residues 14 to 139 of streptavidin from *Streptomyces avidinii.* The N-terminal part of SA was replaced by fused MTB antigens. Importantly, the presence of the complete sequence of ESAT-6, CFP-10 or TB10.4 at the N-terminal part of the SA did not perturb the tetramerization capacity of the fusion constructs (Figure 7A). This was of utmost importance in our approach, as only the tetramers of SA have the substantial affinity of 1 x 10⁻¹⁵ M for biotin (Howarth, 2006). Culture conditions of the producing *E. coli cells,* such as temperature were optimized, in order to obtain production of assembled tetramers already during the expression in bacterial cells, so that the soluble extracted tetrameric proteins could be separated from the monomers by affinity chromatography on IminoBiotin agarose.

### C.2. Highly efficient Ab-mediated binding of ESX-SA fusion proteins to DC surface receptors and their marked endocytosis

We first evaluated the binding of tetramerized ESAT-6-SA fusion protein to DC, through biot-conjugated mAbs specific to various DC surface receptors. Conventional BM-DC, pre-incubated at 4°C with biot-mAbs specific to CD11b, CD11c, MHC-II or DCIR-2, and then with ESAT-6-SA, displayed a marked binding of ESAT-6 at their cell surface, as detected by Alexa647H-anti-ESAT-6 mAb (Figure 7B), and as compared to BM-DC pre-incubated with the irrelevant biot-control lg, showing the specificity of antigen binding to the selected DC surface markers. After 3h incubation at 37°C, the ESAT-6-specific surface fluorescence intensity was substantially reduced, as calculated on the basis of ESAT-6-specific MFI at 4 °C *versus* MFI after 3h incubation at 37°C (Figure 7B). These results strongly suggest that ESAT-6 bound to CD11b, CD11c, MHC-II or DCIR-2 was endocytosed by BM-DC. Plasmacytoid BM-DC, incubated with biot-anti-PDCA-1 mAb, and then with ESAT-6-SA, also showed a cell surface binding of ESAT-6 at 4°C, followed by its internalization at 37°C (Figure 7B, right). Therefore, tetramerized ESAT-6-SA fusion protein is delivered specifically to DC surface receptors by biot-mAbs of the selected specificities and is internalized by DC via CD11borCD11cintegrins, DClR-2C-type lectin, MHC-II or PDCA-1.

### C.3. Marked ESX antigen delivery to the MHC-II presentation pathway

We then investigated the capacity of BM-derived APC, to which ESX-SA fusion proteins were delivered via CD11b, CD11c, MHC-11 or PDCA-1, to present immunodominant MHC-II-restricted ESX epitopes to specific TCR. C57BU6 (H-2^{b})-derived BM-DC were incubated with biot-mAbs specific to DC markers or with biot-control lg, and then incubated with various concentrations of ESAT-6-SA at 4°C. The cells were extensively washed before being cultured with ESX-specific T cells in order to evaluate the presentation of ESAT-6 bound to the targeted DC surface receptors. The BM-DC initially coated with biot-anti-CD11b, -CD11c or - MHC-II, were able to present efficiently the immunodominant ESAT-6 epitope to the I-A^{b}-restricted, ESAT-6:1-20-specific NB11 T-cell hybridomas (Figure 7C, top) or to polyclonal anti-ESAT-6 T splenocytes from M. *tuberculosis* H37Rv-infected C57BU6 mice (Figure 7C, bottom). Incubation of DC at 4°C with biot-control lg, prior to incubation with ESAT-6-SA and extensive washes, did not lead to MHC-II-restricted ESAT-6 presentation (Figures 7C). Moreover, plasmacytoid BM-DC or BM-MΦ, to which ESAT-6-SA was delivered respectively via PDCA-1 (Figure 7D, left) or CD11b (Figure 7D, right), were also able to highly efficiently stimulate the NB11 T-cell hybridoma. BALB/c (H-2^{d})-derived BM-DC, to which TB10.4-SA was delivered via CD11 b (Figure 7E, left) or MHC-11 (Figure 7E, right), were able to present efficiently the TB10.4 immunodominant epitope to I-A^{d}-restricted, TB10.4:74-88-specific 2H1 T-cell hybridoma. Again, incubation of different APC at 4°C with biot-control lg, before incubation with ESAT-6-SA or TB10.4-SA and extensive washes, did not lead to MHC-II-restricted ESX presentation (Figure 7D, E). These data demonstrate that ESX mycobacterial antigens, delivered and bound to the surface of conventional or plasmacytoid BM-DC or BM-MΦ via CD11b, CD11, MHC-11 or PDCA-1 surface molecules, are able to efficiently gain access to the MHC-11 antigen presentation pathway.

### C.4. In vivo binding of ESX antigens to the surface of DC subsets, targeted by minute amounts of ESX-SA and their highly efficient ex vivo presentation

We then evaluated *in vivo* the specificity of ESX antigen binding to DC subsets, as well as the kinetics and efficiency of their presentation to T cells. CD11cYFP C57BU6 mice were injected i.v. with 500 pmoles (= 10 µg/mouse) of ESAT-6-SA, complexed to biot-anti-CD11c mAb or to biot-control lg, at a molar ratio of 2:1, in the presence of 25 µg of the TLR3 agonist Poly I:C. At different time points post injection, low-density cells were prepared from the spleen and analyzed for the presence of ESAT-6 at the surface of DC by use of Alexa647H-anti-ESAT-6 mAb. CD 11 c YFP cells from the recipients of ESAT-6-SA complexed to biot-anti-CD11cmAb - but not from their counterparts injected with ESAT-6-SA complexed to biot-control lg - stained positively for ESAT-6 at 24h and, at a lesser extent, at 48h post injection (Figure 8A, left). In C57BU6 mice injected with 500 pmoles (= 10 µg)/mouse of ESAT-6-SA complexed to biot-anti-CD11b mAb, the binding of ESAT-6 was only detectable, at 3h post-injection, at the surface of CD11c⁺ CD8α (CD11b⁺) - but not at the surface of CD11c⁺ CD8α⁺ (CD11b⁻) - DC (Figure 8A, right). ESAT-6 signal was no more detectable at 24h post injection in the case of targeting to CD11 b.

To study *in vivo* the efficacy of antigen presentation and the specificity of antigen presentation by the targeted DC subsets, after injection of ESX-SA-biot-mAb complexes, targeted APC were purified and co-cultured with ESX-specific, MHC-II-restricted T-cell hybridoma. BALB/c mice were injected with low dose of 50 pmoles (= 1 µg)/mouse of TB10.4-SA, complexed at a molar ratio of 1:1, to mAbs specific to different DC surface receptors. The molar ratio of 1:1, used in this complex formation left free one of the two moles of biot previously fixed per mole of mAb, making possible the *ex vivo* magnetic sorting of biot-mAb-coated DC by use of an anti-biot mAb coupled to magnetic beads. At 3h post-injection, no TB10.4 presentation was detected with total low-density spleen cells recovered from mice injected with TB10.4-SA complexed to biot-control lg. In contrast, positive fractions of cells from mice injected with TB10.4-SA complexed to biot-mAbs specific to CD11b or CD11c, sorted by use of anti-biot beads, were able to markedly stimulate the anti-TB010.4:74-88, I-A^{d}-restricted, 1H2 T-cell hybridoma (Figure 8B, left). As a functional control to show the accurate state of all the sorted cells, both positive and negative cell fractions were able to present the synthetic TB10.4:74-88 peptide to 1H2 T-cell hybridoma (Figure 8B, right).

### C.5. ESX targeting to different surface DC integrins or C-type lectins efficiently triggers ESX-specific Th1 and Th17 responses

We then evaluated the potential of the ESX antigen targeting to DC subsets in immunization of mice. C57BU6 (H-2^{b}) mice were immunized i.v. by a single injection of 50 pmole (= 1 µg)/mouse of ESAT-6-SA, without lg, or complexed at a molar ratio of 2:1, to biot-control lg or biot-mAbs specific to CD11b or CD11 c integrins, to CD205, CD207 or CD209 C-type lectins or to PDCA-1, in the presence of Poly I:C. Control groups immunized with ESAT-6-SA, either without biot-Ig or complexed to biot-control lg, did not develop T-cell responses to ESAT-6. In contrast, mice immunized with ESAT-6-SA complexed to biot-anti-CD11b, - CD11c or -CD205 mounted specific, intense and sensitive IFN-γ (Figure 8C) and lymphoproliferative (data not shown) responses against the immunodominant MHC-II-restricted ESAT-6:1-20 epitope. ESAT-6 antigen targeting to CD207 or PDCA-1 also induced marked and specific T-cell responses, yet at lesser extent. ESAT-6 targeting to CD209 was not efficient at inducing T-cell responses in this context. Immunization by ESAT-6 targeted to CD11b, CD11 c or CD205 induced weak but specific Th17 responses, as well (Figure 8D). Th17 responses were barely detectable in the case of antigen targeting to CD207 or PDCA-1 and were not detectable in the case of CD209. No IL-5 T-cell responses were detected in any experimental groups (data not shown).

We excluded the possibility that the biot-mAbs operated *in vivo* via the FcR, rather than by their specific DC surface ligands, since biot-control lg, complexed to ESAT-6-SA, did not induce T-cell responses (Figure 8C, D) and, FcRγ^{%} mice mounted lymphoproliferative (Figure 8E, left) and IFN-γ (Figure 8E, right) T-cell responses, comparable to those displayed by their WT counterparts.

We then determined the lowest dose of the antigen complexed to biot-mAb, able to induce significant T-cell responses. Immunization with ESAT-6-SA, complexed to biot-anti-CD11b mAb, at different doses, ranging from 250 to 5 pmoles (= 5 to 0.1 µg)/mouse showed that 50 pmoles (= 1 µg/mouse) were enough to induce IFN-γ, IL-2 and IL-17 - but not IL-5 - responses at their maximal intensities. Moreover, an injection dose as low as 5 pmoles (= 0.1 µg)/mouse of ESAT-6-SA was still able to trigger a significant Th1 and Th17 responses, showing the marked efficiency of the antigen delivery approach.

Notably, lymphoproliferative (Figure 9B, left), IFN-γ, IL-2 (Figure 9B, right) and IL-17 responses, induced by this strategy in the presence of Poly I:C, were under the negative control of Treg, as shown by the significant increase of these responses in PC61 mAb-treated mice compared to their control Ig-treated counterparts, immunized with ESAT-6-SA complexed to biot-anti-CD11b mAb.

TB10.4-SA or CFP-10-SA, complexed to biot-anti-CD11b or -CD205 mAbs were also able to induce strong Th1 responses, respectively in BALB/c (H-2^{d}) (Figure 9C) or C3H (H-2^{k}) (Figure 9D) mice, reinforcing and extending the feasibility of the immunization against ESX mycobacterial antigens by use of the developed strategy in different mouse genetic backgrounds and H-2 haplotypes.

### C.6. Substantial boost effect of ESX antigen targeting to DC surface receptors in BCG-primed mice

Priming with BCG, or with its improved recombinant variants, followed by boosting with efficient subunit vaccines, is considered as the most promising prophylactic anti-tuberculosis vaccination strategy (Kaufmann, 2006). We therefore sought to evaluate and to compare the T-cell responses in BCG-primed mice which were then boosted by ESX antigens targeted to different DC surface receptors by the developed approach. BALB/c mice, unprimed or primed s.c. with 1x10⁶ CFU/mouse of BCG at day 0, were boosted i.v., at days 14 and 21, with 50 pmoles (= 1 µg)/ mouse of TB10.4-SA complexed to biot-control lg or to different biot-mAbs specific to C-type lectins or PDCA-1, in the presence of Poly I:C. We then analyzed IFN-γ CD4⁺ T-cell responses, analyzed at day 28 (Figure 10A). In BCG-unprimed mice, no response was detected after the injection of TB10.4-SA complexed to biot-control lg. In contrast, immunization of BCG-unprimed mice with TB10.4-SA complexed to biot-mAbs specific to CD205, CD207, CD209, CDIR-2 or PDCA-1 induced marked levels of IFN-γ responses, with the highest levels obtained with antigen targeting to CD207 and CD209. In BCG-primed mice, boosting with TB10.4-SA complexed to biot-mAbs specific to CD205, CD207, CD209, DCIR-2 or PDCA-1 significantly increased the response, compared to the injection of BCG-primed mice with TB10.4-SA complexed to the biot-control lg. The boost effects for IFN-γ response were comparable for TB10.4 targeting to CD205, CD207, CD209 or DCIR-2 while the effect for PDCA-1 was slightly weaker.

Th17 CD4⁺ T-cell responses (Figure 10B) were not detectable in BCG-unprimed BALB/c mice, immunized with TB10.4-SA complexed to different biot-mAbs. Weak levels of Th17 response were detected in BCG-primed mice injected with TB10.4-SA complexed to biot-control lg. In contrast, Th17 responses were highly significantly increased when BCG-primed mice were boosted with TB10.4 targeted to CD205, CD207 or PDCA-1 and, in a lesser extent, to CD207 or DCIR-2. It is noteworthy that ESAT-6 targeting to CD209, in unprimed C57BL/6 mice was not inducer of Th1 responses (Figure 8C, D) while, TB10.4-SA targeting to CD209, in BALB/c mice was not only immunogenic but displayed a significant boost effect in BCG-primed mice (Figure 10A, B).

We then analyzed TB10.4-specific CD8⁺ T-cell responses in BALB/c mice, by use of the H-2K^{d} pentamer complexed with TB10.3/4:20-28 GYAGTLQSL epitope, shared by TB10.3 and TB10.4 (TB10.3/4:20-28) (Majlessi, 2003). In mice immunized with two injections of TB10.4-SA complexed to biot-mAbs specific to CD11b, CD207, CD209 or PDCA-1, in the presence of Poly I:C, we did not detect specific CD8⁺ T cells (data not shown). In their counterparts immunized by TB10.4 targeted to CD205, we barely detected specific CD8⁺ T cells (<1% pentamer⁺ cells in the CD8⁺ T-cell compartment) (Figure 10C). In BCG-primed mice, injected with TB10.4-SA complexed to biot-control lg, the percentages of such cells were also very weak (approximately 1% pentamer⁺ cells in the CD8⁺ T-cell subset). In contrast, in BCG-primed mice which were subsequently boosted with TB10.4-SA complexed to biot-anti-CD205 mAb, we detected up to 6% of TB10.3/4:20-28-specific CD8⁺ T-cells (Figure 10C). This observation demonstrates the strong capacity of the developed strategy by antigen targeting to CD205 in the cross-priming of anti-mycobacterial CD8⁺ T-cell responses.

### D. Discussion

Rational design of anti-tuberculosis vaccines is restrained by our lack of exhaustive knowledge in the type of protective immune effectors and in the reasons of the limited efficiency of adaptive T-cell responses in eradication of intracellular tubercle bacilli. Considering that the differentiation and specialization of T-cell effectors is dictated by different DC subsets with specialized activities, immunization by mycobacterial antigen targeting to different DC subsets may provide new insights into the type of the anti-tuberculosis adaptive immunity with protective potential. However, as all the mechanisms governing functions of DC subsets are not yet thoroughly understood, it remains difficult to predict which DC subsets/DC surface receptors are the most appropriate to be targeted in order to optimize the protective immunity against mycobacterial infection. To compare the properties and impacts of various DC subsets on the generation of mycobacteria-specific adaptive immune responses and protection, we developed a versatile approach allowing addressing of selected mycobacterial immunogens to different DC subsets/DC surface receptors. Our experimental approach consists of the genetic fusion of full-length sequences of highly immunogenic mycobacterial antigens from the ESX family, i.e., ESAT-6, CFP-10 and TB10.4, to SA, followed by tetramerization of the resulted fusion proteins which leads to the possibility of complex formation between them and individual biot-mAbs of a wide-ranging panel of specificities against DC surface receptors. Such complexes can thus be used to deliver the ESX immunogens to the selected DC surface receptors for direct comparison of the adaptive immune responses, established via the action of different DC subsets.

We first showed *in vitro* that this approach allows delivery of ESX immunogens to various APC surface receptors, either integrins, C-type lectins, MHC-II or PDCA-1. Importantly, ESX antigens bound to CD11b, CD11c, DCIR-2, MHC-11 or PDCA-1 were efficiently endocytosed, most probably due to the crosslinking of the targeted surface receptors by the biot-mAbs, leading to their capping together with the bound ESX-SA cargo. The ESX antigens were then processed and the derived epitopes were loaded on MHC-11 molecules and presented to specific TCR, in a highly sensitive and efficient manner. Furthermore, the antigen delivery was also highly specific *in vivo,* as only the DC subsets, targeted with ESX-SA complexed to selected biot-mAbs: (i) displayed ESX antigens at their surface, as detected from 3h to 24h after i.v. injection by cytofluorometry using anti-ESX mAb, and (ii) when positively sorted *ex vivo,* were able to present ESX antigens to specific MHC-II-restricted T-cell hybridomas.

Besides efficient presentation of prominent mycobacterial antigens by DC, an appropriate activation of the latter is crucial for proper induction of T-cell *responses. A priori* it is conceivable that agonistic biot-mAbs carrying the ESX-SA, could by themselves give the DC maturation signal to the targeted subset. CD11b and CD11c, heterodimerized with CD18, form respectively αₘβ2 and αxβ2 integrins which are phagocytic receptors for complement coated particles. These β2 integrins are both signal transducing receptors, as their ligation by their natural ligands or by specific mAbs induces phosphorylation of Mitogen-Activated Protein (MAP) kinases and upregulation of DNA-binding activity of NF-κκB, leading notably to the transcription and secretion of lL-1β, Macrophage Inflammatory Proteins (MIP)-1α and MIP-1β and thus may have an important role in the recruitment of other inflammatory cells during initiation of the immune response (Ingalls, 1995, Rezzonico, 2000, Rezzonico, 2001). However, full activation of DC subsequent to surface ligation of CD11b or CD11c with mAbs has not been reported. Hence, it is known that partially activated DC are rather tolerogenic or inducer of Treg (Joffre, 2009). Interaction of C-type lectins with their different natural ligands, i.e., pathogen-derived carbohydrates, may activate the signal transduction pathways and NF-_{K}B nuclear translocation and thereby expression of pro-inflammatory cytokines, i.e., IL-6, IL-12p70 and IL-23 or alternatively may negatively affect TLR-mediated DC activation, leading to IL-10 production and an anti-inflammator y microenvironment (Geijtenbeek, 2009) (Gringhuis, 2009). In contrast, to triggering of C-type lectins by their natural ligands, only few information are available on *in vivo* activation/maturation of DC by mAbs specific to C-type lectins. So far, mAb-mediated antigen targeting to CD205, without co-stimulatory signal, induces a T-cell division followed by T-cell peripheral deletion and tolerance (Bonifaz, 2002), while mAb-mediated antigen targeting to DNGR-1 (Clec9A) is immunogenic and induces high titers of IgG antibody responses (Caminschi, 2008). In contrast, in a parallel investigation, in antigen targeting to DNGR-1, an anti-CD40 agonistic mAb has been systematically used as adjuvant for the generation of CD4⁺ and CD8⁺ T-cell responses (Sancho, 2008). In the absence of more detailed information on the potential of DC activation by mAbs specific to DC surface receptors, for further immunizations by ESX antigen targeting to DC subsets, we opted for systematic use of a DC maturation signal, i.e., the synthetic analog of dsRNA, Poly I:C. This choice was based on the well-established structural interaction of Poly I:C with endosomal TLR3 or with cytosolic dsRNA sensors, i.e., Retinoic acid-Inducible Gene-I (RIG-I) and Melanoma Differentiation-Associated gene-5 (MDA5) RNA helicases, probably expressed by different cell types (Kawai, 2008). Moreover, Poly I:C displays a marked capacity, not only to induce type I IFN, necessary to induce both Th1 and CTL responses, but also to activate NK cells. DC triggered via TLR3 are able to derive Th17 differentiation, as well (Veldhoen, 2006).

By use of different biot-mAbs specific to β2 integrins, diverse C-type lectins or PDCA-1, in the presence of Poly I:C, we directly compared the efficiencies of ESX targeting to different DC subsets/DC surface receptors in the induction of T-cell responses. Remarkably, a single injection of only 1 µg (= 50 pmoles)/mouse of ESX-SA complexed to biot-mAbs specific to CD11b, CD11c or CD205, induced specific, intense and highly sensitive Th1 - but not Th2 - responses. ESX-SA complexed to biot-mAbs specific to DC207 or PDCA-1 induced less intense and less sensitive, yet still marked Th1 responses. The efficient induction of Th1 responses by ESX antigen targeting to β2 integrins is in accordance with: (i) the substantial potential of other CD11b targeting delivery vectors, such as the recombinant adenylate cyclase CyaA of *Bordetella pertussis* in the induction of T-cell immunity against diverse pathogens, including mycobacteria, or tumor antigens (Guermonprez, 2001) (Majlessi, 2006) (Hervas-Stubbs, 2006) (Preville, 2005), and (ii) the notable efficiency of mAb-mediated OVA antigen targeting to CD11c, a much more specific marker of DC, albeit expressed at low levels on activated CTL, NK cells and macrophages of marginal zones. Highly efficient CD4⁺ and CD8⁺ T cell triggering in this case has been explained by the delivery of the antigen towards CD11c⁺ cells both in the marginal zones and to CD11c⁺ cross-presenting DC in the T-cell zone (Kurts, 2008).

Among the C-type lectins evaluated in the present study, CD205 was the most efficient at inducing Th1 cells in primary responses to ESX antigens. This endocytic integral transmembrane mannose receptor, is expressed at high levels by cortical thymic epithelium and DC subsets, including the splenic CD8⁺ DC population. CD205 also may act as a receptor for necrotic and apoptotic cells (Shrimpton, 2009). CD205 is rapidly taken up after binding with carbohydrates. Its cytosolic domain mediates highly efficient endocytosis and recycling through the late endosomes and MHC-II rich compartments, compared to the most of the other surface endocytic receptors, whose ligation induces endocytosis through early and more peripheral endosomes (Jiang, 1995).

The significant efficiency of ESX antigen targeting to CD207 (Langerin, Clec4K) C-type lectin is also in accordance with the results obtained with OVA antigen targeting to this C-type lectin leading to strong proliferative responses of both OT-I or OT-II TCR transgenic T cells (Valladeau, 2002) (Idoyaga, 2008). CD207 is a type II transmembrane endocytic receptor which is highly expressed by the skin immature Langerhans cells and dermal DC, and at much lower levels by spleen CD11c⁺ CD8α⁺ DC. CD207 is detected in an endosomal recycling compartment and is potent inducer of organelles consisting of typical superimposed pentalamellar membranes, i.e. Birbeck granules, and routes endocytosed antigens into these organelles. Maturation of Langerhans DC is concomitant with downregulation of CD207 and disappearance of Birbeck granules (Kissenpfennig, 2005). Most importantly for the anti-*M. tuberculosis* vaccination, CD207 mRNA is detectable in the lungs in mice and in epithelium lining the human airways (Valladeau, 2002) and therefore can be of particular interest in the induction of T cells directly close to the potential site of potential mycobacterial infection.

In contrast to ESX antigen targeting to CD205 and CD207, a single dose injection of ESX-SA complexed to biot-anti-CD209 (DC-SIGN) in C57BU6 mice failed to induce specific Th1 responses but was inefficient in BALB/c mice. The reason of this discrepancy is not yet elucidated. CD209 is expressed by myeloid DC and is involved in upregulation of TLR-induced IL-10 production, yet as a function of its different natural ligands, i.e., mannose or fucose, can induce or inhibit production of IL-12 and IL-6 in human DC (Gringhuis, 2009). Absence of Th1 responses subsequent to immunization with ESX-SA complexed to anti-CD209 mAb ESX suggests that the interaction of the used mAb with mouse CD209 would be anti-inflammatory and not appropriate for the induction of Th1 and Th17 responses.

We also show that ESX antigen targeting to PDCA-1 (CD317) allowed ESX antigen routing to MHC-II machinery of BM-derived plasmacytoid DC *in vitro* and induced marked specific Th1 responses *in vivo.* This surface marker is predominantly expressed by plasmacytoid DC in naive mice. Following viral stimulation, due to the production of type-I/II IFN, PDCA-1 become detectable on other DC subsets, myeloid CD11b⁺ cells, NK, NKT, T and B cells (Blasius, 2006). Thus, we cannot exclude that in the presence of Poly I:C, and thereby efficient production of type I IFN, PDCA-1 would be expressed on other cells than plasmacytoid DC, enlarging the spectrum of cells to which biot-anti-PDCA-1 mAb could deliver ESX.

A large body of data has long established the necessary - but not sufficient - role of Th1 cells and IFN-γ production in the control of mycobacterial infections, while the contribution of Th17 cells and IL-17 remains debatable. Besides the early production of IL-17 by lung TCRγ□ T cells (Lockhart, 2006), CD4⁺ Th17 cells can be readily detected in mice and humans exposed to mycobacteria (Umemura, 2007) (Scriba, 2008). However, IL-23p19°/° mice, with normal Th1 but decreased Th17 responses, develop tuberculosis symptoms similar to those observed in WT mice, with comparable mycobacterial loads (Chackerian, 2006). Moreover, IL-17°/° and WT mice control in a similar manner the growth of M. *bovis* BCG, given at high dose by aerosol route (Umemura, 2007). Therefore, according to these data, compared to Th1 responses, Th17 cells do not seem to contribute directly to the control of primary mycobacterial infections. Nevertheless, in C57BU6 mice vaccinated with the ESAT-6:1-20 peptide, adjuvanted with a strong inducer of Th17 responses, and then challenged with M. *tuberculosis,* Th17 cells populate the lungs 3-4 days before the wave of Th1-cell recruitment and trigger the production of CXCL9, CXCL10 and CXCL11 chemokines, which certainly contribute to the chemo-attraction of Th1 cells (Khader, 2007). These data support at least an indirect role of Th17 in the set up of anti-mycobacterial immunity subsequent to vaccination. Taking in account this observation, in parallel to antigen-specific IFN-γ responses, we followed IL-17-producing specific CD4⁺ T cells in mice immunized by ESX antigen targeting to DC subsets. In mice immunized with a single injection of ESX-SA complexes in the presence of Poly I:C, only targeting to CD11 b and CD11 c integrins or to CD205 C-type lectin, and in a lesser extent to CD207 or PDCA-1, was able to induce Th17 responses. It is interesting to note that the good inducers of Th17 responses were also inducers of the highest Th1 responses, in accordance with the hypothesis that Th17 cells may pave the way for the recruitment/activation of Th1 cells (Khader, 2007).

As priming with live attenuated mycobacteria followed by boosting with subunit vaccines, is of the most promising prophylactic anti-tuberculosis vaccination strategies (Kaufmann, 2006), we also analyzed the boosting potential of ESX antigen targeting to DC subsets by use of TB10.4 (Rv0288, ESX-H) antigen, another promising protective ESX antigen (Hervas-Stubbs, 2006; Dietrich, 2005). This antigen is of higher interest in the development of innovative sub-unit vaccine candidate compared to ESAT-6 and CFP-1 0, due to the importance of the latter in the diagnostic tests. In mice primed with BCG and then boosted with TB10.4-SA targeted to CD205, CD207, CD209 or DCIR-2, a comparable boost effect of IFN-γ responses was obtained. The best boost effect at the level of Th17 response was obtained with TB10.4 targeting to CD205, followed by CD207 and PDCA-1.

We investigated several immunization protocols, i.e., single injection or boost immunization after BCG priming, with TB10.4 antigen targeting to different DC subsets to induce CD8⁺ T-cell priming. Among all the conditions evaluated, we only detected efficient TB10.4-specific CD8⁺ T-cell cross priming, in mice primed with BCG and then boosted with TB10.4 targeted to CD205. In addition to its capacity to shuttle antigens from the extracellular space into a specialized MHC-II rich lysosomal compartments, CD205 is also able to efficiently introduce antigens to the MHC-I processing machinery, in a Transporters of Antigen Presentation (TAP)-dependent manner. So far, compared to the critical role of CD4⁺ T cells, the contribution of CD8⁺ T cells to the protection in experimental tuberculosis was underestimated, probably due to the absence, in mice, of several CD8⁺ T-cell populations, including CD1-restricted CD8⁺ T cells. A recent study, performed in the sensitive model of rhesus macaques, described a previously unappreciated contribution of CD8⁺ T cells. Indeed, Ab-mediated depletion of CD8⁺ T cells in BCG-vaccinated and then M. *tuberculosis-challenged* macaques leads to a marked increase in mycobacterial burden and remarkably less-organized and necrotic granulomas *versus* well-contained granluomas in their control isotype-treated counterparts (Chen, 2009). Therefore, our observation that BCG priming followed by TB10.4-SA targeting to CD205 trigger efficiently CD8⁺ T-cell responses is of major importance in the design of subunit anti-tuberculosis booster vaccine.

The Th1 and Th17 responses induced by ESX antigen targeting, at least to CD11b in the presence of Poly I:C, were under the negative control of Treg. We recently demonstrated that the Th1 responses induced by BCG vaccination were also negatively controlled by Treg and that attenuation of this subset in BCG-immunized BALB/c mice leads to weak, albeit significant and reproducible, improvement of the protection against M. *tuberculosis* aerosol challenge (Jaron, 2008). It will be of major interest to evaluate the Treg activity in the case of ESX antigen targeting to DC subsets in the presence of other co-stimulatory signals. Moreover, our recent observations in the OVA antigen model delivered by latex beads in the presence of a large panel of TLR2 to 9 agonists did not allow selection of an adjuvant minimizing the Treg induction, suggesting that Treg activity is probably not a consequence of the quality of inflammation. It has been hypothesized that indirect and partial maturation of DC induced by cytokines, in a bystander manner, therefore can be the cause of Treg induction, which for the rest, can be protective by avoiding excessive inflammation and tissue damage (Joffre, 2009).

ESX targeting to DC surface receptors allowed substantial reduction of the effective dose of antigen for immunization without impairment of T-cell immunity, as exemplified by the low dose of 5 pmoles (= 0.1 µg)/mouse of ESX-SA, complexed to biot-anti-CD11 b mAbs which induced highly significant ESX-specific Th1 and Th17 responses. It is noteworthy that except for the anti-CD11c mAb which is a hamster IgG, all the other mAbs used in this study were rat IgG, thereby minimizing the risk of introduction of different xenogeneic T helper determinants in the case of different antigen targeting assays and thus making possible the direct comparison of the effect of the different DC surface receptors targeted. Importantly, the facts that: (i) FcγR°/° and WT mice mounted comparable adaptive immune responses to mAb-mediated ESX targeting to DC and (ii) ESX-SA fusion proteins complexed to biot-control lg did not induce detectable adaptive immune responses, show that the mechanism responsible of targeting, endocytosis and further antigen presentation does not involve FcγR.

### REFERENCES

1. Bedoui, S., P. G. Whitney, J. Waithman, L. Eidsmo, L. Wakim, I. Caminschi, R. S. Allan, M. Wojtasiak, K. Shortman, F. R. Carbone, A. G. Brooks, and W. R. Heath. 2009. Cross-presentation of viral and self antigens by skin-derived CD103+ dendritic cells. Nature immunology 10:488-495.
2. Blasius, A. L., E. Giurisato, M. Cella, R. D. Schreiber, A. S. Shaw, and M. Colonna. 2006. Bone marrow stromal cell antigen 2 is a specific marker of type I IFN-producing cells in the naive mouse, but a promiscuous cell surface antigen following IFN stimulation. J lmmunol 177:3260-3265.
3. Bonifaz, L., D. Bonnyay, K. Mahnke, M. Rivera, M. C. Nussenzweig, and R. M. Steinman. 2002. Efficient targeting of protein antigen to the dendritic cell receptor DEC-205 in the steady state leads to antigen presentation on major histocompatibility complex class I products and peripheral CD8+ T cell tolerance. The Journal of experimental medicine 196:1627-1638.
4. Boscardin, S. B., J. C. Hafalla, R. F. Masilamani, A. O. Kamphorst, H. A. Zebroski, U. Rai, A. Morrot, F. Zavala, R. M. Steinman, R. S. Nussenzweig, and M. C. Nussenzweig. 2006. Antigen targeting to dendritic cells elicits long-lived T cell help for antibody responses. The Journal of experimental medicine 203:599-606.
5. Brandt, L., T. Oettinger, A. Holm, A. B. Andersen, and P. Andersen. 1996. Key epitopes on the ESAT-6 antigen recognized in mice during the recall of protective immunity to Mycobacterium tuberculosis. J Immunol 157:3527-3533.
6. Brodin, P., I. Rosenkrands, P. Andersen, S. T. Cole, and R. Brosch. 2004. ESAT-6 proteins: protective antigens and virulence factors? Trends in microbiology 12:500-508.
7. Caminschi, I., A. I. Proietto, F. Ahmet, S. Kitsoulis, J. S. Teh, J. C. Lo, A. Rizzitelli, L. Wu, D. Vremec, S. L. van Dommelen, I. K. Campbell, E. Maraskovsky, H. Braley, G. M. Davey, P. Mottram, N. van de Velde, K. Jensen, A. M. Lew, M. D. Wright, W. R. Heath, K. Shortman, and M. H. Lahoud. 2008. The dendritic cell subtype restricted C-type lectin Clec9A is a target for vaccine enhancement. Blood.
8. Castro, F. V., A. L. Tutt, A. L. White, J. L. Teeling, S. James, R. R. French, and M. J. Glennie. 2008. CD11c provides an effective immunotarget for the generation of both CD4 and CD8 T cell responses. European journal of immunology 38:2263-2273.
9. Chackerian, A. A., S. J. Chen, S. J. Brodie, J. D. Mattson, T. K. McClanahan, R. A. Kastelein, and E. P. Bowman. 2006. Neutralization or absence of the interleukin-23 pathway does not compromise immunity to mycobacterial infection. Infect Immun 74:6092-6099.
10. Chen, C. Y., D. Huang, R. C. Wang, L. Shen, G. Zeng, S. Yao, Y. Shen, L. Halliday, J. Fortman, M. McAllister, J. Estep, R. Hunt, D. Vasconcelos, G. Du, S. A. Porcelli, M. H. Larsen, W. R. Jacobs, Jr., B. F. Haynes, N. L. Letvin, and Z. W. Chen. 2009. A critical role for CD8 T cells in a nonhuman primate model of tuberculosis. PLoS pathogens 5:e1000392.
11. Coombes, J. L., K. R. Siddiqui, C. V. Arancibia-Carcamo, J. Hall, C. M. Sun, Y. Belkaid, and F. Powrie. 2007. A functionally specialized population of mucosal CD103+ DCs induces Foxp3+ regulatory T cells via a TGF-beta and retinoic acid-dependent mechanism. The Journal of experimental medicine 204:1757-1764.
12. de Heer, H. J., H. Hammad, M. Kool, and B. N. Lambrecht. 2005. Dendritic cell subsets and immune regulation in the lung. Seminars in immunology 17:295-303.
13. Dickgreber, N., P. Stoitzner, Y. Bai, K. M. Price, K. J. Farrand, K. Manning, C. E. Angel, P. R. Dunbar, F. Ronchese, J. D. Fraser, B. T. Backstrom, and I. F. Hermans. 2009. Targeting antigen to MHC class II molecules promotes efficient cross-presentation and enhances immunotherapy. J lmmunol 182:1260-1269.
14. Dietrich, J., C. Aagaard, R. Leah, A. W. Olsen, A. Stryhn, T. M. Doherty, and P. Andersen. 2005. Exchanging ESAT6 with TB10.4 in an Ag85B fusion molecule-based tuberculosis subunit vaccine: efficient protection and ESAT6-based sensitive monitoring of vaccine efficacy. J Immunol 174:6332-6339.
15. Dudziak, D., A. O. Kamphorst, G. F. Heidkamp, V. R. Buchholz, C. Trumpfheller, S. Yamazaki, C. Cheong, K. Liu, H. W. Lee, C. G. Park, R. M. Steinman, and M. C. Nussenzweig. 2007. Differential antigen processing by dendritic cell subsets in vivo. Science (New York, N.Y 315:107-111.
16. Frigui, W., D. Bottai, L. Majlessi, M. Monot, E. Josselin, P. Brodin, T. Garnier, B. Gicquel, C. Martin, C. Leclerc, S. T. Cole, and R. Brosch. 2008. Control of M. tuberculosis ESAT-6 secretion and specific T cell recognition by PhoP. PLoS pathogens 4:e33.
17. Geijtenbeek, T. B., and S. I. Gringhuis. 2009. Signalling through C-type lectin receptors: shaping immune responses. Nature reviews 9:465-479.
18. GeurtsvanKessel, C. H., M. A. Willart, L. S. van Rijt, F. Muskens, M. Kool, C. Baas, K. Thielemans, C. Bennett, B. E. Clausen, H. C. Hoogsteden, A. D. Osterhaus, G. F. Rimmelzwaan, and B. N. Lambrecht. 2008. Clearance of influenza virus from the lung depends on migratory langerin+CD11 b- but not plasmacytoid dendritic cells. The Journal of experimental medicine 205:1621-1634.
19. Gringhuis, S. I., J. den Dunnen, M. Litjens, M. van der Vlist, and T. B. Geijtenbeek. 2009. Carbohydrate-specific signaling through the DC-SIGN signalosome tailors immunity to Mycobacterium tuberculosis, HIV-1 and Helicobacter pylori. Nature immunology 10:1081-1088.
20. Guermonprez, P., N. Khelef, E. Blouin, P. Rieu, P. Ricciardi-Castagnoli, N. Guiso, D. Ladant, and C. Leclerc. 2001. The adenylate cyclase toxin of Bordetella pertussis binds to target cells via the alpha(M)beta(2) integrin (CD11 b/CD18). The Journal of experimental medicine 193:1035-1044.
21. Hervas-Stubbs, S., L. Majlessi, M. Simsova, J. Morova, M. J. Rojas, C. Nouze, P. Brodin, P. Sebo, and C. Leclerc. 2006. High frequency of CD4+ T cells specific for the TB10.4 protein correlates with protection against Mycobacterium tuberculosis infection. Infect lmmun 74:3396-3407.
22. Howarth, M., D. J. Chinnapen, K. Gerrow, P. C. Dorrestein, M. R. Grandy, N. L. Kelleher, A. El-Husseini, and A. Y. Ting. 2006. A monovalent streptavidin with a single femtomolar biotin binding site. Nature methods 3:267-273.
23. Idoyaga, J., C. Cheong, K. Suda, N. Suda, J. Y. Kim, H. Lee, C. G. Park, and R. M. Steinman. 2008. Cutting edge: langerin/CD207 receptor on dendritic cells mediates efficient antigen presentation on MHC I and II products in vivo. J Immunol 180:3647-3650.
24. Ingalls, R. R., and D. T. Golenbock. 1995. CD11c/CD18, a transmembrane signaling receptor for lipopolysaccharide. The Journal of experimental medicine 181:1473-1479.
25. Jaron, B., E. Maranghi, C. Leclerc, and L. Majlessi. 2008. Effect of attenuation of Treg during BCG immunization on anti-mycobacterial Th1 responses and protection against Mycobacterium tuberculosis. PloS one 3:e2833.
26. Jiang, W., W. J. Swiggard, C. Heufler, M. Peng, A. Mirza, R. M. Steinman, and M. C. Nussenzweig. 1995. The receptor DEC-205 expressed by dendritic cells and thymic epithelial cells is involved in antigen processing. Nature 375:151-155.
27. Joffre, O., M. A. Nolte, R. Sporri, and C. Reis e Sousa. 2009. Inflammatory signals in dendritic cell activation and the induction of adaptive immunity. Immunological reviews 227:234-247.
28. Johnson, T. S., K. Mahnke, V. Storn, K. Schonfeld, S. Ring, D. M. Nettelbeck, H. J. Haisma, F. Le Gall, R. E. Kontermann, and A. H. Enk. 2008. Inhibition of melanoma growth by targeting of antigen to dendritic cells via an anti-DEC-205 single-chain fragment variable molecule. Clin Cancer Res 14:8169-8177.
29. Kamath, A. B., J. Woodworth, X. Xiong, C. Taylor, Y. Weng, and S. M. Behar. 2004. Cytolytic CD8+ T cells recognizing CFP10 are recruited to the lung after Mycobacterium tuberculosis infection. The Journal of experimental medicine 200:1479-1489.
30. Kaufmann, S. H., S. Baumann, and A. Nasser Eddine. 2006. Exploiting immunology and molecular genetics for rational vaccine design against tuberculosis. Int J Tuberc Lung Dis 10:1068-1079.
31. Kawai, T., and S. Akira. 2008. Toll-like receptor and RIG-I-like receptor signaling. Annals of the New York Academy of Sciences 1143:1-20.
32. Khader, S. A., G. K. Bell, J. E. Pearl, J. J. Fountain, J. Rangel-Moreno, G. E. Cilley, F. Shen, S. M. Eaton, S. L. Gaffen, S. L. Swain, R. M. Locksley, L. Haynes, T. D. Randall, and A. M. Cooper. 2007. IL-23 and IL-17 in the establishment of protective pulmonary CD4+ T cell responses after vaccination and during Mycobacterium tuberculosis challenge. Nature immunology 8:369-377.
33. Kissenpfennig, A., S. Ait-Yahia, V. Clair-Moninot, H. Stossel, E. Badell, Y. Bordat, J. L. Pooley, T. Lang, E. Prina, I. Coste, O. Gresser, T. Renno, N. Winter, G. Milon, K. Shortman, N. Romani, S. Lebecque, B. Malissen, S. Saeland, and P. Douillard. 2005. Disruption of the langerin/CD207 gene abolishes Birbeck granules without a marked loss of Langerhans cell function. Molecular and cellular biology 25:88-99.
34. Kreiter, S., A. Selmi, M. Diken, M. Sebastian, P. Osterloh, H. Schild, C. Huber, O. Tureci, and U. Sahin. 2008. Increased antigen presentation efficiency by coupling antigens to MHC class I trafficking signals. J Immunol 180:309-318.
35. Kurts, C. 2008. CD11c: not merely a murine DC marker, but also a useful vaccination target. European journal of immunology 38:2072-2075.
36. Lahoud, M. H., A. I. Proietto, F. Ahmet, S. Kitsoulis, L. Eidsmo, L. Wu, P. Sathe, S. Pietersz, H. W. Chang, I. D. Walker, E. Maraskovsky, H. Braley, A. M. Lew, M. D. Wright, W. R. Heath, K. Shortman, and I. Caminschi. 2009. The C-type lectin Clec12A present on mouse and human dendritic cells can serve as a target for antigen delivery and enhancement of antibody responses. J lmmunol 182:7587-7594.
37. Lindquist, R. L., G. Shakhar, D. Dudziak, H. Wardemann, T. Eisenreich, M. L. Dustin, and M. C. Nussenzweig. 2004. Visualizing dendritic cell networks in vivo. Nature immunology 5:1243-1250.
38. Lockhart, E., A. M. Green, and J. L. Flynn. 2006. IL-17 production is dominated by gammadelta T cells rather than CD4 T cells during Mycobacterium tuberculosis infection. J Immunol 177:4662-4669.
39. Majlessi, L., P. Brodin, R. Brosch, M. J. Rojas, H. Khun, M. Huerre, S. T. Cole, and C. Leclerc. 2005. Influence of ESAT-6 secretion system 1 (RD1) of Mycobacterium tuberculosis on the interaction between mycobacteria and the host immune system. J Immunol 174:3570-3579.
40. Majlessi, L., B. Combaluzier, I. Albrecht, J. E. Garcia, C. Nouze, J. Pieters, and C. Leclerc. 2007. Inhibition of phagosome maturation by mycobacteria does not interfere with presentation of mycobacterial antigens by MHC molecules. J Immunol 179:1825-1833.
41. Majlessi, L., M. J. Rojas, P. Brodin, and C. Leclerc. 2003. CD8+-T-cell responses of Mycobacterium-infected mice to a newly identified major histocompatibility complex class I-restricted epitope shared by proteins of the ESAT-6 family. Infect Immun 71:7173-7177.
42. Majlessi, L., M. Simsova, Z. Jarvis, P. Brodin, M. J. Rojas, C. Bauche, C. Nouze, D. Ladant, S. T. Cole, P. Sebo, and C. Leclerc. 2006. An increase in antimycobacterial Th1-cell responses by prime-boost protocols of immunization does not enhance protection against tuberculosis. Infect Immun 74:2128-2137.
43. Meyer-Wentrup, F., D. Benitez-Ribas, P. J. Tacken, C. J. Punt, C. G. Figdor, I. J. de Vries, and G. J. Adema. 2008. Targeting DCIR on human plasmacytoid dendritic cells results in antigen presentation and inhibits IFN-alpha production. Blood 111:4245-4253.
44. Mohamadzadeh, M., T. Duong, S. J. Sandwick, T. Hoover, and T. R. Klaenhammer. 2009. Dendritic cell targeting of Bacillus anthracis protective antigen expressed by Lactobacillus acidophilus protects mice from lethal challenge. Proc Natl Acad Sci U S A 106:4331-4336.
45. Mouries, J., G. Moron, G. Schlecht, N. Escriou, G. Dadaglio, and C. Leclerc. 2008. Plasmacytoid dendritic cells efficiently cross-prime naive T cells in vivo after TLR activation. Blood 112:3713-3722.
46. Preville, X., D. Ladant, B. Timmerman, and C. Leclerc. 2005. Eradication of established tumors by vaccination with recombinant Bordetella pertussis adenylate cyclase carrying the human papillomavirus 16 E7 oncoprotein. Cancer research 65:641-649.
47. Pym, A. S., P. Brodin, L. Majlessi, R. Brosch, C. Demangel, A. Williams, K. E. Griffiths, G. Marchal, C. Leclerc, and S. T. Cole. 2003. Recombinant BCG exporting ESAT-6 confers enhanced protection against tuberculosis. Nat Med 9:533-539.
48. Randolph, G. J., J. Ochando, and S. Partida-Sanchez. 2008. Migration of dendritic cell subsets and their precursors. Annual review of immunology 26:293-316.
49. Reis e Sousa, C. 2006. Dendritic cells in a mature age. Nature reviews 6:476-483.
50. Rezzonico, R., R. Chicheportiche, V. Imbert, and J. M. Dayer. 2000. Engagement of CD11b and CD11c beta2 integrin by antibodies or soluble CD23 induces IL-1beta production on primary human monocytes through mitogen-activated protein kinase-dependent pathways. Blood 95:3868-3877.
51. Rezzonico, R., V. Imbert, R. Chicheportiche, and J. M. Dayer. 2001. Ligation of CD11b and CD11c beta(2) integrins by antibodies or soluble CD23 induces macrophage inflammatory protein 1alpha (MIP-1alpha) and MIP-1beta production in primary human monocytes through a pathway dependent on nuclear factor-kappaB. Blood 97:2932-2940.
52. Sancho, D., D. Mourao-Sa, O. P. Joffre, O. Schulz, N. C. Rogers, D. J. Pennington, J. R. Carlyle, and C. Reis e Sousa. 2008. Tumor therapy in mice via antigen targeting to a novel, DC-restricted C-type lectin. The Journal of clinical investigation 118:2098-2110.
53. Scriba, T. J., B. Kalsdorf, D. A. Abrahams, F. Isaacs, J. Hofmeister, G. Black, H. Y. Hassan, R. J. Wilkinson, G. Walzl, S. J. Gelderbloem, H. Mahomed, G. D. Hussey, and W. A. Hanekom. 2008. Distinct, specific IL-17- and IL-22-producing CD4+ T cell subsets contribute to the human anti-mycobacterial immune response. J Immunol 180:1962-1970.
54. Shortman, K., M. H. Lahoud, and I. Caminschi. 2009. Improving vaccines by targeting antigens to dendritic cells. Exp Mol Med 41:61-66.
55. Shortman, K., and S. H. Naik. 2007. Steady-state and inflammatory dendritic-cell development. Nature reviews 7:19-30.
56. Shrimpton, R. E., M. Butler, A. S. Morel, E. Eren, S. S. Hue, and M. A. Ritter. 2009. CD205 (DEC-205): a recognition receptor for apoptotic and necrotic self. Molecular immunology 46:1229-1239.
57. Simeone, R., D. Bottai, and R. Brosch. 2009. ESX/type VII secretion systems and their role in host-pathogen interaction. Current opinion in microbiology 12:4-10.
58. Steinman, R. M. 2008. Dendritic cells in vivo: a key target for a new vaccine science. Immunity 29:319-324.
59. Steinman, R. M., and J. Banchereau. 2007. Taking dendritic cells into medicine. Nature 449:419-426.
60. Sun, C. M., J. A. Hall, R. B. Blank, N. Bouladoux, M. Oukka, J. R. Mora, and Y. Belkaid. 2007. Small intestine lamina propria dendritic cells promote de novo generation of Foxp3 T reg cells via retinoic acid. The Journal of experimental medicine 204:1775-1785.
61. Takai, T., M. Li, D. Sylvestre, R. Clynes, and J. V. Ravetch. 1994. FcR gamma chain deletion results in pleiotrophic effector cell defects. Cell 76:519-529.
62. Trumpfheller, C., J. S. Finke, C. B. Lopez, T. M. Moran, B. Moltedo, H. Soares, Y. Huang, S. J. Schlesinger, C. G. Park, M. C. Nussenzweig, A. Granelli-Piperno, and R. M. Steinman. 2006. Intensified and protective CD4+ T cell immunity in mice with anti-dendritic cell HIV gag fusion antibody vaccine. The Journal of experimental medicine 203:607-617.
63. Umemura, M., A. Yahagi, S. Hamada, M. D. Begum, H. Watanabe, K. Kawakami, T. Suda, K. Sudo, S. Nakae, Y. lwakura, and G. Matsuzaki. 2007. IL-17-mediated regulation of innate and acquired immune response against pulmonary Mycobacterium bovis bacille Calmette-Guerin infection. J lmmunol 178:3786-3796.
64. Valladeau, J., V. Clair-Moninot, C. Dezutter-Dambuyant, J. J. Pin, A. Kissenpfennig, M. G. Mattei, S. Ait-Yahia, E. E. Bates, B. Malissen, F. Koch, F. Fossiez, N. Romani, S. Lebecque, and S. Saeland. 2002. Identification of mouse langerin/CD207 in Langerhans cells and some dendritic cells of lymphoid tissues. J Immunol 168:782-792.
65. Veldhoen, M., R. J. Hocking, C. J. Atkins, R. M. Locksley, and B. Stockinger. 2006. TGFbeta in the context of an inflammatory cytokine milieu supports de novo differentiation of IL-17-producing T cells. Immunity 24:179-189.
66. Villadangos, J. A., and P. Schnorrer. 2007. Intrinsic and cooperative antigen-presenting functions of dendritic-cell subsets in vivo. Nature reviews 7:543-555.
67. Wang, W. W., D. Das, and M. R. Suresh. 2009. A versatile bifunctional dendritic cell targeting vaccine vector. Mol Pharm 6:158-172.
68. WHO. 2007. WHO report 2007. Global tuberculosis control.
69. Sano, T., Pandori, M. W., Chen, X., Smith, C. L., Cantor, C. R. 1995. Recombinant Core Streptavidins. J. Biol. Chem; 270(47): 28204 - 28209.
70. Denis O, et al. 1998. Infect Immun. 1998 Apr;66(4):1527-33.
71. Sørensen AL et al. 1995. Infect Immun. 1995 63(5):1710-7.
72. Berthet FX et al. 1998. Microbiology. 1998 144:3195-203.
73. Bottai D and Brosch R. 2009. Mol Microbiol. 2009 Aug;73(3):325-8.
74. Howarth M., Chinnapen D. J-F., Gerrow K., Dorrestein P. C., Grandy M. R., Kelleher N. L., El-Husseini A., Ting A. Y. A monovalent streptavidin with a single femtomolar biotin binding site. Nature Methods 3, 267 - 273 (2006).
75. Wu S.-C. and Wong S.-L. Engineering Soluble Monomeric Streptavidin with Reversible Biotin Binding Capability. 2005. The Journal of Biological Chemistry, 280, No. 24, 23225-23231.

## Claims

1. A combination of compounds comprising or consisting of :
(i) a fusion polypeptide comprising or consisting of:
- a streptavidin (SA) or avidin polypeptide; and
- one or several effector molecule(s),
wherein said fusion polypeptide retains the property of SA and avidin polypeptides to bind biotin;
(ii) one or several biotinylated targeting molecule(s), which is(are) capable of targeting and in particular specifically interacting with:
- subset(s) of cells, in particular with antigen presenting cells (APC) and/or subset(s) of APC, for example dendritic cells (DC) or B lymphocytes, and/or with
- cell surface molecule(s), in particular cell surface receptors, for example of APC and/or subset(s) of APC, including DC or B lymphocytes,
wherein (i) and (ii) are present in distinct compositions or in the same composition.

2. Combination according to claim 1, wherein the fusion polypeptide further comprises one or several linker(s), in particular one or several flexible linker(s), which is(are) located, for example, between the SA or avidin polypeptide and an effector molecule.

3. Combination according to claim 1 or 2, wherein the avidin polypeptide is a deglycosylated version of avidin, in particular neutravidin.

4. Combination according to any one of claims 1 to 3, wherein the fusion polypeptide is in the form of a monomer, or, preferably, in the form of a tetramer, and in particular in the form of a homotetramer or in the form of a heterotetramer, wherein
- a) at least one monomer of the tetramer comprises or consists of (i) a monomer of the SA or avidin polypeptide and (ii) one or several effector molecule(s); and
- b) the other monomers of the tetramer comprise or consist of a monomer of the SA or avidin polypeptide, and optionally one or several effector molecule(s) or the other monomers of the tetramer comprise or consist of (i) a monomer of the SA or avidin polypeptide and (ii) one or several effector molecule(s) different from the ones as defined in (a).

5. Combination according to any one of claims 1 to 4, wherein the SA polypeptide consists of:
- the amino acid sequence ranging from amino acid residues 13 to 139 or 14 to 139 in the SA protein from *Streptomyces avidinii* (SEQ ID NO.2 and 41 respectively); or
- a amino acid sequence having at least 70%, preferably at least 80% and more preferably at least 90% or 95% identity with the above-mentioned amino acid sequence SEQ ID NO.2 or SEQ ID NO. 41, and which retains the property of the SA protein to bind biotin.

6. Combination according to any one of claims 1 to 5, wherein the effector molecule(s) is(are) chosen among the following group:
- a polypeptide molecule suitable for eliciting an immune response, for example an epitope, an antigen or a fragment thereof which comprises at least one epitope, said epitope, antigen or fragment thereof being derived from an allergen, a toxin, a tumoral cell or an infectious agent, in particular a bacteria, a parasite, a fungus or a virus;
- a cytokine, a polypeptide drug, a toxin, a toxoid, an enzyme, an oncoprotein, a protein which regulates cell cycle or metabolism, a fluororescent polypeptidic marker or a polypeptide binding a nucleic acid, an aptamer or a recombinant ligand capable of binding biologically active molecules, for example cytokines having modulating activity on cells of the immune system and in particular on dendritic cells or on lymphocytes.

7. Combination according to any one of claims 1 to 6, wherein one or several effector molecule(s) comprise(s) or consist(s) of a Chlamydia antigen, a Mycoplasma antigen, a Mycobacteria antigen, for example, an antigen from *Mycobacterium tuberculosis or Mycobacterium leprae,* a Plasmodia antigen, for example, an antigen from *Plasmodium berghei, Plasmodium vivax* or *Plasmodium falciparum,* a hepatitis virus antigen, a poliovirus antigen, an HIV virus antigen, for example, a HIV protein, a HPV virus antigen, for example, a E7 antigen of a HPV virus, especially the E7 antigen of HPV16, a CMV virus antigen, for example, the pp65 protein, an influenza virus antigen, a choriomeningitis virus antigen, or a tumor-associated antigen, or comprises or consists of a part of an amino acid sequence of any these antigens which comprises at least one epitope.

8. Combination according to any one of claims 1 to 7, wherein the fusion polypeptide further comprises one or several ligand(s), in particular one or several recombinant ligand(s), for example one or several protein scaffold(s).

9. Combination according to any one of claims 1 to 18, wherein the biotinylated targeting molecule(s) is(are) polypeptide molecule(s) capable of specifically interacting with one or several cell surface receptor(s) chosen from the following group:
- C-type lectins, in particular:
- members of the mannose receptor family, for example CD205 endocytic C-type lectins (DEC205),
- members of the asialoglycoprotein receptor family, for example CD207 (Langerin, Clec4K), or CD209 (DC-Specific ICAM3-Grabbing Non-integrin, DC-SIGN), or
- members of the DC Immunoreceptor (DCIR) subfamily of asialoglycoproteoin receptor, for example DCIR-2 (Clec4A);
- MHC-I and MHC-II;
- PDCA-1;
- Integrins, for example β2 integrins, or α and βintegrin subunits, for example CD11 b and CD11 c;
- Dendritic cell inhibitory receptor 2 (DCIR-2); and
- Clec12A.

10. Combination according to any one of claims 1 to 9, wherein the biotinylated targeting molecule(s) is(are) capable of specifically interacting with cells, subset of cells, or surface molecule(s), and especially surface receptor(s), of cells which induce a CD4+ immune response and/or a CD8+ immune response.

11. Combination according to any one of claims 1 to 10, wherein the biotinylated targeting molecule is chosen from:
- biotinylated antibodies, in particular a biotinylated monoclonal antibodies, or biotinylated antibody-like molecules;
- biotinylated ligands, in particular biotinylated scaffold ligands or biotinylated non-proteinaceous ligands,
- biotinylated polysaccharides, biotinylated nucleic acids, in particular DNAs or RNAs, or biotinylated lipids,
which biotinylated antibodies, antibody-like molecules, ligands, polysaccharides, nucleic acids or lipids are capable of specifically interacting with subset(s) of cells, in particular subset(s) of DC or B lymphocytes, and/or with cell surface molecule(s) and in particular cell surface receptor(s), for example of DC or B lymphocytes.

12. Combination according to any one of claims 1 to 11, which further comprises one or several biotinylated, non-targeting molecule(s), which can be for example chosen from the following group: biotinylated antigens or fragments thereof which comprise at least one epitope, biotinylated protoxins, biotinylated nucleic acids, in particular RNAs or DNAs, for example cDNAs, biotinylated adjuvant molecules and biotinylated cytokines, for example IL-2, IL-10, IL-12, IL-17, IL-23, TNFα or IFNγ.

13. Combination according to any one of claims 1 to 12, wherein the fusion polypeptide, the biotinylated targeting molecule(s), and if present, biotinylated, non-targeting molecule(s), are present in the same composition, wherein the fusion polypeptide is complexed to the biotinylated targeting molecule(s), and optionally to biotinylated, non-targeting molecule(s) as defined in claim 12.

14. Combination according to any one of claims 1 to 13, further comprising a pharmaceutically acceptable carrier, and optionally an adjuvant, an immunostimulant, for example Poly I:C (polyinosinic:polycytidylic acid or polyinosinic-polycytidylic acid sodium salt), and/or a further therapeutically active molecule, which are combined with the fusion polypeptide and/or with the biotinylated targeting molecule(s), and/or, if present, with biotinylated, non-targeting molecule(s).

15. Combination according to any one of claims 1 to 14, for use in prophylaxis and/or in therapy for targeting one or several effector molecule(s) of the fusion polypeptide to subset(s) of cells, in particular dendritic cells (DC) or B lymphocytes or subset(s) of DC or B lymphocytes, and/or to cell surface molecule(s), and especially cell surface receptor(s), in particular surface molecule(s) or receptor(s) of DC or B lymphocytes, for use to elicit a T-cell immune response and/or a B-cell immune response in a human or non-human host in need thereof.

16. Combination according to any one of claims 1 to 15, for use to select a cell population for use for diagnosing or immunomonitoring a disease in a mammal or or to stimulate a cell population for use in recall response assays from a sample from a human or a non-human mammal.

17. Combination according to any one of claims 1 to 16, for use for the prevention or the treatment of a disease selected from neoplasia, cancers and infectious diseases selected from viral-, retroviral-, bacterial-, parasite- or fungus-induced diseases.

18. Use of a combination according to any one of claims 1 to 17, for targeting, *in vitro* or *ex vivo,* one or several effector molecule(s) of the fusion polypeptide to subset(s) of cells and/or cell surface molecule(s), and in particular dendritic cells (DC) or B lymphocytes, subset(s) of DC or B lymphocytes and/or surface molecule(s) or receptor(s) of DC or B lymphocytes.

19. A method for the production of a polypeptide comprising a streptavidin (SA) or avidin polypeptide, and in particular a fusion polypeptide as defined in any one of claims 1 to 8, and more particularly a SA or avidin polypeptide or a fusion polypeptide as defined in any one of claims 1 to 8 capable of forming a soluble tetramer said method comprising: expressing, for example at a temperature of 20°C or less than 20°C, said polypeptide in an *E. coli* cell and more preferably an *E. coli* BL21 λDE3 cell or an *E. coli* Artic Express DE3 cell, from a polynucleotide, a plasmid or a vector encoding said polypeptide.

20. A method for the production of a polypeptide according to claim 19, wherein the expressed polypeptide is purified using one or several IminoBiotin-Agarose columns (Sigma), wherein, optionally, the columns with fixed polypeptide are washed with a solution comprising 0.5 M NaCl and are eluted with a solution without any salt.

21. A method for the *in vitro* or *ex vivo* selection of a subset of antigen presenting cells (APC) to which the targeting of an antigen or a fragment thereof comprising at least one T-cell epitope can induce a T-cell immune response directed against said antigen or fragment thereof, wherein said method comprises the steps of:
- exposing T cells, in particular CD8+ and/or CD4+ T cells, to a subset of APC binding a fusion polypeptide as defined in any one of claims 1 to 8, through biotinylated targeting molecule(s) as defined in any one of claims 1 or 9 to 11, wherein the fusion polypeptide comprises said antigen or fragment thereof; and
- detecting a change in activation of the T cells,
wherein the change in activation of the T cell is for example a change in IL-2, IL-4, IL-5, IL-17 or IFN-γ production.

22. A method according to claim 21, which comprises the steps of:
a) exposing a subset of APC to (i) biotinylated targeting molecules as defined in any one of claims 1 or 9 to 11, which are capable of targeting these APCs and in particular of interacting with one or several cell receptor(s) present on the surface of this subset of APCs, and to (ii) a fusion polypeptide as defined in any one of claims 1 to 8, which comprises said antigen or fragment thereof, wherein optionally said fusion polypeptide has been previously produced by the method of claim 19 or 20; and
b) exposing T cells, in particular CD8+ or CD4+ T cells, to the subset of APCs provided by step a); and
c) detecting *in vitro* a change in activation of the T cells,
wherein the change in activation of the T cell is for example a change in IL-2, IL-4, IL-5, IL-17 or IFN-γ production.

23. A method for the *in vitro* or *ex vivo* stimulation of specific T lymphocytes by targeting an antigen or fragment thereof comprising at least one T-cell epitope to antigen presenting cells, wherein said method comprises the steps of:
- exposing T cells, in particular CD8+ or CD4+ T cells, present in PMBC or whole blood to a fusion polypeptide as defined in any one of claims 1 to 8, which comprises said antigen or fragment thereof, and to a biotinylated targeting molecule(s) as defined in any one of claims 1 or 9 to 11, which is capable of targeting one or several cell receptor(s) of antigen presenting cells, and wherein optionally said fusion polypeptide has been previously produced by the method of claim 19 or 20; and
- detecting *in vitro* a change in activation of the T cells,
wherein the change in activation of the T cells is for example a change in IL-2, IL-4, IL-5, IL-17 or IFN-γ production.

24. A kit, in particular a kit for a diagnostic test of a disease in a mammal and/or for immunomonitoring a disease in a mammal and/or for the prevention and/or the treatment of a disease in a mammal, which comprises :
- a fusion polypeptide as defined in any one of claims 1 to 8, or a polynucleotide, a plasmid or a recombinant vector encoding said fusion polypeptide, or a cell able to express said fusion polypeptide; and
- instructions explaining how to use said fusion polypeptide in conjunction with biotinylated targeting molecule(s) in order that effector molecule(s) comprised in said fusion polypeptide be delivered into or onto subset(s) of cells targeted via said biotinylated targeting molecule(s); and
- optionally, biotinylated targeting molecule(s) as defined any one of claims 1 or 9-11 and/or biotinylated molecule(s) as defined in claim 12.
